(19) Europäisches Patentamt
European Patent Office
Office européen des brevets

(11) **EP 1 357 156 B1**

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(51) Int Cl.:
***C09C 1/30*** *(2006.01)*

(21) Anmeldenummer: **03007875.2**

(22) Anmeldetag: **07.04.2003**

(54) **Silanmodifizierter oxidischer oder silikatischer Füllstoff, Verfahren zur seiner Herstellung und seine Verwendung**

Silane modified oxidic or silicate filler, method for its manufacture and its use

Charge de type oxyd ou silicate modifiée au silane, procédé de sa fabrication et son utilisation

(84) Benannte Vertragsstaaten:
**AT BE BG CH CY CZ DE DK EE ES FI FR GB GR HU IE IT LI LU MC NL PT SE SI SK TR**

(30) Priorität: **25.04.2002 DE 10218350**

(43) Veröffentlichungstag der Anmeldung:
**29.10.2003 Patentblatt 2003/44**

(73) Patentinhaber: **Evonik Degussa GmbH**
**45128 Essen (DE)**

(72) Erfinder:
• **Korth, Karsten, Dr.**
**79639 Grenzach-Wyhlen (DE)**
• **Eichenauer, Kurt**
**63628 Bad Soden-Salmünster (DE)**
• **Pieter, Reimund, Dr.**
**64625 Bensheim (DE)**
• **Klockmann, Oliver, Dr.**
**50858 Köln (DE)**
• **Heidlas, Jürgen, Dr.**
**83308 Trostberg (DE)**
• **Ober, Martin**
**83352 Altenmarkt (DE)**
• **Zobel, Rudolf**
**97348 Willanzheim (DE)**

(56) Entgegenhaltungen:
EP-A- 1 295 850      DE-A- 19 928 851
US-A- 6 013 234      US-A- 6 022 404

## Beschreibung

Die Erfindung betrifft ein Verfahren zur Herstellung von silanmodifiziertem oxidischem oder silikatischem Füllstoff.

Es ist bekannt, oxidische oder silikatische Verbindungen mit Organosiliziumverbindungen zu behandeln, um durch diese Behandlung den Verbund zwischen anorganischem Füllstoff und dem verwendeten organischen Polymer in füllstoffverstärkten Elastomeren zu stärken und damit die Eigenschaften der Füllstoffe in den Polymeren zu verbessern.

Aus DE 2141159, DE 2212239 und US 3,978,103 ist bekannt, daß schwefelhaltige Organosiliziumverbindungen, wie Bis-(3-Triethoxysilylpropyl)tetrasulfan oder 3-Mercaptopropyltriethoxysilan als Silanhaftvermittler oder Verstärkungsadditiv in oxidisch gefüllten Kautschukmischungen, unter anderem für Laufflächen und andere Teile von Autoreifen, eingesetzt werden.

Um die erheblichen Probleme bei der Verarbeitung, wie zum Beispiel Pre-Scorch, Scorch und Plastizitätsverhalten, von Mercaptosilanen zu umgehen, ist es bekannt, als Kopplungsagenz für Reifenteile meist polysulfidische Organosilane, wie beispielsweise das Bis-(3-triethoxysilylpropyl)tetrasulfan oder das Bis-(3-triethoxysilylpropyl)disulfan (DE 2542534, DE2405758, DE19541404, DE19734295), die für kieselsäuregefüllte Vulkanisate einen Kompromiss in Bezug auf Vulkanisationssicherheit, einfache Herstellung und Verstärkungsleistung darstellen, zu verwenden.

Die bekannte Einbringung der entsprechenden Additive, speziell der Organosilane und der unmodifizierten Füllstoffe in die unvulkanisierten Polymermischungen kann auf unterschiedlichen Wegen erfolgen.

Das in-situ Verfahren beinhaltet einen gemeinsamen Mischvorgang von Füllstoffen, wie Ruß und Kieselsäure, mit Organosilanen und den verwendeten Polymeren.

Das ex-situ Verfahren beinhaltet eine Modifikation des Füllstoffs mit dem entsprechenden Organosilan oder einer Mischung von verschiedenen Organosilanen, bevor der Füllstoff mit dem Polymer zur Gummirohmischung verarbeitet wird.

Bekannt ist weiterhin die Flüssigdosierung von Organosilanen (US 3,997,356) bei der Rohmischungsherstellung für Gummimischungen oder auch die Dosierung des aktiven Füllstoffs über eine vorgebildete physikalische Mischung aus Organosilan und Füllstoff (DE 3314742, US 4,076,550). Nachteil dieser thermisch nicht vorbehandelten Abmischungen ist die mangelnde Lagerstabilität und damit die häufig nicht gegebene Eigenschaftsstabilität der Produkte.

Die US-PS 4,151,154 beschreibt oxidische silikatische Füllstoffe, deren Oberfläche einer Behandlung mit zwei unterschiedlichen Typen von Organosiliziumverbindungen ausgesetzt wird. Die oxidischen Teilchen werden dabei so behandelt, daß sie eine größere Affinität zu Wasser zeigen und sich auch leichter in wässrigen Systemen verteilen lassen.

Aus der US 3,567,680 ist die Modifizierung von in Wasser suspendiertem Kaolin mit verschiedenen Organosilanen bekannt. Die beschriebenen Organosiliziumverbindungen sind jedoch in den für die Modifizierung nötigen Mengen wasserlöslich, so daß in diesem Fall die Behandlung des Füllstoffes aus einer wässrigen Lösung heraus erfolgen kann.

Die US 4,044,037 beschreibt Arylpolysulfide und mit diesen Verbindungen behandelte mineralische Füllstoffe, die in Kautschukmischungen eingesetzt werden. Die Herstellung erfolgt in einer wässrig/alkoholischen Formulierung, die 80 bis 99,9 Gew.-% Alkohol enthält.

Ferner ist aus EP-PS 01 26 871 ein Verfahren bekannt, bei dem man die Oberfläche silikatischer Füllstoffe mit Hilfe einer wässrigen Emulsion von wasserunlöslichen Organosiliziumverbindungen modifiziert.

Es ist bekannt, daß durch Auflösen der Organosiliziumverbindung in einem organischen Lösungsmittel und anschließender Behandlung von Füllstoffen, z.B. Clays, deren Füllstoffoberfläche modifiziert werden kann (US 3,227,675).

Die bekannten Verfahren zur Modifizierung von Füllstoffen für Gummi- und Kunststoffanwendungen mit oberflächenaktiven Organosilanen oder deren Mischungen haben den Nachteil, daß diese auf der Verwendung von Wasser, organischen Lösungsmitteln oder dem direkten Aufsprühen der Organosiliziumverbindung auf die Oberfläche des Füllstoffs mit einer nachfolgenden Wärmebehandlung, der Temperreaktion, basieren. Die bekannten wasserunlöslichen, kautschuktypischen Organosilane können häufig nur in kohlenwasserstoffbasiernden Lösungsmitteln, die meist gesundheitsschädlich und gut brennbar sind, effektiv mit Füllstoffen chemisch verbunden werden.

Die bekannten, mit Organosilanen ex-situ modifizierten Füllstoffe haben den Nachteil, daß die Gummieigenschaften bisher nicht besser, sondern tendenziell eher schlechter als bei den in-situ miteinander gemischten Füllstoffen und Silanen sind.

Im Falle von Füllstoffen mit großer, spezifischer Oberfläche, beziehungsweise einer hohen Oberflächenstruktur, ist die Silanisierung zudem häufig nicht homogen. Eine Diffusion der Silanmoleküle in tieferliegende Schichten von hochporösen Füllstoffen, wie beispielsweise gefällten Kieselsäuren, ist mit den bisher bekannten Modifizierungsmethoden nicht oder nur unvollständig zu erreichen: Makroskopisch vorgeformte Füllstoffe werden deshalb durch die bekannten Silanisierungsverfahren nur ungenügend und unvollständig modifiziert.

Zudem können durch die bekannten Silanisierungsverfahren und den anschließend häufig notwendigen Trocknungsverfahren keine vorgeformten Füllstoffe erfolgreich und zerstörungfrei beziehungsweise abriebarm silanisiert werden. Durch die bekannten Verfahren (US 4,151,154: DE 3314742 C2; US 3,567,680) werden beziehungsweise würden vorgeformten Füllstoffe in Ihrer Struktur zerstört beziehungsweise beeinträchtigt. So werden beispielsweise auf Walzen geformte Granulate von Kieselsäuren (DE 3014007) durch die Einbringung und länger andauernde Bewegung in einem Mischer oder ähnlichen Aggregaten sehr schnell zu einem Kieselsäurepulver minderer Qualität (höherer Staub- und Feinanteil) abgebaut.

Aus DE 10122269.6 ist ein Verfahren zur Umsetzung von mindestens einem biopolymeren, biooligomeren, oxidischen oder silikatischen Füllstoff in einem verdichteten Gas mit mindestens einem Silan bekannt.

Nachteilig ist die dort beschriebene Verwendung von pulverförmigen und granulatförmigen Füllstoffen. Pulverförmige Kieselsäuren sind unter industriellen Bedingungen, beispielsweise aufgrund ihres hohen Staubanteils, ihrer geringen Schüttdichte, ihres mangelhaften Fließverhaltens und der dadurch oft schlechten Dosierbarkeit von Nachteil. Granulate können nachträglich aus pulverförmigen Kieselsäuren durch mechanische Verdichtung gewonnen werden. Da es einen zusätzlichen Verarbeitungsschritt bedeutet, versucht man solche Prozesse aus wirtschaftlichen Erwägungen zu vermeiden. Diese Granulate zerfallen unter mechanischer Belastung leicht wieder in das pulverförmige Ausgangsmaterial, zudem verschlechtern sich häufig die anwendungstechnischen Eigenschaften der Kieselsäuren durch die nachträgliche Granulation und die damit verbundene mechanische Belastung der Partikel.

Aufgabe der vorliegenden Erfindung ist es, einen staubarmen silanmodifizierten oxidischen oder silikatischen Füllstoff direkt aus einem staubarmen mikrogeperlten oder mikrogranulären, oxidischen oder silikatischen Füllstoff herzustellen. Der silanmodifizierte oxidische oder silikatische Füllstoff soll eine zufriedenstellende, quantitativ leicht variierbare Belegung mit den entsprechenden kautschukreaktiven Silanen aufweisen und zudem vergleichbare oder bessere Eigenschaften als bekannte, in-situ hergestellte Silan-Füllstoffmischungen und zudem verbesserte gummitechnische Eigenschaften als bekannte, ex-situ hergestellte Silan-Füllstoffabmischungen im Kautschuk aufweisen.

Eine weitere Aufgabe der Erfindung ist es, den zu modifizierenden mikrogeperlten oder mikrogranulären, oxidischen oder silikatischen Füllstoff in einer staubarmen Darreichungsform be- beziehungsweise verarbeiten zu können. Die äußere, makroskopische Form dieser vorgeformten mikrogeperlten oder mikrogranulären, oxidischen oder silikatischen Füllstoffe soll während des Modifizierungsprozesses weitgehend erhalten bleiben. Ein weitgehend staubfreier beziehungsweise staubarmer silanmodifizierter oxidischer oder silikatischer Füllstoff soll erhalten werden.

Eine weitere Aufgabe der vorliegenden Erfindung ist es, ein Verfahren zur Modifizierung von mikrogeperlten oder mikrogranulären, oxidischen oder silikatischen Füllstoffen mit Silanen bereitzustellen, bei dem die Modifizierungsreaktion nicht in Wasser oder organischen Lösungsmitteln durchgeführt wird.

Mit dem erfindungsgemäßen Verfahren können silanmodifizierte oxidische oder silikatische Füllstoffe hergestellt werden, wobei die Perlfraktion kleiner als 75 $\mu$m (Fein- beziehungsweise Staubanteil) weniger als 15 Gew.-%, bevorzugt weniger als 10 Gew.-%, besonders bevorzugt weniger als 7 Gew.-%, insbesondere bevorzugt weniger als 5 Gew. %, bestimmt durch Siebanalyse, und der Medianwert der Partikelgröße zwischen 130 $\mu$m und 500 $\mu$m, bevorzugt zwischen 130 $\mu$m und 450 $\mu$m, besonders bevorzugt zwischen 150 $\mu$m und 400 $\mu$m, insbesondere bevorzugt zwischen 175 $\mu$m und 350 $\mu$m, bestimmt durch Laserbeugung ohne Ultraschallbehandlung, beträgt.

Der mit dem erfindungsgemäßen Verfahren hergestellte Füllstoff kann einen statistisch ermittelten Mittelwert des Formfaktors größer 0,805, bevorzugt größer 0,82, besonders bevorzugt größer 0,84, insbesondere bevorzugt größer 0,86, aufweisen.

Der mit dem erfindungsgemäßen Verfahren hergestellte Füllstoff kann einen statistisch ermittelten Mittelwert des Kreisformfaktors von größer 0,55, bevorzugt größer 0,57, besonders bevorzugt größer 0,60, und insbesondere bevorzugt größer 0,62, aufweisen.

Der mit dem erfindungsgemäßen Verfahren hergestellte Füllstoff kann Mikroporen im Bereich von <2 nm zwischen 0 und 0,5 ml/g, bevorzugt zwischen 0 und 0,3 ml/g, besonders bevorzugt zwischen 0 und 0,1 ml/g, aufweisen.

Der mit dem erfindungsgemäßen Verfahren hergestellte Füllstoff kann Mesoporen im Bereich zwischen 2 und 30 nm zwischen 0 und 1 ml/g, bevorzugt zwischen 0 und 0,75 ml/g, besonders bevorzugt zwischen 0 und 0,5 ml/g, aufweisen.

Der mit dem erfindungsgemäßen Verfahren hergestellte Füllstoff kann Mesoporen im Bereich zwischen 2 und 50 nm zwischen 0 und 5 ml/g, bevorzugt zwischen 0 und 2,5 ml/g, besonders bevorzugt zwischen 0 und 1,5 ml/g, aufweisen.

Der mit dem erfindungsgemäßen Verfahren hergestellte Füllstoff kann Makroporen im Bereich ab 30 nm zwischen 0 und 10 ml/g, bevorzugt zwischen 0 und 7,5 ml/g, besonders bevorzugt zwischen 0 und 5 ml/g, aufweisen.

Der mit dem erfindungsgemäßen Verfahren hergestellte Füllstoff kann Makroporen im Bereich ab 50 nm zwischen 0 und 10 ml/g, bevorzugt zwischen 0 und 7,5 ml/g, besonders bevorzugt zwischen 0 und 5 ml/g, aufweisen.

Der mit dem erfindungsgemäßen Verfahren hergestellte Füllstoff kann eine BET-Oberfläche zwischen 0,5 m$^2$/g und 500 m$^2$/g, bevorzugt zwischen 0,5 und 300 m$^2$/g, besonders bevorzugt zwischen 0,5 und 250 m$^2$/g, aufweisen.

Der mit dem erfindungsgemäßen Verfahren hergestellte Füllstoff kann Searszahlen (Verbrauch 0,1 KOH) zwischen 1 und 50 ml pro 5 g Probe aufweisen.

Der mit dem erfindungsgemäßen Verfahren hergestellte Füllstoff kann einen Gehalt an Schwefel in reiner oder chemisch gebundener Form zwischen 0,05 und 25 Gew.-%, bevorzugt zwischen 0,05 und 10 Gew.-%, besonders bevorzugt zwischen 0,05 und 4 Gew.-%, aufweisen.

Der mit dem erfindungsgemäßen Verfahren hergestellte Füllstoff kann einen Gehalt an Kohlenstoff in reiner oder chemisch gebundener Form zwischen 0,1 und 25 Gew.-%, bevorzugt zwischen 0,1 und 10 Gew.-%, besonders bevorzugt zwischen 0,1 und 5 Gew.-%, aufweisen.

Der mit dem erfindungsgemäßen Verfahren hergestellte Füllstoff kann einen Gehalt an physikalisch und chemisch gebundenem Alkohol zwischen 0 und 25 Gew.-%, bevorzugt zwischen 0 und 15 Gew.-%, besonders bevorzugt zwischen 0 und 10 Gew.-%, aufweisen.

Der mit dem erfindungsgemäßen Verfahren hergestellte Füllstoff kann einen chemisch oder physikalisch gebundenen Restgehalt des vom Silan stammenden Alkohols von kleiner 75 Mol-%, bevorzugt kleiner 50 Mol-%, besonders bevorzugt kleiner 30 Mol-%, insbesondere bevorzugt kleiner 20 Mol-%, der Ausgangsmenge des Alkohols im verwendeten Silan aufweisen.

Der mit dem erfindungsgemäßen Verfahren hergestellte silanmodifizierte oxidische oder silikatische Füllstoff kann überwiegend perlförmig, kugelförmig, rund und/oder homogen geformt sein.

Der mit dem erfindungsgemäßen Verfahren hergestellte silanmodifizierte oxidische oder silikatische Füllstoff kann erhältlich sein, indem man mindestens einen mikrogeperlten oder mikrogranulierten, oxidischen oder silikatischen Füllstoff in einem verdichteten Gas mit mindestens einem Silan umsetzt.

Der mit dem erfindungsgemäßen Verfahren hergestellte silanmodifizierte oxidische oder silikatische Füllstoff kann 0,1 bis 50 Gew.-%, vorzugsweise 0,1 bis 25,0 Gew.-%, besonders bevorzugt 0,1 bis 10 Gew.-%, Silan enthalten.

Der mit dem erfindungsgemäßen Verfahren hergestellte silanmodifizierte oxidische oder silikatische Füllstoff kann 50 bis 99,9 Gew.-% mikrogeperlten oder mikrogranulierten, oxidischen oder silikatischen Füllstoff enthalten.

Die durch Siebung ermittelbare Perlverteilung des mit dem erfindungsgemäßen Verfahren hergestellten silanmodifizierten oxidischen oder silikatischen Füllstoffs kann gleich oder ähnlich der durch Siebung ermittelten Perlverteilung des unbehandelten mikrogeperlten oder mikrogranulierten, oxidischen oder silikatischen Füllstoffs sein.

Die prozentuale Abweichung der Perlfraktionen, bestimmt durch Siebanalyse, zwischen dem Ausgangsstoff, einem mikrogeperlten oder mikrogranulierten, oxidischen oder silikatischen Füllstoff, und dem Endprodukt, einem silanmodifizierten oxidischen oder silikatischen Füllstoff, kann für die Perlfraktion kleiner 75 $\mu$m und die Perlfraktion zwischen 75 und 150 $\mu$m nicht mehr als 100 Gew.-%, bevorzugt nicht mehr als 75 Gew.-%, besonders bevorzugt nicht mehr als 50 Gew.-%, insbesondere bevorzugt nicht mehr als 20 Gew.-%, betragen.

Das Verhältnis der zwei Siebfraktionen >300 $\mu$m und 150 $\mu$m - 300 $\mu$m kann kleiner als 10:1, bevorzugt kleiner 7:1, besonders bevorzugt kleiner 4:1, betragen.

Der mit dem erfindungsgemäßen Verfahren hergestellte silanmodifizierte oxidische oder silikatische Füllstoff kann eine Perlfraktion größer als 1000 $\mu$m von kleiner 30 Gew.-%, bevorzugt kleiner 20 Gew.-%, besonders bevorzugt kleiner 10 Gew.-%, aufweisen.

Der mit dem erfindungsgemäßen Verfahren hergestellte silanmodifizierte oxidische oder silikatische Füllstoff kann eine Perlfraktion größer als 500 $\mu$m von kleiner 30 Gew.-%, bevorzugt kleiner 20 Gew.-%, besonders bevorzugt kleiner 10 Gew.-%, aufweisen.

Das Silan kann chemisch und/oder physikalisch mit der Oberfläche der Füllstoffe verbunden sein.

Gegenstand der Erfindung ist ein Verfahren zur Herstellung von einem silanmodifiziertem oxidischem oder Silikatischem Füllstoff mit einer Perlfraktion Kleiner als 75 $\mu$m von weniger als 15 Gew.-%, bestimmt durch Siebanalyse, und einem Medianwert der Partikelgröße zwischen 130 und 500 $\mu$m, bestimmt durch Lserbeugung ohne Ultraschallbehandlung mikrogeperlten oder mikrogranulierten, oxidischen oder silikatischen Füllstoff in einem mittles Druck und / oder Temperatur verdichteten Gas mit mindestens einem Silan umsetzt.

Als Silan kann man eine Organosiliziumverbindung oder Mischungen von Organosiliziumverbindungen der allgemeinen Formel (I)

$$Z\text{-}A\text{-}S_x\text{-}A\text{-}Z \qquad \text{(I)}$$

verwenden, in der

x eine Zahl von 1 bis 14, vorzugsweise 1 bis 8, besonders bevorzugt 2 bis 6, ist,

Z gleich $SiX^1X^2X^3$ ist und

$X^1$, $X^2$, $X^3$ jeweils unabhängig voneinander bedeuten können Wasserstoff (-H),

Halogen (-Cl, -Br, -I) oder Hydroxy (-OH),

ein Alkylsubstituent, vorzugsweise Methyl- ,Ethyl-, Propyl- oder Butyl-,

ein Alkylsäure- $(C_xH_{2x+1})$-C(=O)O-, Alkenylsäuresubstituent, beispielsweise Acetoxy- $CH_3$-(C=O)O-,

ein substituierter Alkyl-, beziehungsweise

Alkenylsäuresubstituent, zum Beispiel Oximato- $R^1_2$C=NO-,

eine lineare oder verzweigte, ringförmige

Kohlenwasserstoffkette mit 1-8 Kohlenstoffatomen,

ein Cycloalkanrest mit 5-12 Kohlenstoffatomen,

ein Benzylrest oder ein halogen- oder alkylsubstituierter Phenylrest,

Alkoxygruppen, vorzugsweise ($C_1$-$C_{24}$) Alkoxy, besonders bevorzugt Methoxy- ($CH_3O$-) oder Ethoxy- ($C_2H_5O$-), sowie Dodecyloxy- ($C_{12}H_{25}O$-), Tetradecyloxy- ($C_{14}H_{29}O$-), Hexadecyloxy-$C_{16}H_{33}O$-) und Octadecyloxy- ($C_{18}H_{37}O$-), mit linearen oder verzweigten Kohlenwasserstoffketten mit ($C_{1-24}$)-Atomen, Alkoxygruppen mit linearen oder verzweigten Polyetherketten mit $C_1$-$C_{24}$ Atomen,

eine Cycloalkoxygruppe mit ($C_{5-12}$)-Atomen,

eine halogen- oder alkylsubstituierte Phenoxygruppe oder eine Benzyloxygruppe,

A eine lineare oder verzweigte, gesättigte oder ungesättigte aliphatische, aromatische oder gemischt aliphatische/aromatische zweibindige $C_1$-$C_{30}$ umfassende Kohlenwasserstoffkette, vorzugsweise $C_1$-$C_3$, besonders bevorzugt (-$CH_2$-), (-$CH_2$-)$_2$, (-$CH_2$-)$_3$, (-$CH(CH_3)$-$CH_2$-) oder (-$CH_2$-$CH(CH_3)$-), ist.

A kann linear oder verzweigt sein und gesättigte wie auch ungesättigte Bindungen enthalten. A kann anstatt mit Wasserstoffsubstituenten mit verschiedensten Substituenten versehen sein, wie zum Beispiel -CN, Halogenen, beispielsweise -Cl, -Br oder -F, Alkoholfunktionalitäten -OH, Alkoxiden -$OR^1$ beziehungsweise -O-(C=O)-$R^1$ ($R^1$ = Alkyl, Aryl). Als A können bevorzugt $CH_2$, $CH_2CH_2$, $CH_2CH_2CH_2$, $CH_2CH(CH_3)$, $CH_2CH_2CH_2CH_2$, $CH_2CH_2CH(CH_3)$, $CH_2CH(CH_3)CH_2$, $CH_2CH_2CH_2CH_2CH_2$, $CH_2CH(CH_3)CH_2CH_2$, $CH_2CH_2CH(CH_3)CH_2$, $CH(CH_3)CH_2CH(CH_3)$ oder $CH_2CH(CH_3)CH(CH_3)$ verwendet werden.

Als Silan der allgemeinen Formel (I) kann man beispielsweise folgende Verbindungen verwenden:

$[(MeO)_3Si(CH_2)_3]_2S$, $[(MeO)_3Si(CH_2)_3]_2S_2$, $[(MeO)_3Si(CH_2)_3]_2S_3$,
$[(MeO)_3Si(CH_2)_3]_2S_4$, $[(MeO)_3Si(CH2)_3]_2S_5$, $[(MeO)_3Si(CH_2)_3]_2S_6$,
$[(MeO)_3Si(CH_2)_3]_2S_7$, $[(MeO)_3Si(CH_2)_3]_2S_8$, $[(MeO)_3Si(CH_2)_3]_2S_9$,
$[(MeO)_3Si(CH_2)_3]_2S_{10}$, $[(MeO)_3Si(CH_2)_3]_2S_{11}$, $[(MeO)_3Si(CH_2)_3]_2Si_2$,
$[(EtO)_3Si(CH_2)_3]_2S$, $[(EtO)_3Si(CH_2)_3]_2S_2$, $[(EtO)_3Si(CH_2)_3]_2S_3$,
$[(EtO)_3Si(CH_2)_3]_2S_4$, $[(EtO)_3Si(CH_2)_3]_2S_5$, $[(EtO)_3Si(CH_2)_3]_2S_6$,
$[(EtO)_3Si(CH_2)_3]_2S_7$, $[(EtO)_3Si(CH_2)_3]_2S_8$, $[(EtO)_3Si(CH_2)_3]_2S_9$,
$[(EtO)_3Si(CH_2)_3]_2S_{10}$, $[(EtO)_3Si(CH_2)_3]_2S_{11}$, $[(EtO)_3Si(CH_2)_3]_2S_{12}$,
$[(EtO)_3Si(CH_2)_3]_2S_{13}$, $[(EtO)_3Si(CH_2)_3]_2S_{14}$, $[(C_3H_7O)_3Si(CH_2)_3]_2S$,
$[(C_3H_7O)_3Si(CH_2)_3]_2S_2$, $[(C_3H_7O)_3Si(CH_2)_3]_2S_3$, $[(C_3H_7O)_3Si(CH_2)_3]_2S4$,
$[(C3H_7O)_3Si(CH_2)_{3]2}S_5$, $[(C_3H_7O)_3Si(CH_2)_3]_2S_6$, $[(C_3H_7O)_3Si(CH_2)_3]_2S_7$,
$[(C_3H_7O)_3Si(CH_2)_3]_2S_8$, $[(C_3H_7O)_3Si(CH_2)_3]_2S_9$, $[(C_3H_7O)_3Si(CH_2)_3]_2S_{10}$,
$[(C_3H_7O)_3Si(CH_2)_3]_2S_{11}$, $[(C_3H_7O)_3Si(CH_2)_3]_2S_{12}$, $[(C3_HO)_3Si(CH_2)_3]_2S_{13}$
oder $[(C_3H_7O)_3Si(CH_2)_3]_2S_{14}$

oder auch

$(C_{12}H_{25}O)(EtO)_2Si(CH_2)_3]S_x[(CH_2)_3Si(OEt)_3]$,
$[(C_{12}H_{25}O)_2(EtO)Si(CH_2)_3]S_x[(CH_2)_3Si(OEt)_3]$,
$[(C_{12}H_{25}O)_3Si(CH_2)_3]S_x[(CH_2)_3Si(OEt)_3]$,
$[(C_{12}H_{25}O)(EtO)_2Si(CH_2)_3]S_x[(CH_2)_3Si(C_{12}H_{25}O)(OEt)_2]$,
$[(C_{12}H_{25}O)_2(EtO)Si(CH_2)_3]S_x[(CH_2)_3Si(C_{12}H_{25}O)(OEt)_2]$,
$[(C_{12}H_{25}O)_3Si(CH_2)_3]S_x[(CH_2)_3Si(C_{12}H_{25}O)(OEt)_2]$,
$[(C_{12}H_{25}O)(EtO)_2Si(CH_2)_3]S_x[(CH_2)_3Si(C_{12}H_{25}O)_2(OEt)]$,
$[(C_{12}H_{25}O)_2(EtO)Si(CH_2)_3]S_x[(CH_2)_3Si(C_{12}H_{25}O)_2(OEt)]$,
$[(C_{12}H_{25}O)_3Si(CH_2)_3]S_x[(CH_2)_3Si(C_{12}H_{25}O)_2(OEt)]$,
$[(C_{12}H_{25}O)(EtO)_2Si(Ch_2)_3]S_x[(CH_2)_3Si(C_{12}H_{2S}O)3]$,
$[(C_{12}H_{25}O)_2(EtO)Si(CH_2)_3]S_x(CH_2)_3Si(C_{12}H_{25}O)_3]$,
$[(C_{12}H_{25}O)_3Si(CH_2)_3]S_x[(CH_2)_3Si(C_{12}H_{25}O)_3]$,
$(C_{14}H_{29}O)(EtO)_2Si(CH_2)_3]S_x[(CH_2)_3Si(OEt)_3]$,
$[(C_{14}H_{25}O)_2(EtO)Si(CH_2)_3]S_x[(CH_2)_3Si(OEt)_3]$,
$[(C_{14}H_{29}O)_3Si(CH_2)_3]S_x[(CH_2)_3Si(OEt)_3]$,
$[(C_{14}H_{29}O)(EtO)_2Si(CH_2)_3]S_x[(CH_2)_3Si(C_{14}H_{29}O)(OEt)_2]$,
$[(C_{14}H_{29}O)_2(EtO)Si(CH_2)_3]S_x[(CH_2)_3Si(C_{14}H_{29}O)(OEt)_2]$,
$[(C_{14}H_{29}O)_3Si(CH_2)_3]S_x[(CH_2)_3Si(C_{14}H_{29}O)(OEt)_2]$,
$[(C_{14}H_{29}O)(EtO)_2Si(CH_2)_3]S_x[(Ch_2)_3Si(C_{14}H_{29}O)_2(OEt)]$,
$[(C_{14}H_{29}O)_2(EtO)Si(CH_2)_3]S_x[(CH_2)_3Si(C_{14}H_{29}O)_2(OEt)]$,
$[(C_{14}H_{29}O)_3Si(CH_2)_3]S_x[(CH_2)_3Si(C_{14}H_{29}O)_2(OEt)]$,
$[(C_{14}H_{29}O)(EtO)_2Si(CH_2)_3]S_x[(CH_2)_3Si(C_{14}H_{29}O)_3]$,
$[(C_{14}H_{29}O)_2(EtO)Si(CH_2)_3]S_x[(CH_2)_3Si(C_{14}H_{29}O)_3]$,

$[(C_{14}H_{29}O)_3Si(CH_2)_3]S_x[(CH_2)_3Si(C_{14}H_{29}O)_3]$,
$(C_{16}H_{33}O)(EtO)_2Si(CH)_3]S_x[(CH_2)_3Si(OEt)_3]$,
$[(C_{16}H_{33}O)_2(EtO)Si(CH)_3]S_x[(CH_2)_3Si(OEt)_3]$,
$[(C_{16}H_{33}O)_3Si(CH_2)_3]S_x[(CH_2)_3Si(OEt)_3]$,
$[(C_{16}H_{33}O)(EtO)_2Si(CH_2)_3]S_x[(CH_2)_3Si(C_{16}H_{33}O)(OEt)_2]$,
$[(C_{19}H_{33}O)_2(EtO)Si(CH_2)_3]S_x[(CH_2)_3Si(C_{16}H_{33}O)(OEt)_2]$,
$[(C_{16}H_{33}O)_3Si(CH_2)_3]S_x[(CH_2)_3Si(C_{16}H_{33}O)(OEt)_2]$,
$[(C_{16}H_{33}O)(EtO)_2Si(CH_2)_3]S_x[(CH_2)_3Si(C_{16}H_{33}O)_2(OEt)]$,
$[(C_{16}H_{33}O)_2(EtO)Si(CH_2)_3]S_x[(CH_2)_3Si(C_{16}H_{33}O)_2(OEt)]$,
$[(C_{16}H_{33}O)_3Si(CH_2)_3]S_x[(CH_2)_3Si(C_{16}H_{33}O)_2(OEt)]$,
$[(C_{16}H_{33}O)(EtO)_2Si(CH_2)_3]S_x[(CH_2)_3Si(C_{16}H_{33}O)_3]$,
$[(C_{16}H_{33}O)_2(EtO)Si(CH_2)_3]S_x[(CH_2)_3Si(C_{16}H_{33}O)_3]$,
$[(C_{16}H_{33}O)_3Si(CH_2)_3]S_x[(CH_2)_3Si(C_{16}H_{33}O)_3]$,
$(C_{18}H_{37}O)(EtO)_2Si(CH_2)_3]S_x[(CH_2)_3Si(OEt)_3]$,
$[(C_{18}H_{37}O)_2(EtO)Si(CH_2)_3]S_x[(CH_2)_3Si(OEt)_3]$,
$[(C_{16}H_{37}O)_3Si(CH_2)_3]S_x[(CH_2)_3Si(OEt)_3]$,
$[(C_{18}H_{37}O)(EtO)_2Si(CH_2)_3]S_x[(CH_2)_3Si(C_{18}H_{37}O)(OEt)_2]$,
$[(C_{18}H_{37}O)_2(EtO)Si(CH_2)_3]S_x[(CH_2)_3Si(C_{18}H_{37}O)(OEt)_2]$,
$[(C_{18}H_{37}O)_3Si(C_H2)_3]S_x[(CH_2)_3Si(C_{18}H_{37}O)(OEt)_2]$,
$[(C_{18}H_{37}O)(EtO)_2Si(CH_2)_3S_x[(CH_2)_3Si(C_{18}H_{37}O)_2(OEt)]$,
$[(C_{18}H_{37}O)_2(EtO)Si(CH_2)_3]S_x[(CH_2)_3Si(C_{18}H_{37}O)_2(OEt)]$,
$[(C_{18}H_{37}O)_3Si(CH_2)_3]S_x[(CH_2)3Si(C_{18}H_{37}O)_2(OEt)]$,
$[(C_{18}H_{37}O)(EtO)_2Si(CH)_3]S_x[(CH_2)_3Si(C_{18}H_{37}O)_3]$,
$[(C_{19}H_{37}O)_2(EtO)Si(CH)_3]S_x[(CH_2)_3Si(C_{18}H_{37}O)_3]$,
$[(C_{18}H_{37}O)_3Si(CH_2)_3]S_x[(CH_2)_3Si(C_{18}H_{37}O)_3]$,

oder auch generell

$[(C_yH_{yx+1}O)(R)_2Si(CH)_3]S_x[(CH_2)_3Si(R)_3]$,
$[(C_yH_{2y+1}O)_2(R)Si(CH_2)_3]S_x[(CH_2)_3Si(R)_3]$,
$[(C_yH_{2y+1}O)_3Si(CH_2)_3]S_x[(CH_2)_3Si(R)_3]$,
$[(C_yH_{2y+1}O)(R)_2Si(CH_2)_3]S_x[(CH_2)_3Si(C_yH_{2y+1}O)(R)_2]$,
$[(C_yH_{2y+1}O)_2(R)Si(CH_2)_3]S_x[(CH_2)_3Si(C_yH_{2y+1}O)(R)_2]$,
$[(C_yH_{2y+1}O)_3Si(CH_2)_3]S_x[(CH_2)_3Si(C_yH_{2y+1}O)(R)_2]$,
$[(C_yH_{2y+1}O)(R)_2Si(CH_2)_3]S_x[(CH_2)_3Si(C_yH_{2y+1}O)_2(R)]$,
$[(C_yH_{2y+1}O)_2(R)Si(CH_2)_3]S_x[(CH_2)_3Si(C_yH_{2y+1}O)_2(R)]$,
$[(C_yH_{2y+1}O)_3Si(CH_2)_3S_x[(CH_2)_3Si(C_yH_{2y+1}O)_2(R)]$,
$[(C_yH_{2y+1}O)(R)_2Si(CH_2)_3S_x[(CH_2)_3Si(C_yH_{2y+1}O)_3]$,
$[(C_yH_{2y+1}O)_2(R)Si(CH_2)_3S_x[(CH_2)_3Si(C_yH_{2y+1}O)_3]$ oder
$(C_yH_{2y+1}O)_3Si(CH_2)_3S_x[(CH_2)_3Si(C_yH_{2y+1}O)_3]$,

mit $x = 1-14$, $y = 10-24$ und $R = (MeO)$ oder/und $(EtO)$, oder
Mischungen der einzelnen, oben benannten Silane.

Als Silan können auch solche Verbindungen, wie beschrieben in DE 198 44 607 verwendet werden.

Als Silan kann man eine Organosiliziumverbindung oder Mischungen von Organosiliziumverbindungen der allgemeinen Formel (II)

$$X^1X^2X^3Si\text{-}A\text{-}S\text{-}SiR^1R^2R^3 \qquad (II)$$

verwenden, in der

$X^1$, $X^2$, $X^3$ und A unabhängig voneinander dieselbe Bedeutung wie in Formel (I) haben,

$R^1$, $R^2$, $R^3$ jeweils unabhängig voneinander sind und $(C_1\text{-}C_{16})$ Alkyl, vorzugsweise $(C_1\text{-}C_4)$ Alkyl, besonders bevorzugt Methyl- und Ethyl-,

$(C_1\text{-}C_{16})$ Alkoxy, vorzugsweise $(C_1\text{-}C_4)$ Alkoxy, besonders bevorzugt Methoxy und Ethoxy,

$(C_1\text{-}C_{16})$ Haloalkyl, Aryl, $(C_7\text{-}C_{16})$ Aralkyl, -H, Halogen oder $X^1X^2X^3Si\text{-}A\text{-}S\text{-}$ bedeuten.

Als Silan der allgemeinen Formel (II) kann man beispielsweise folgende Verbindungen verwenden:

$(EtO)_3\text{-}Si\text{-}(CH_2)_3\text{-}S\text{-}Si(CH3)3$, $[(EtO)_3\text{-}Si\text{-}(CH_2)_3\text{-}S]_2Si(CH_3)_2$,

$[(EtO)_3-Si-(CH_2)_3-S]_3Si(CH_3)$, $[(EtO)_3-Si-(CH_2)_3-S]_2Si(OEt)_2$,
$[(EtO)_3-Si-(CH_2)_3-S]_4Si$, $(EtO)_3-Si-(CH_2)_3-S-Si(OEt)_3$,
$(MeO)_3-Si-(CH_2)_3-S-Si(C_2H_5)_3$, $[(MeO)_3-Si-(CH_2)_3-S]_2Si(C_2H_5)_2$,
$[(MeO)_3-Si-(CH_2)_3-S]_3Si(CH_3)$, $[MeO)_3-Si-(CH_2)_3-S]_2Si(OMe)_2$,
$[(MeO)_3-Si-(CH_2)_3-S]_4Si$, $(MeO)_3-Si-(CH_2)_3-S-Si(OMe)_3$,
$(EtO)_3-Si-(CH_2)_2-CH(CH_3)-S-Si(CH_3)_3$,
$(EtO)_3-Si-(CH_2)_2-CH(CH_3)-S-Si(C_2H_5)_3$,
$(EtO)_3-Si-(CH_2)_2-CH(CH_3)-S-Si(C_6H_5)_3$ oder
$(EtO)_3-Si-(CH_2)_2(p-C_6H_4)-S-Si(CH_3)_3$.

Als Silan kann man eine Organosiliziumverbindung oder Mischungen von Organosiliziumverbindungen der allgemeinen Formel (III)

$$X^1X^2X^3Si\text{-Alk} \qquad (III)$$

verwenden, in der
$X^1$, $X^2$ und $X^3$ jeweils unabhängig voneinander dieselbe Bedeutung wie in Formel (I) haben und
Alk ein geradkettiges, verzweigtes oder zyklisches $(C_1-C_{18})$Alkyl, beispielsweise Methyl-, Ethyl-, n-Propyl, n-Butyl, n-Pentyl, n-Hexyl, Isopropyl oder tert.-Butyl, $(C_1-C_5)$ Alkoxy, beispielsweise Methoxy-, Ethoxy-, Propoxy-, Butoxy-, Isopropoxy-, Isobutoxy-, t-Butoxy- oder Pentoxy, Halogen, beispielsweise Fluor, Chlor, Brom oder Jod, Hydroxy, Thiol, Nitril, $(C_1-C_4)$ Haloalkyl, $-NO_2$, $(C_1-C_8)$ Thioalkyl, $-NH_2$, $-NHR^1$, $-NR^1R^2$, $NH(SiX^1X^2X^3)$, Alkenyl, Allyl-, Vinyl-, Aryl oder $(C_7-C_{16})$ Aralkyl ist.

Unter der Bezeichnung Alkenyl- können die Vinylgruppe sowie geradkettige, verzweigte oder zyklische Fragmente zusammengefaßt werden, die eine oder mehrere Kohlenstoffdoppelbindungen enthalten können.

Unter der Bezeichnung zyklische Alkyl- oder Alkenylfragmente können sowohl monocyclische als auch bicyclische oder polyzyklische Strukturen, sowie zyklische Strukturen, die mit Alkylsubstituenten, beispielsweise Norbornyl-, Norbornenyl-, Ethylnorbornyl-, Ethylnorbornenyl-, Ethylcyclohexyl-, Ethylcyclohexenyl-, oder Cyclohexylcyclohexyl-Gruppen versehen sind, zusammengefaßt werden.

Unter Aryl können Phenyle, Biphenyle oder sonstige benzoide Verbindungen verstanden werden, die gegebenenfalls mit $(C_1-C_3)$Alkyl-, $(C_1-C_3)$Alkoxy-, Halogen-, Hydroxy- oder mit Heteroatomen, wie $NR^1R^2OR^1$, $PR^1R^2R^3$, SH oder $SR^1$ substituiert sind.

Als Silan der allgemeinen Formel (III) kann man beispielsweise folgende Verbindungen verwenden:

$(C_{12}H_{25}O)_3-Si-(CH_2)_{16}-H$, $(C_{14}H_{29}O)_3-Si-(CH_2)_{16}-H$,
$(C_{16}H_{33}O)_3-Si-(CH_2)_{16}-H$, $(C_{18}H_{37}O)_3-Si-(CH_2)_{16}-H$, $(EtO)_3-Si-(CH_2)_3-H$,
$(MeO)_3-Si-(CH2)_3-H$, $(EtO)_3-Si-C(CH_3)_3$, $(MeO)_3-Si-C(CH_3)_3$,
$(EtO)_3-Si-(CH_2)_8-H$, $(MeO)_3-Si-(CH_2)_8-H$, $(EtO)_3-Si-(CH_2)_{16}-H$,
$(MeO)_3-Si-(CH_2)_{16}-H$, $(Me)_3Si-(OMe)$, $((Et)_3Si-(OMe)$,
$(C_3H_7)_3Si-(OMe)$, $(C_6H_5)_3Si-(OMe)$, $(Me)_3Si-(OEt)$, $((Et)_3Si-(OEt)$,
$(C_3H_7)_3Si-(OEt)$, $(C_6H_5)_3Si-(OEt)$, $(Me)_3Si-(OC_3H_7)$,
$(Et)_3Si-(OC_3H_7)$, $(C_3H_7)_3Si-(OC_3H_7)$, $(C_6H_5)_3Si-(OC_3H_7)$, $(Me)_3SiCl$,
$(Et)_3SiCl$, $(C_3H_7)_3SiCl$, $(C_6H_5)_3SiCl$, $Cl_3-Si-CH_2-CH=CH_2$, $(MeO)_3-SiCH_2-CH=CH_2$, $(EtO)_3-Si-CH_2-CH=CH_2$,
$(EtO)_3-Si-CH_2-CH=CH_2$ $Cl_3-SiCH=CH_2$, $(MeO)_3-Si-CH=CH_2$, $(EtO)_3-Si-CH=CH_2$, $(Me_3Si)_2N-C(O)-H$ oder
$(Me_3Si)_2N-H$.

Als Silan kann man eine Organosiliziumverbindung oder eine Mischung von Organosiliziumverbindungen der allgemeinen Formeln (IV) oder (V)

$$[[(ROC(=O))_p-(G)_j]_k-Y-S]_r-G-(SiX^1X^2X^3)_s \qquad (IV)$$

$$[(X^1X^2X^3Si)_q-G]_a-[Y-[S-G-SiX^1X^2X^3]_b]_c \qquad (V)$$

verwenden,
in der Y eine polyvalente Spezies $(Q)_zD(=E)$ darstellt, wobei folgendes gilt:
p ist 0 bis 5, r ist 1 bis 3, z ist 0 bis 2, q ist 0 bis 6, a ist 0 bis 7, b ist 1 bis 3, j ist 0 bis 1, aber es kann, wenn p = 1 auch häufig 0 sein, c ist 1 bis 6, bevorzugt 1 bis 4, t ist 0 bis 5, s ist 1 bis 3, k ist 1 bis 2, unter der Voraussetzung, daß

(1) falls (D) ein Kohlenstoff, Schwefel oder Sulfonyl ist, gilt, daß a + b = 2 und k = 1,

(2) falls (D) ein Phosphoratom ist, gilt, daß a + b = 3 solange c ≥ 1 und b = 1, wobei a = c + 1,

(3) falls (D) ein Phosphoratom ist, gilt, daß k = 2, ist,

Y eine polyvalente Spezies $(Q)_z D(=E)$ darstellt, bevorzugt - C(=NR)-, -SC(=NR)-, -SC(=O)-, (-NR)C(=O)-, (-NR)C(=S)-,-OC(=O)-, -OC(=S)-, -C(=O)-, -SC(=S)-, -C(=S)-, -S(=O)-,-S(=O)$_2$-,-OS(=O)$_2$-, -(NR)S(=O)$_2$-, -SS(=O)-, -OS(=O)-, (NR)S(=O), -SS(=O)$_2$-, (-S)$_2$P(=O)-, -(-S)P(=O)-, -P(=O) (-)$_2$, (-S)$_2$P(=S)-, -(-S)P(=S)-, -P(=S) (-)2, (-NR)$_2$P(=O)-, -(-NR) (-S)P(=O)-, (-O) (-NR)P(=O)-, (-O) (-S)P(=O)-, (-O)$_2$P(=O)-. -(-O)P(=O)-, -(-NR)P(=O)-, (-NR)$_2$P(=S)-, -(-NR) (-S)P(=S)-, (-O) (-NR)P(=S)-, (-O) (-S)P(=S)-, (-O)$_2$P(=S)-, -(-O)P(=S)-, oder -(-NR)P(=S)-,

in jeder dieser Gruppen ist das Atom (D) doppelt mit dem Heteroatom (E) verbunden, das wiederum mit dem Schwefelatom (S) verbunden ist, welches mittels einer Gruppe (G) mit dem Siliziumatom (Si) verknüpft ist,

$R^1$ unabhängig voneinander H,

eine gerade, zyklische oder verzweigte Alkylkette, vorzugsweise $(C_1-C_{18})$Alkyl, besonders bevorzugt $(C_1-C_4)$Alkyl bedeuten,

gegebenenfalls Alkylketten die ungesättigte Anteile wie Doppelbindungen (Alkene), Dreifachbindungen (Alkine) oder auch Alkylaromaten (Aralkyl) oder Aromaten beinhalten und die die gleichen Bedeutungen wie in Formel (II) aufweisen,

G unabhängig von den übrigen Substituenten, Wasserstoff, eine gerade, zyklische oder verzweigte Alkylkette mit $(C_1-C_{18})$ bedeutet, gegebenenfalls können die Alkylketten einen ungesättigten Anteil, wie Doppelbindungen (Alkene), Dreifachbindungen (Alkine) oder auch Alkylaromaten (Aralkyl) oder Aromaten beinhalten,

wenn p = 0 in Formel (IV) ist, ist G vorzugsweise Wasserstoff (H),

G entspricht nicht der Struktur eines α, β-ungesättigten Fragments, welches mit dem Y -Fragment in der Weise verbunden ist, daß ein α, β-ungesättigtes Thiocarbonylfragment entsteht, $X^1$, $X^2$ und $X^3$ jeweils unabhängig voneinander die Bedeutung wie in Formel (I) besitzen.

Ein Index p von 0 bis 2 ist bevorzugt, wobei $X^1$,$X^2$ oder $X^3$ ein RO- beispielsweise RC(=O)O- ist. Besonders bevorzugt ist ein Fragment mit p = 0, $X^1$, $X^2$ beispielsweise $X^3$ = Ethoxy- und mit G = Alkylgerüst beziehungsweise substituiertes Alkylgerüst mit $C_3$ bis $C_{12}$. Es kann mindestens ein X ungleich -$R^1$ sein.

In $(Q)_z D(=E)$ kann Q Sauerstoff, Schwefel oder (-NR-), D kann Kohlenstoff, Schwefel, Phosphor oder Sulfonyl, E kann Sauerstoff, Schwefel oder (=$NR^1$) sein.

Bevorzugte Beispiele für die Funktion (-YS-) in den Formeln (IV) und (V) sind:

Thiocarboxylatester -C(=O)-S-, Dithiocarboxylate -C(=S)-S-, Thiocarbonatester -O-C(=O)-S-, Dithiocarbonateester -S-C(=O)-S- und -O-C(=S)-S-, Trithiocarbonatester -S-C(=S)-S-, Dithiocarbamatester -N-C(=S)-S-, Thiosulfonateester -S(=O)$_2$-S-, Thiosulfatester -O-S(=O)$_2$-S-, Thiosulfamatester (-N-)S(=O)$_2$-S-, Thiosulfinatester -C-S(=O)-S-, Thiosulfitester -O-S(=O)-S-, Thiosulfimatester N-S(=O)-S-, Thiophosphatester P(=O)(O-)$_2$(S-), Dithiophosphatester P(=O) (O-)(S-)$_2$ oder P(=S) (O-)$_2$(S-), Trithiophosphatester P(=O) (S-)$_3$ oder P(=S)(O-)(S-)$_2$, Tetrathiophosphatester P(=S) (S-)$_3$, Thiophosphamtester -P(=O)(-N-)(S-), Dthiophosphamatester -P(=S)(-N-)(S-, Thiophosphoramidatester (-N-)P(=O)(O-)(S-), Dithiophosphoramidatester (-N-)P(=O) (S-)$_2$ oder (-N-)P(=S)(O-)(S-) oder Trithiophosphoramidatester (-N-)P(=S)(S-)$_2$.

Als Silan der allgemeinen Formel (IV) oder (V) kann man beispielsweise folgende Verbindungen verwenden:

2-Triethoxysilyl-1 -ethylthioacetat, 2-Trimethoxysilyl-1 - ethylthioacetat, 2-(Methyldimethoxysilyl)- 1 -ethylthioacetat, 3-Trimethoxysilyl-1-propylthioacetat, Triethoxysilylmethylthioacetat, Trimethoxysilylmethylthioacetat, Triisopropoxysilylmethylthioacetat, Methyldiethoxysilylmethylthioacetat, Methyldimethoxysilylmethylthioacetat, Methyldiisopropoxysilylmethylthioacetat, Dimethylethoxysilylmethylthioacetat, Dimethylmethoxysilylmethylthioacetat, Dimethylisopropoxysilylmethylthioacetat, 2-Triisopropoxysilyl-1 -ethylthioacetat, 2-(Methyldiethoxysilyl)- 1-ethylthioacetat, 2-(Methyldiisopropoxysilyl)- 1-ethylthioacetat, 2-(Dimethylethoxysilyl)- 1 -ethylthioacetat, 2-(Dimethylmethoxysilyl)-1 -ethylthioacetat, 2-(Dimethylisopropoxysilyl)-1 -ethylthioacetat, 3-Triethoxysilyl-1-propylthioacetat, 3-Triisopropoxysilyl-1-propylthioacetat, 3-Methyldiethoxysilyl-1-propylthioacetat, 3-Methyldimethoxysilyl-1-propylthioacetat, 3-Methyldiisopropoxysilyl-1-propylthioacetat, 1-(2-Triethoxysilyl-1-ethyl)-4-thioacetylcyclohexan, 1-(2-Triethoxysilyl-1-ethyl)-3-thioacetylcyclohexan, 2-Triethoxysilyl-5-thioacetylnorbornen, 2-Triethoxysilyl-4-thioacetylnorbomen, 2-(2-Triethoxysilyl-1-ethyl)-5-thioacetylnorbornen, 2-(2-Triethoxysilyl-1-ethyl)-4-thioacetylnorbornen, 1-(1 -oxo-2-thia-5-triethoxysilylpenyl)benzoesäure, 6-Triethoxysilyl-1-hexylthioacetat, 1-Triethoxysilyl-5-hexylthioacetat, 8-Triethoxysilyl-1-octylthioacetat, 1-Triethoxysilyl-7-octylthioacetat, 6-Triethoxysilyl-1-hexylthioacetat, 1-Triethoxysilyl-5-octylthioacetat, 8-Trimethoxysilyl-1-octylthioacetat, 1-Trimethoxysilyl-7-octylthioacetat, 10-Triethoxysilyl-1-decylthioacetat, 1-Triethoxysilyl-9-decylthioacetat, 1-triethoxysilyl-2-butylthioacetat, 1-Triethoxysilyl-3-butylthioacetat, 1-Triethoxysilyl-3-methyl-2-butylthioacetat, 1-Triethoxysilyl-3-methyl-3-butylthioacetat, 3-Trimethoxysilyl-1-propylthiooctoat, 3-Triethoxysilyl-1-propylthiopalmitat, 3-Triethoxysilyl-1-propylthiooctoat, 3-Triethoxysilyl-1-propylthiobenzoat, 3-Triethoxysilyl-1-propylthio-2-ethylhexanoat, 3 -Methyldiacetoxysilyl-1-propylthioacetat, 3-Triacetoxysilyl- 1 -propylthioacetat, 2-Methyldiacetoxysilyl- 1 -ethylthioacetat, 2-Triacetoxysilyl- 1 -ethylthioacetat, 1 -Methyldiacetoxysilyl- 1 -ethylthioacetat oder 1 -Triacetoxysilyl- 1 -ethylthioacetat.

Verbindungen der Formeln IV und V sind auch in EP0958298 oder WO9909036 beschrieben.

Als Silan kann man eine Organosiliziumverbindung oder eine Mischung von Organosiliziumverbindungen der allgemeinen Formel (VI)

$$X^1X^2X^3Si\text{-}A\text{-}Sub \qquad (VI)$$

verwenden, wobei $X^1$, $X^2$, $X^3$ und A, jeweils unabhängig voneinander, die Bedeutung gemäß Formel (I) haben und Sub -SH, -NH$_2$, -NH (A-SiX$^1$X$^2$X$^3$) , -N (A-SiX$^1$X$^2$X$^3$)$_2$, O-C(O)-CMe=CH$_2$, oder -SCN ist.

Als Silan der allgemeinen Formel (VI) kann man beispielsweise folgende Verbindungen verwenden:

(MeO)$_3$Si-(CH$_2$)$_3$-SH, (MeO)$_3$Si-(CH2)$_3$-NH$_2$, (MeO)$_3$Si-(CH$_2$)$_3$-SCN, (MeO)$_3$Si-(CH$_2$)$_3$-O-C(O)CMe=CH$_2$,
(EtO)$_3$Si-(CH$_2$)$_3$-NH$_2$, (EtO)$_3$Si-(CH$_2$)$_3$-SH, (EtO)$_3$Si-(CH$_2$)$_3$-SCN, (EtO)$_3$Si-(CH$_2$)$_3$-O-C(O)CMe=CH$_2$,
(C$_3$H$_7$O)$_3$Si-(CH$_2$)$_3$-SH, (C$_3$H$_7$O)$_3$Si-(CH$_2$)$_3$-SCN, (C$_3$H$_7$O)$_3$Si-(CH$_2$)$_3$-O-C(O)CMe=CH$_2$, (C$_3$H$_7$O)$_3$Si-(CH$_2$)$_3$-NH$_2$,
[(MeO)$_3$Si-(CH$_2$)$_3$-]$_2$NH, [(EtO)$_3$Si-(CH$_2$)$_3$-]$_2$NH, [(C$_3$H$_7$O)$_3$Si-(CH$_2$)$_3$]$_2$NH,
oder
(C$_{12}$H$_{25}$O)$_2$(MeO)-Si-(CH$_2$)$_3$-SH, (C$_{12}$H$_{25}$O)$_2$(EtO)-Si-(CH$_2$)$_3$-SH, (C$_{12}$H$_{25}$O)$_2$(C$_{14}$H$_{29}$O)-Si-(CH$_2$)$_3$-SH, (C$_{12}$H$_{25}$-O)$_2$(C$_{16}$H$_{33}$O)-Si-(CH$_2$)$_3$-SH, (C$_{12}$H$_{25}$O)$_2$(C$_{18}$H$_{37}$O)-Si-(CH$_2$)$_3$-SH, (C$_{14}$H$_{29}$O)$_2$(MeO) -Si- (CH$_2$)$_3$-SH, (C$_{14}$H$_{29O}$)$_2$(EtO)-Si-(CH$_2$)$_3$-SH, (C$_{14}$H$_{29}$O)$_2$(C$_{12}$H$_{25}$O)-Si-(CH$_2$)$_3$-SH, (C$_{14}$H$_{29}$O)$_2$(C$_{16}$H$_{33O}$)-Si-(CH$_2$)$_3$-SH, (C$_{14}$H$_{29}$O)$_2$(C$_{18}$H$_{37}$O)-Si-(CH$_2$)$_3$-SH, (C$_{16}$H$_{33}$O)$_2$(MeO)-Si-(CH$_2$)$_3$-SH, (C$_{16}$H$_{33}$O)$_2$(EtO)-Si-(CH$_2$)$_3$-SH, (C$_{16}$H$_{33}$O)$_2$(C$_{12-}$H$_{25}$O)-Si-(CH$_2$)$_3$-SH, (C$_{16}$H$_{33O}$)$_2$(C$_{14}$H$_{29}$O)-Si-(CH$_2$)$_3$-SH, (C$_{16}$H$_{33}$O)$_2$(C$_{18}$H$_{37}$O)-Si-(CH$_2$)$_3$-SH, (C$_{18}$H$_{37}$O)$_2$(MeO)-Si-(CH$_2$)$_3$-SH, (C$_{18}$H$_{37}$O)$_2$(EtO)-Si-(CH$_2$)$_3$-SH, (C$_{18}$H$_{37}$O)$_2$(C$_{12}$H$_{25}$O)-Si-(CH$_2$)$_3$-SH, (C$_{18}$H$_{37}$O)$_2$(C$_{14}$H$_{29}$O)-Si-(CH$_2$)$_3$-SH, (C$_{18}$H$_{37}$O)$_2$(C$_{16}$H$_{33}$O)-Si-(CH$_2$)$_3$-SH,
oder
(C$_{12}$H$_{25}$O)$_2$(MeO)-Si-(CH$_2$)$_3$-NH$_2$, (C$_{12}$H$_{25}$O)$_2$(EtO)-Si-(CH$_2$)$_3$-NH$_2$, (C$_{12}$H$_{25}$O)$_2$(C$_{14}$H$_{29}$O)-Si-(CH$_2$)$_3$-NH$_2$, (C$_{12-}$H$_{25}$O)$_2$(C$_{16}$H$_{33}$O)-Si-(CH$_2$)$_3$-NH$_2$, (C$_{12}$H$_{25}$O)$_2$(C$_{18}$H$_{37}$O)-Si-(CH$_2$)$_3$-NH$_2$, (C$_{14}$H$_{29}$O)$_2$(MeO)-Si-(CH$_2$)$_3$-NH$_2$, (C$_{14-}$H$_{29}$O)$_2$(EtO)-Si-(CH$_2$)$_3$-NH$_2$, (C$_{14}$H$_{29}$O)$_2$(C$_{12}$H$_{25}$O)-Si-(CH$_2$)$_3$-NH$_2$, (C$_{14}$H$_{29}$O)$_2$(C$_{16}$H$_{33}$O)-Si-(CH$_2$)$_3$-NH$_2$, (C$_{14-}$H$_{29}$O)$_2$(C$_{18}$H$_{37}$O)-Si-(CH$_2$)$_3$-NH2, (C$_{16}$H$_{33}$O)$_2$(MeO)-Si-(CH$_2$)$_3$-NH$_2$, (C$_{16}$H$_{33}$O)$_2$(EtO)-Si-(CH$_2$)$_3$-NH$_2$, (C$_{16}$H$_{33}$O)$_2$(C$_{12}$H$_{25}$O)-Sie-(CH$_2$)$_3$-NH$_2$, (C$_{16}$H$_{33}$O)$_2$(C$_{14}$H$_{29}$O)-Si-(CH$_2$)$_3$-NH$_2$, (C$_{16}$H$_{33}$O)$_2$(C$_{18}$H$_{37}$O)-Si- (CH$_2$)$_3$-NH$_2$, (C$_{18-}$H$_{37}$O)$_2$(MeO) -Si- (CH$_2$)$_3$-NH$_2$, (C$_{18}$H$_{37}$O)$_2$(EtO)-Si-(CH$_2$)$_3$-NH$_2$, (C$_{18}$H$_{37}$O)$_2$(C$_{12}$H$_{25}$O)-Si-(CH$_2$)$_3$-NH$_2$, (C$_{18}$H$_{37}$O)$_2$(C$_{14}$H$_{29}$O)-Si-(CH$_2$)$_3$-NH$_2$, (C$_{18}$H$_{37}$O)$_2$(C$_{16}$H$_{33}$O)-Si-(CH$_2$)$_3$-NH2,
oder
(C$_{12}$H$_{25}$O)$_2$(C$_{14}$H$_{29}$O)-Si-(CH$_2$)$_3$-SCN, (C$_{12}$H$_{25}$O)$_2$(C$_{16}$H$_{33}$O)-Si-(CH$_2$)$_3$-SCN, (C$_{12}$H$_{25}$O)$_2$(C$_{18}$H$_{37}$O)-Si- (C-H$_2$)$_3$-SCN, (C$_{14}$H$_{29}$O)$_2$(C$_{12}$H$_{25}$O)-Si-(CH$_2$)$_3$-SCN, (C$_{14}$H$_{29}$O)$_2$(C$_{16}$H$_{33}$O)-Si-(CH$_2$)$_3$-SCN, (C$_{14}$H$_{29}$O)$_2$(C$_{18}$H$_{37-}$O)-Si-(CH$_{-2}$)$_3$-SCN, (C$_{16}$H$_{33}$O)$_2$(C$_{12}$H$_{25}$O)-Si-(CH$_2$)$_3$-SCN, (C$_{16}$H$_{33}$O)$_2$(C$_{14}$H$_{29}$O)-Si-(CH$_2$)$_3$-SCN, (C$_{16}$H$_{33}$O)$_2$(C$_{18}$H$_{37}$O)-Si-(CH$_2$)$_3$-SCN, (C$_{18}$H$_{37}$O)$_2$(C$_{12}$H$_{25}$O)-Si-(CH$_2$)$_3$-SCN, (C$_{18}$H$_{37}$O)$_2$(C$_{14}$H$_{29}$O)-Si-(CH$_2$)$_3$-SCN, (C$_{18-}$H$_{37}$O)$_2$(C$_{16}$H$_{33}$O)-Si-(CH$_2$)$_3$-SCN,
oder
(C$_{12}$H$_{25}$O)$_2$(MeO)-Si-(CH$_2$)$_3$-O-C(O)CMe=CH$_2$, (C$_{12}$H$_{25}$O)$_2$(EtO)-Si-(CH$_2$)$_3$-O-C(O)CMe=CH$_2$, (C$_{14}$H$_{29}$O)$_2$(MeO)-Si-(CH$_2$)$_3$-O-C(O)CMe=CH$_2$, (C$_{14}$H$_{29}$O)$_2$(EtO)-Si-(CH$_2$)$_3$-O-C(O)CMe=CH$_2$, (C$_{16}$H$_{33}$O)$_2$(MeO)-Si-(CH$_2$)$_3$-O-C(O)CMe=CH$_2$, (C$_{16}$H$_{33}$O)$_2$(EtO)-Si-(CH$_2$)$_3$-O-C(O)CMe=CH$_2$, (C$_{18}$H$_{37}$O)$_2$(MeO)-Si-(CH$_2$)$_3$-O-C(O)CMe=CH$_2$, (C$_{18}$H$_{37}$O)$_2$(EtO)-Si-(CH$_2$)$_3$-O-C(O)CMe=CH$_2$,
oder
[(C$_y$H$_{2y+1}$O) (EtO)$_2$Si(CH$_2$)$_3$]-NH$_2$, [(C$_y$H$_{2y+1}$O)$_2$(EtO)Si(CH$_2$)$_3$]-NH$_2$, [(C$_y$H$_{2y+1}$O) (EtO)$_2$Si(CH$_2$)$_3$]-SH, [(C$_y$-H$_{2y+1}$O)$_2$(EtO)Si(CH$_2$)$_3$]-SH, (C$_y$H$_{2y+1}$O) (EtO)$_2$Si(CH$_2$)$_3$]-SCN, [(C$_y$H$_{2y+1}$O)$_2$(EtO)Si(CH$_2$)$_3$]-SCN, [(C$_y$H$_{2y+1}$O) (EtO)$_2$Si(CH$_2$)$_3$]-O-C(O)-CMe=CH$_2$, [(C$_y$H$_{2y+1}$O)$_2$(EtO)Si(CH$_2$)$_3$]-O-C(O)-CMe=CH$_2$, mit y = 10-24, oder Mischungen der oben genannten Silane.

Als Silan kann man Oligomere, das heisst Oligo- und Polysiloxane, oder Cooligomere der Silane der allgemeinen Formel (I)-(VI) oder deren Mischungen verwenden. Die Siloxane können durch Oligomerisierung oder Cooligomerisierung der entsprechenden Silanverbindungen der allgemeinen Formeln (I)-(VI) durch Wasserzugabe und dem Fachmann auf diesem Gebiet bekannte Additivzugabe erhalten werden.

Oligomere Silane sind zum Beispiel in EP 652 245 B1, EP 0 700 951 B1, EP 0 978 525 A2 und DE 199 29 021 A1 beschrieben.

Als Silane im Sinne der vorliegenden Erfindung zur Modifizierung von Füllstoffen können auch Mischungen von Silanen verwendet werden, beispielsweise Mischungen der Silane der allgemeinen Formel (I)-(VI) oder Mischungen der oligomeren oder polymeren Siloxane von Silanen der allgemeinen Formel (I)-(VI) oder Mischungen von Silanen der allgemeinen Formel (I)-(VI) mit Mischungen der oligomeren oder polymeren Siloxane von Silanen der allgemeinen Formel (I)-(VI).

Als unbehandelter mikrogeperlter oder mikrogranulärer oxidischer oder silikatischer Füllstoff kann ein natürlicher und/oder synthetischer Füllstoff verwendet werden.

Der mikrogeperlte oder mikrogranuläre oxidische oder silikatische Füllstoff kann mit den Kautschuken verträglich sein und die für diese Verwendung notwendige Feinteiligkeit und Verstärkungswirkung in der Polymermatrix aufweisen.

Als natürlicher, silikatischer Füllstoff kann Silikat, beispielsweise Kaolin, Mica, Kieselgur, Diatomeenerde, Talkum, Wollastonit oder Clay oder auch Silikate unter anderem in Form von Glaskugeln, gemahlenen Glassplittern (Glasmehl), Glasfasern oder Glasgeweben verwendet werden.

Als oxidische Füllstoffe können alle Arten von Metalloxiden, beispielsweise Aluminiumoxid, Aluminiumhydroxid oder - trihydrat, Zinkoxid, Boroxide, Magnesiumoxide oder auch Übergangsmetalloxide, wie Titandioxid, verwendet werden.

Als oxidischer oder silikatischer Füllstoff können des weiteren Aluminiumsilikate, Silikate, Zeolithe, gefällte Kieselsäuren mit BET-Oberflächen (gemessen mit gasförmigem Stickstoff) von 1 bis 1000 m$^2$/g, bevorzugt bis 300 m$^2$/g, verwendet werden.

Beispielsweise können die von der Degussa AG vertriebene Fällungskieselsäure Ultrasil 7005, sowie die von PPG Industries Inc. vertriebenen Kieselsäure Hi-Sil® 210 und die von der Rhodia vertriebenen Produkte Zeosil 1115 MP, Zeosil 1135 MP, Zeosil 1165 MP, Zeosil 1165 MPS oder Zeosil 1205 MP verwendet werden.

Auch Kieselsäuren anderer Hersteller, die ähnliche Eigenschaften beziehungsweise Produktcharakteristiken wie die oben genannten Kieselsäuren aufweisen oder ähnliche, vergleichbare analytischen Daten (speziell BET-Oberflächen, CTAB-Oberflächen, BET/CTAB Verhältnis, Searszahl, Perlfraktionen- oder Partikelgrößenverteilungen, Formfaktoren, Kreisformfaktoren und DBP Index) aufweisen, können problemlos zur Herstellung der silanmodifizierten oxidischen oder silikatischen Füllstoffs verwendet werden.

Als verdichtetes Gas können Verbindungen, die unter normalen Temperatur- und Druckbedingungen gasförmig und als Reaktionsmatrix für die Silan/Füllstoffmischungen geeignet sind, verwendet werden. Beispielsweise können Kohlendioxid, Helium, Stickstoff, Distickstoffmonoxid, Schwefelhexafluorid, gasförmige Alkane mit 1 bis 5 C-Atomen (Methan, Ethan, Propan, n-Butan, Isobutan, Neopentan), gasförmige Alkene mit 2 bis 4 C-Atomen (Ethylen, Propylen, Buten), gasförmige Alkine (Acetylen, Propin und Butin-1), gasförmige Diene (Propadien), gasförmige Fluorkohlenwasserstoffe, Chlor- und/oder Fluorchlorkohlenwasserstoffe (Freone, CKC, HCFC) oder deren aufgrund der laufenden Gesetzgebung verwendeten Ersatzstoffe oder Ammoniak, sowie Gemische dieser Stoffe verwendet werden.

Vorzugsweise kann als verdichtetes Gas Kohlendioxid verwendet werden, da es ungiftig, nicht brennbar, wenig reaktionsfähig und kostengünstig ist. Außerdem können die erforderlichen superkritischen Bedingungen bzw. nahe kritischen Bedingungen leicht erreicht werden, denn der kritische Druck beziehungsweise die kritische Temperatur liegen bei nur 73 bar und 31°C.

Verdichtete Gase können nach E.Stahl, K.W.Quirin, D.Gerard, "Verdichtete Gase zur Extraktion und Raffination", Springer-Verlag, Seite 12-13 definiert werden. Verdichtete Gase können überkritische Gase, kritische Gase oder Gase im verflüssigten Zustandsbereich sein.

Die erfindungsgemäße Verwendung eines verdichteten Gases ist überraschenderweise ausgesprochen vorteilhaft. Unerwartet gut werden zum Beispiel entsprechend der vorliegenden Erfindung handelsübliche, mikrogeperlte oder mikrogranulierte, oxidische oder silikatische Füllstoffe, und speziell Kieselsäuren, nicht nur an der Oberfläche oder im oberflächennahen Bereich sondern auch vergleichsweise homogen innerhalb einer Mikroperle oder Mikrogranulats silanisiert.

Aufgrund des hohen Löse- und Diffusionsvermögens, der niedrigen Viskosität und der Fähigkeit speziell organischen Silanen beziehungsweise organischen oligomeren beziehungsweise polymeren Siloxanen hohe Diffusionsgeschwindigkeiten im verdichteten Gas zu ermöglichen, eignen sich verdichtete Gase überraschend gut für das Imprägnieren von mikroporösen, meso- und makroporösen Feststoffen mit monomeren beziehungsweise oligomeren Silanverbindungen. Die Silanverbindungen können durch das verdichtete Gas in die Poren, Kanäle und auf die sogenannten "inneren Oberflächen" der mikrogeperlten oder mikrogranulierten porösen Füllstoffe transportiert werden. Dort werden sie dann chemisch oder/und physikalisch angebunden und immobilisiert.

Verdichtete Gase können vorteilhafterweise, da sie unter Normalbedingungen gasförmig vorliegen, nach der abgeschlossenen Silanisierung des Füllstoffs leicht von diesem abgetrennt werden und besitzen speziell im Fall von Kohlendioxid zudem kaum umweltgefährliches Potential, da sie im natürlichen Kohlenstoffkreislauf aufgehen oder leicht recycliert werden können. Das ist, im Vergleich zu bekannten Verfahren, ein bedeutender technischer Vorteil, da einerseits eine homogene Reaktionsmatrix durch das verdichtete Fluid analog zu bekannten organischen Lösungsmitteln gewährt wird, aber gleichzeitig ein aufwendiger Entfernungschritt, zum Beispiel die Entfernung eines Lösungsmittels im Vakuum unter thermischer Belastung, vermieden werden kann.

Das verdichtete Gas kann in einem luftdicht abgeschlossenen Raum oder Behälter unter Druck gesetzt werden, in der sich das zu behandelnde Material befindet. Während dieses Prozesses kann der Druck, im allgemeinen ausgehend von Atmosphärendruck, bis zum Arbeitsdruck des erfindungsgemäßen Verfahrens gesteigert werden.

Die verwendeten Silane können in dem verdichteten Gas ungelöst, teilweise oder auch vollständig gelöst vorliegen.

Der mikrogeperlte oder mikrogranulierte, oxidische oder silikatische Füllstoff und das Silan können zuerst durchmischt beziehungsweise in Kontakt gebracht und dann mit dem im verdichteten Zustand vorliegenden Gas gemischt beziehungsweise in Kontakt gebracht werden.

Der mikrogeperlte oder mikrogranulierte, oxidische oder silikatische Füllstoff kann erst mit dem im verdichteten Zustand vorliegenden Gas durchmischt beziehungsweise in Kontakt gebracht und dann mit dem Silan gemischt beziehungsweise in Kontakt gebracht werden.

Das Silan kann erst mit dem im verdichteten Zustand vorliegenden Gas durchmischt beziehungsweise in Kontakt gebracht und dann mit dem entsprechenden mikrogeperlten oder mikrogranulierten, oxidischen oder silikatischen Füllstoff gemischt beziehungsweise in Kontakt gebracht werden.

Unter "in Kontakt gebracht" kann verstanden werden, daß das genannte Material eingetaucht, benetzt oder bedeckt ist, gelöst oder ungelöst vorliegt, suspendiert beziehungsweise adsorbiert oder absorbiert vorliegt.

Das "in Kontakt bringen" kann beispielsweise in einem Behälter oder in einem hermetisch abgeschlossenen Raum hergestellt werden, in den der unmodifizierte Füllstoff, die Silankomponente und das potentiell in den verdichteten Zustand transformierbare Gas geeignet eingebracht wird.

Der Kontakt zwischen unmodifiziertem Füllstoff und Silankomponente kann dabei mittels verschiedener technischer Lösungen bewerkstelligt werden. Vorzugsweise kann dies durch ein geeignetes Mischaggregat mit innen angebrachter Flüssigkeitsdosierung, wie sie dem Fachmann auf diesem Gebiet gut bekannt sind, erfolgen. Dies können beispielsweise, aber nicht ausschließlich, Mischer sein, wie sie von den Firmen Drais, Eirich, Forberg, Gericke, Lödige, Ruberg angeboten werden.

Das Mischaggregat kann eine homogene, abriebarme Verteilung des verwendeten Silans auf dem mikrogeperlten oder mikrogranulierten, oxidischen oder silikatischen Füllstoff gewährleisten. Der Energieeintrag kann vorzugsweise gering sein. Es können Freifallmischer (zum Beispiel Trommelmischer) und Mischer mit rotierenden Werkzeugen und niedriger Partikelbeanspruchung (Froudezahl < 1) für diesen Zweck verwendet werden.

Der Kontakt zwischen homogen vermischter Silan- und Füllstoffkomponente mit dem potentiell in den verdichteten Zustand transformierbaren Gas kann beispielsweise in einem Behälter oder in einem hermetisch abgeschlossenen Raum hergestellt werden, in den das Gemisch aus Füllstoff und Silan geeignet eingebracht werden kann. Unter "Kontakt herstellen" kann verstanden werden, daß das genannte Material in das Imprägnierfluid eingetaucht wird und von diesem benetzt und bedeckt wird, vorzugsweise, daß der mikrogeperlte oder mikrogranulierte, oxidische oder silikatische Füllstoff vollständig eingetaucht ist, oder auch daß alle äußeren und inneren Oberflächen des mikrogeperlten oder mikrogranulierten, oxidischen oder silikatischen Füllstoffs mit dem verdichteten Gas in Kontakt sind.

Die Löslichkeit der Silankomponente in dem verdichteten Gas kann abhängig sein von der Art desselben, vom Druck und der Temperatur. Sie kann auch moduliert und optimiert werden, indem man Druck und Temperatur variiert, um die physikalischen Eigenschaften des verdichteten Gases einzustellen. In manchen Fällen kann die Konzentration des Silans in der als Reaktionsmedium verwendeten Lösung die Wirksamkeit der Behandlung beeinflussen.

Bei dem erfindungsgemäßen Verfahren können 10-250 Gew.-Teile mikrogeperlter oder mikrogranulärer, oxidischer oder silikatischer Füllstoff mit 0,1-50 Gew.-Teilen, vorzugsweise 0,5-15 Gew.-Teilen, Silan umgesetzt werden.

Bei dem erfindungsgemäßen Verfahren kann der Druck, der auch Arbeitsdruck genannt wird, im allgemeinen zwischen 1 und 500 bar, vorzugsweise zwischen 1 und 200 bar, besonders bevorzugt zwischen 1 und 150 bar, sein.

Die Temperatur (Arbeitstemperatur), bei dem das Verfahren durchgeführt werden kann, liegt zwischen 0 und 300°C, vorzugsweise zwischen 0 und 200°C, besonders bevorzugt zwischen 0 und 130°C.

Die Reaktion kann man in einem typischen Reaktionsgefäß für Hochtemperatur-/Hochdruckreaktionen beziehungsweise Hochdruckextraktionen durchführen.

Der Druck kann während der Modifizierung auf unterschiedlichen Druckniveaus für Zeiträume von 5-720 min, vorzugsweise 5-240 min, besonders bevorzugt 5-30 min, konstant gehalten werden und der Füllstoff kann während dieser Zeit in dem verdichteten Gas eintauchen, von diesem durchflossen oder in diesem gerührt werden.

Dem mikrogeperlten oder mikrogranulierten, oxidischen oder silikatischen Füllstoff und/oder dem Silan kann vor der Umsetzung im verdichteten Gas Additive zugesetzt werden.

Der silanmodifizierte oxidische oder silikatische Füllstoff kann während der Umsetzung im verdichteten Gas mit zusätzlichen Additiven in Kontakt gebracht werden.

Während der Umsetzung des mikrogeperlten oder mikrogranulierten, oxidischen oder silikatischen Füllstoffs im verdichteten Gas, kann man in den zugeführten oder abgeführten Strom an verdichteten Gas der den silanmodifizierten oxidischen oder silikatischen Füllstoff durchströmt, zusätzlich Additive einbringen.

Als Additive können Ammoniak, Schwefeldioxid, Wasser, kurzkettige oder langkettige Alkohole, beispielsweise Methanol, Ethanol, Propanol, Butanol, Dodecanol, Tetradecanol, Hexadecanol, Octadecanol, oder andere Alkohole mit Molgewichten > 50 g/mol, kurzkettige oder langkettige Polyether oder Polyetheralkohole, beispielsweise Diethylenglycol, Triethylenglycol oder andere mit Molgewichten > 100 g/mol, kurzkettige oder langkettige Amine mit Molgewichten > 50 g/mol, Emulsionsbildner oder auch kurzkettige oder langkettige Silikonöle, beispielsweise Silikonöle mit Molgewichten > 100 g/mol, oder Mischungen der oben genannten Verbindungen verwendet werden. Der silanmodifizierte oxidische oder silikatische Füllstoff kann während der Modifizierungsreaktion mit weiteren Stoffen, zusätzlich zum verdichteten Gas oder verdichteten Gasgemischen, in Kontakt kommen.

Der mit dem Silan vermischte mikrogeperlte oder mikrogranulierte, oxidische oder silikatische Füllstoff kann kontinuierlich mit einem geeigneten Rühraggregat in der Hochdruckapparatur beziehungsweise dem Hochdruckgefäß umgewälzt werden. Dabei kann die Rührgeschwindigkeit der herrschenden Temperatur und dem dabei herrschenden Druck angepasst werden.

Als Rühraggregat können Hubrührer, Blattrührer, Balkenrührer, Lochbalkenrührer, Kreuzbalkenrührer, Ankerrührer, Gitterrührer, Schaufelwalze, Propellerrührer, Schneckenrührer, Turbinenrührer, Scheibenrührer, Planetenrührer, Kreiselmischer oder Impellerrührer verwendet werden.

Das Rühraggregat im Hochdruckgefäß kann mit 1-100 Umdrehungen, vorzugsweise 1-50 Umdrehungen, Hubbewegungen oder Umwälzungen pro Minute arbeiten.

Der mit einem Silan vermischte mikrogeperlte oder mikrogranuläre, oxidische oder silikatische Füllstoff kann während der Modifikationsreaktion kontinuierlich von einem verdichteten Gas benetzt und durchströmt werden, ohne dabei in dem Hochdruckgefäß umgewälzt zu werden beziehungsweise durch Rühraggregate weiter durchmischt zu werden.

Im Anschluß an die Oberflächenmodifizierung kann der silanmodifizierte oxidische oder silikatische Füllstoff eine Evakuierungs- oder Druckentspannungsstufe mit Abtrennung des verdichteten Gases und der zugesetzten Additive oder eines Teils der zugesetzten Additive vom Endprodukt umfassen.

Die Evakuierungs- oder Druckentspannungsstufe kann in einer Zeit zwischen 1 min und 180 min, vorzugsweise zwischen 1 min und 120 min, besonders bevorzugt zwischen 1 min und 60 min, durchgeführt werden.

Die Evakuierungs- oder Druckentspannungsstufe kann bei Temperaturen zwischen 1 und 300°C, vorzugsweise zwischen 1 und 200°C, besonders bevorzugt zwischen 1 und 150°C, und ganz besonders bevorzugt bei Temperaturen zwischen 1 und 130°C, durchgeführt werden.

Der silanmodifizierte oxidische oder silikatische Füllstoff kann einem zusätzlichen Kompaktierungs- oder Bearbeitungsschritt unterzogen werden.

Der silanmodifizierte oxidische oder silikatische Füllstoff kann in Farben, Lacken, Druckfarben, Beschichtungen, Coatings, Kleb- und Schmierstoffen, Kosmetika, Zahnpasten, Bauhilfsmitteln oder als Füllstoff in vulkanisierbaren Kautschuken, Siliconen oder Kunststoffen verwendet werden.

Kautschukmischungen können Kautschuk, den silanmodifizierten oxidischen oder silikatischen Füllstoff, gegebenenfalls gefällte Kieselsäure und / oder Ruß und / oder weitere Kautschukhilfsmittel enthalten.

Für die Herstellung der Kautschukmischungen können Naturkautschuk oder Synthesekautschuke verwendet werden. Bevorzugte Synthesekautschuke sind beispielsweise bei W. Hofmann, Kautschuktechnologie, Genter Verlag, Stuttgart 1980, beschrieben. Sie umfassen unter anderem Polybutadien (BR), Polyisopren (IR), Styrol/Butadien-Copolymerisate mit Styrolgehalten von 1 bis 60, vorzugsweise 5 bis 50 Gew.-% (E- oder S-SBR), Isobutylen/Isopren-Copolymerisate (IIR), Butadien/Acrylnitril-Copolymere mit Acrylnitrilgehalten von 5 bis 60, vorzugsweise 10 bis 50 Gew. -% (NBR), Chloropren (CR), Ethylen/Propylen/Dien-Copolymerisate (EPDM), sowie Mischungen dieser Kautschuke.

Die Kautschukmischungen können weitere Kautschukhilfsprodukte, wie beispielsweise Reaktionsbeschleuniger, -verzögerer, Alterungsschutzmittel, Stabilisatoren, Verarbeitungshilfsmittel, Weichmacher, Wachse, Metalloxide sowie Aktivatoren, wie Triethanolamin, Polyethylenglykol oder Hexantriol, organisch modifizierte Silane sowie andere Kautschukhilfsprodukte, die der Kautschukindustrie bekannt sind, enthalten.

Die Kautschukmischung kann zusätzlich Alkylsilane oder/und Silikonöle enthalten.

Die Kautschukhilfsmittel können in üblichen Mengen, die sich unter anderem nach dem Verwendungszweck richten, eingesetzt werden. Übliche Mengen sind zum Beispiel Mengen von 0,1 bis 50 Gew.-% bezogen auf Kautschuk.

Als Vernetzer können Schwefel, organische Schwefelspender oder Radikalbildner dienen. Die Kautschukmischungen können darüber hinaus Vulkanisationsbeschleuniger enthalten.

Beispiele für geeignete Vulkanisationsbeschleuniger sind Mercaptobenzthiazole, Sulfenamide, Guanidine, Thiurame, Dithiocarbamate, Thioharnstoffe und Thiocarbonate.

Die Vulkanisationsbeschleuniger und Vernetzer können in Mengen von 0,1 bis 10 Gew.-%, bevorzugt 0,1 bis 5 Gew.-%, bezogen auf Kautschuk, eingesetzt werden.

Die Abmischung der Kautschuke mit dem silanmodifizierten oxidischen oder silikatischen Füllstoff, gegebenenfalls mit gefällter Kieselsäure und / oder Ruß und / oder weiteren Kautschukhilfsmitteln kann in üblichen Mischaggregaten, wie Walzen, Innenmischern und Mischextrudern, durchgeführt werden. Üblicherweise können solche Kautschukmischungen in Innenmischern hergestellt werden, wobei zunächst in einer oder mehreren aufeinanderfolgenden thermomechanischen Mischstufen die Kautschuke, der silanmodifizierte oxidische oder silikatische Füllstoff, gegebenenfalls die gefällte Kieselsäure und / oder Ruß und / oder weitere Kautschukhilfsmittel bei 100 bis 170°C eingemischt werden. Dabei kann sich die Zugabereihenfolge und der Zugabezeitpunkt der Einzelkomponenten entscheidend auf die erhaltenen Mischungseigenschaften auswirken. Die so erhaltene Kautschukmischung kann dann in bekannter Weise in einem Innenmischer oder auf einer Walze bei 40-110°C mit den Vernetzungschemikalien versetzt und zur sogenannten Rohmischung für die nachfolgenden Prozeßschritte, wie zum Beispiel Formgebung und Vulkanisation, verarbeitet werden.

Die Vulkanisation der Kautschukmischungen kann bei Temperaturen von 80 bis 200°C, bevorzugt 130 bis 180°C, gegebenenfalls unter Druck von 10 bis 200 bar erfolgen.

Die Kautschukmischungen eignen sich zur Herstellung von Formkörpern aus Kautschuk, zum Beispiel für die Herstellung von Luftreifen für Personen- und Lastkraftwagen, Reifenlaufflächen für Personen- und Lastkraftwagen, Reifenbestandteilen für Personen- und Lastkraftwagen, wie zum Beispiel Seitenwand, Innenliner und Unterbau, Kabelmänteln, Schläuchen, Treibriemen, Förderbändern, Walzenbelägen, Fahrrad- und Motorradreifen und deren Bestanteilen, Schuhsolen, Dichtungsringen, Profilen und Dämpfungselementen.

Die silanmodifizierten oxidischen oder silikatischen Füllstoffe weisen gegenüber den rein physikalischen Abmischungen, zum Beispiel von Bis (3-triethoxysilylpropyl)tetrasulfan mit Kieselsäure (US-PS 4,076,550), den Vorteil einer hohen Lagerstabilität und damit Leistungsstabilität auf. Zudem weisen die Füllstoffe einen erheblich geringeren Gehalt an potentiell freisetzbaren Alkoholen, beispielsweise Methanol oder Ethanol, als physikalische Abmischungen von Silanen mit Füllstoffen auf, sind besser dispergierbar und weisen insgesamt für den Anwender in der kautschukverarbeitenden Industrie ein besseres verarbeitungstechnisches Verhalten (staubärmer, homogene Mischungsherstellung, Einsparen von Mischstufen und Mischzeiten, eigenschaftsstabile Mischungen nach der ersten Mischstufe) auf.

Die silanmodifizierten oxidischen oder silikatischen Füllstoffe weisen gegenüber bekannten silanmodifizierten Füllstoffen, zum Beispiel von Bis (3-triethoxysilylpropyl)tetrasulfan auf Kieselsäure (VP Coupsil 8108 von Degussa), den Vorteil einer höheren Lagerstabilität und damit höheren Leistungsstabilität auf. Zudem weisen die Füllstoffe einen im Vergleich erheblich geringeren Gehalt an potentiell freisetzbarem Alkohol, im allgemeinen Ethanol, auf, sind besser dispergierbar und weisen insgesamt für den Anwender in der kautschukverarbeitenden Industrie ein besseres verarbeitungstechnisches Verhalten (staubärmer, homogene Mischungsherstellung, Einsparen von Mischstufen und Mischzeiten) auf. Bei Lagerung werden weniger flüchtige organische Bestandteile (VOC) freigesetzt.

Gegenüber dem in situ-Verfahren und dem dabei notwendigen unbehandelten Füllstoff, besitzen die silanmodifizierten oxidischen oder silikatischen Füllstoffe die Vorteile eines verbesserten Wassergehaltes des behandelten Füllstoffs, einer geringeren Feuchtigkeitsaufnahme, sowie eines höheren Stampfgewichts, eines besseren Fließverhaltens und einer höheren Schüttdichte gegenüber dem unbehandeltem Füllstoff.

Während des Mischprozesses des in-situ-Verfahrens muss eine chemische Reaktion durchgeführt werden, bei der eine optimale Prozesskontrolle erforderlich ist, und durch die während der Silanisierungsreaktion erhebliche Mengen Alkohol freigesetzt werden. Diese entweichen in der Folge aus der Mischung und führen so zu Problemen in der Abluft. Dies wird bei der Verwendung der silanmodifizierten oxidischen oder silikatischen Füllstoffe verringert oder vermieden.

Mikrogeperlte oder mikrogranuläre Materialien besitzen meist höhere Schüttgutdichten, was sich auf die Wirtschaftlichkeit des Materialtransports von Rohstoff und Produkt positiv auswirkt. Die silanisierten mikrogeperlten oder mikrogranulären Füllstoffe besitzen ein ähnlich vorteilhaftes Fließ- und Förderverhalten im Vergleich zu pulverförmigen Kieselsäuren wie die als Ausgangsstoff verwendeten mikrogeperlten oder mikrogranulären Füllstoffe.

Beispiele:

Beispiele für die Herstellung silanmodifizierter oxidischer oder silikatischer Füllstoffe

Die nachfolgend aufgeführten Versuche werden in einer Hochdruck-Extraktionsanlage für Feststoffe mit einem Autoklavenvolumen von 50 1 durchgeführt.

Jeweils 8 kg gefällte Kieselsäure Ultrasil 7005 (Degussa AG; Analytische Kenngrößen: BET = 185 $m^2$/g gemäß ISO 5794/AnnexD, CTAB-Oberfläche = 173 $m^2$/g, Trocknungsverlust = 5,5 Gew.-% (DIN ISO787-2), werden in einem Rubergmischer mit 640 g Si69 (Degussa AG; Bis-(triethoxysilylpropyltetrasulfan)) physikalisch vorbelegt und vermischt. Anschließend werden in einigen Versuchen zusätzliche Mengen Wasser auf das Gemisch von Kieselsäure und Silan aufgesprüht.

Die physikalisch mit Si69 vorbelegte Kieselsäure wird in einen Einsatzbehälter (Volumen 35 1), der mit Sinterplatten oben und unten verschlossen wird, eingefüllt. Der vollständig gefüllte Einsatzbehälter wird in den Autoklaven einer Hochdruckextraktionsanlage eingesetzt (Festbett). Der Autoklav wird mit Hilfe einer Hochdruck-Membranpumpe auf Druck gebracht und gemäß den in den Tabellen 1-5 aufgeführten Drücken und Temperaturen in festgelegten Zeiten mit definierten Mengen Kohlendioxid, die von einer Hochdruckpumpe gefördert werden, durchströmt. Unter Primärre-

13

aktion versteht man die chemische und / oder physikalische Immobilisierung des Silans auf dem Füllstoff. Unter der Extraktion versteht man die teilweise / vollständige Hydrolyse des Silans und Entfernung des Alkohols. In einigen Beispielen (Tabellen 4 und 5) wird eine bestimmte Wassermenge in den $CO_2$-Strom vor Eintritt in den Autoklaven dosiert. Zusätzlich werden bei den Beispielen der Tabelle 5 Druckpulsationen zwischen 60 und 100 bar zur besseren Verteilung des Silans auf der Kieselsäureoberfläche durchgeführt. Das beladene Kohlendioxid wird nach dem Festbettextraktor einem Abscheider zugeführt, in dem es unter Druckabsenkung und/oder Temperaturerhöhung in den gasförmigen Zustand überführt wird, wobei die Löslichkeit für die Inhaltsstoffe des Fluids (zum Beispiel extrahiertes Ethanol) herabgesetzt und diese dadurch weitgehend abgeschieden werden. Nach dem Abscheider wird das gasförmige Kohlendioxid über einen Kühler kondensiert und einem Pufferbehälter zugeführt, von dem ausgehend es wieder von der Hochdruckpumpe angesaugt und zur Extraktion eingesetzt werden kann (Kreisprozess).

Bei den Beispielen 6 bis 9 (Tabelle 2) und 10 bis 15 (Tabelle 3) wird das Kohlendioxid unter den jeweils angegebenen Druck- und Temperaturbedingungen nicht zum Abscheider geführt, sondern für eine gewisse Zeit unter Beibehaltung von Druck und Temperatur über eine Bypass-Hochdruckpumpe umgewälzt, indem es direkt in einer Schleife wieder in den Autoklaven gefahren wird. Erst zur Durchführung der Extraktion wird der Durchfluss unter den angegebenen Bedingungen - wie in den Tabellen 2 + 3 angegeben - zum Abscheider geführt.

Um zu demonstrieren, daß die Durchflußrichtung für die Herstellung des Füllstoffs frei variiert werden kann, wird bei den Beispielen 10 bis 15 (Tabelle 3) die Richtung der Durchströmung mit Kohlendioxid gesplittet, das heißt: In den angegebenen Verhältnissen wird das Kohlendioxid wechselseitg von unten beziehungsweise von oben durch den Autoklaven gefördert, wobei Strömungsrichtung, durchgesetzte Menge Kohlendioxid sowie Druck- und Temperaturbedingungen, wie in der Tabelle 3 angegeben, eingehalten werden.

**Tabelle 1:**

| Beispiel Nr. | Kieselsäure | Silanbelegung in phf (Si69) | Primärreaktion | | | Extraktion | | |
|---|---|---|---|---|---|---|---|---|
| | | | Druck in bar | Temperatur in °C | Zeit in min | Druck in bar | Temperatur in °C | Kohlendioxiddurchsatz in kg |
| | | | | | | | | |
| | | | | | | | | |
| 1 | Ultrasil 7005 | 8.8 | 150 | durchschnittlich 65 | 80 | 270 | durchschnitt-lich 90 | 100 |
| | | | | | | | | |
| | | | | | | | | |
| 2 | Ultrasil 7005 | 9 | 200 | durchschnittlich 70 | 80 | 270 | durchschnittlich 90 | 90 |
| | | | | | | | | |
| | | | | | | | | |
| 3 | Ultrasil 7005 | 9,2 | 260 | durchschnittlich 70 | 80 | 270 | durchschnittlich 90 | 100 |
| | | | | | | | | |
| | | | | | | | | |
| 4 | Ultrasil 7005 | 9,0 | 200 | durchschnittlich 85 | 80 | 270 | durchschnittlich 90 | 90 |
| | | | | | | | | |
| | | | | | | | | |
| 5 | Ultrasil 7005 | 9,2 | 260 | durchschnittlieh 85 | 80 | 270 | durchschnittlich 90 | 90 |
| | | | | | | | | |

**Tabelle 2:**

| Beispiel Nr. | Kieselsäure | Silanbelegung phf (Si69) | Umwälzung | | | | | | | | Extraktion | | | |
|---|---|---|---|---|---|---|---|---|---|---|---|---|---|---|
| | | | Einspeisung | | | | Primärreaktion | | | | | | | |
| | | | Druck in bar | Temp. in °C | Zeit in min | $CO_2$-Durch-fluß. in kg | Druck in bar | Temp. in °C | Zeit in min | $CO_2$-Durchfluß. in kg | Druck in bar | Temp. in °C | Zeit in min | $CO_2$-Durchfluß. in kg |
| 6 | Ultrasil 7005 | 9 | 150 | 50 | 50 | 62 | 240 | 90 | 60 | 150 | 270 | 95 | 30 | 80 |
| 7 | Ultrasil 7005 | 9 | 160 | 40 | 50 | 63 | 290 | 90 | 90 | 200 | 270 | 90 | 30 | 90 |
| 8 | Ultrasil 7005 | 9,4 | 150 | 50 | 50 | 120 | 240 / 240 | 70 / 90 | 30 / 60 | 130 | 270 | 90 | 30 | 100 |
| 9 | Ultrasil 7005 | 9,4 | 170 | 70 | 50 | 80 | 210 | 90 | 90 | 135 | 270 | 90 | 30 | 100 |

**Tabelle 3:**

| Beispiel Nr. | Silanbelegung phf (Si69) | Umwälzung | | | | | | | | | | Extraktion | | | |
|---|---|---|---|---|---|---|---|---|---|---|---|---|---|---|---|
| | | Einspeisung | | | | | Primärreaktion | | | | | | | | |
| | | Druck in bar | Temp. in °C | Zeit in min | Durchfluß-richtung | $CO_2$-Durchfluß. in kg | Druck in bar | Temp. in °C | Zeit in min | Durchfluß-richtung | $CO_2$-Durchfluß. in kg | Druck in bar | Temp. in °C | Zeit in min | $CO_2$-Durchfuß. in kg |
| 10 | 9,4 | 150 | 50 | 55 | | 140 | 240 | 90 | 30 | ↓ | 60 | 270 | 90 | 30 | 100 |
| | | | | | | | 240 | 90 | 30 | ↑ | 60 | | | | |
| 11 | 9,4 | 150 | 50 | 12 | t | 30 | 240 | 90 | 10 | ↓ | 20 | 270 | 90 | 30 | 100 |
| | | 150 | 50 | 60 | ↓ | 150 | 240 | 90 | 10 | ↑ | 20 | | | | |
| | | | | | | | 240 | 90 | 20 | ↓ | 50 | | | | |
| | | | | | | | 240 | 90 | 20 | ↑ | 50 | | | | |
| 12 | 9,4 | 150 | 50 | 65 | ↓ | 74 | 260 | 95 | 65 | ↑ | 125 | 270 | 95 | 30 | 90 |
| 13 | 9,4 | 150 | 50 | 55 | ↓ | 70 | 290 | 90 | 10 | ↑ | 30 | 270 | 93 | 30 | 90 |
| | | 150 | 50 | 30 | t | 30 | 290 | 90 | 50 | ↓ | 95 | | | | |
| 14 | 9,4 | 150 | 50 | 55 | ↓ | 60 | 240 | 90 | 10 | ↑ | 25 | 270 | 90 | 30 | 90 |
| | | 150 | 50 | 55 | t | 30 | 240 | 90 | 45 | ↓ | 105 | | | | |

| Beispiel Nr. | Silanbelegung phf (Si69) | Umwälzung | | | | | | | | | | Extraktion | | | |
|---|---|---|---|---|---|---|---|---|---|---|---|---|---|---|---|
| | | Einspeisung | | | | | Primärreaktion | | | | | Druck in bar | Temp. in °C | Zeit in min | $CO_2$-Durchfuß. in kg |
| | | Druck in bar | Temp. in °C | Zeit in min | Durchfluß-richtung | $CO_2$-Durchfluß. in kg | Druck in bar | Temp. in °C | Zeit in min | Durchfluß-richtung | $CO_2$-Durchfluß. in kg | | | | |
| 15 | 9,4 | 150 | 50 | 35 | ↑ | 40 | 150 | 50 | 30 | ↓ | 50 | 270 | 90 | 30 | 90 |
| | | 150 | 50 | 35 | ↓ | 40 | 290 | 90 | 55 | ↑ | 125 | | | | |

EP 1 357 156 B1

**Tabelle 4:**

| Beispiel Nr. | Kieselsäure | Silanbelegung in phf (Si69) | Wasserzusatz in phf | Wasserzudosierung in sc-$CO_2$ | Druck in bar | Temperatur in °C | Zeit in min | Kohlendioxiddurchsatz in kg |
|---|---|---|---|---|---|---|---|---|
| | | | | | | | | |
| 16 | Ultrasil 7005 | 8 | 0 | | 100 | durchschnittlich 70 | 10 | |
| | | | | | | | | |
| | | | | | | | | |
| Extraktion | Ultrasil 7005 | | | + 0,2% $H_2O$ bez. auf $CO_2$ | 100 | 80-87 | 60 | 160 kg |
| | | | | | | | | |
| | | | | | | | | |
| 17 | Ultrasil 7005 | 8 | 5 | | 100 | durchschnitt- 70 | | |
| | | | | | | | | |
| | | | | | | | | |
| Extraktion | Ultrasil 7005 | | | +0,2% $H_2O$ bez. auf $CO_2$ | 100 | 80-87 | 60 | 160 kg |
| | | | | | | | | |
| | | | | | | | | |
| 18 | Ultrasil 7005 | 8 | 8 | | 100 | durchschnittlich 70 | 10 | |
| | | | | | | | | |
| | | | | | | | | |
| Extraktion | Ultrasil 7005 | | | + 0,2% $H_2O$ bez. auf $CO_2$ | 100 | 77-88 | 80 min | 160 kg |
| | | | | | | | | |

**Tabelle 5:**

| | Kieselsäure | Silanbelegung in phf (Si69) | Wasserzusatz in phf | Wasserzudosierung in sc-$CO_2$ | Druck in bar | Temperatur in °C | Zeit in min | Kohlendioxiddurchsatz in kg | Einspeisungsrichtung des $CO_2$ |
|---|---|---|---|---|---|---|---|---|---|
| 19 | Ultrasil 7005 | 8 | 0 | + 0, 2% $H_2O$ bez. auf $CO_2$ | 8 Druckpulsationen zwischen 60 und 100 bar | durchschnittlich 70 | 150 | | ↓ |
| Extraktion | Ultrasil 7005 | | | + 0,2% $H_2O$ bez. auf $CO_2$ | 100 | 85 | 60 | 160 kg | ↓ |
| 20 | Ultrasil 7005 | 8 | 5 | + 0,2% $H_2O$ bez. auf $CO_2$ | 8 Druckpulsationen zwischen 60 und 100 bar | durchschnittlich 70 | 150 | | ↓ |
| Extraktion | Ultrasil 7005 | | | + 0,2% $H_2O$ bez. auf $CO_2$ | 100 | 85 | 60 | 160 kg | ↓ |
| 21 | Ultrasil 7005 | 8 | 8 | + 0,2% $H_2O$ bez. auf $CO_2$ | 8 Druckpulsationen zwischen 60 und 100 bar | durchschnittlich 70 | 150 | | ↓ |
| Extraktion | Ultrasil 7005 | | | + 0,2% $H_2O$ | 100 | 85 | 60 | 160 kg | ↓ |

(fortgesetzt)

| | Kieselsäure | Silanbelegung in phf (Si69) | Wasserzusatz in phf | Wasserzudosierung in sc-$CO_2$ | Druck in bar | Temperatur in °C | Zeit in min | Kohlendioxiddurchsatz in kg | Einspeisungsrichtung des $CO_2$ |
|---|---|---|---|---|---|---|---|---|---|
| | | | | bez. auf $CO_2$ | | | | | |

21

Die Searszahlen werden in Anlehnung an G.W. Sears, Analyt. Chemistry 12 (1956) 1982 nach folgender Vorschrift bestimmt:

Der Füllstoff wird vor der Titration in einer Mühle vermahlen und dabei homogenisiert und zerkleinert.

2,5 g der so erhaltenen Probe werden in einem 250 ml Titriergefäß mit 60 ml Methanol versetzt und sobald der Feststoff vollständig benetzt ist weitere 40 ml Wasser zu der Suspension gegeben.

Mit einem Rührer (Ultra-Turrax) wird die Suspension für 30 sec dispergiert und anschließend mit weiteren 100 ml Wasser verdünnt. Die Suspension wird innerhalb von mindestens 20 Minuten auf 25°C temperiert.

Die Titration erfolgt folgendermaßen an einem Titroprozessor mit pH-Elektrode (z.B. DL 67, Mettler Toledo mit Elektrode DG 111 SC):

- zunächst 120 sec rühren;
- Suspension mit 0,1 N Kalilauge oder Salzsäure auf pH 6 einstellen;
- 20 ml NaCl-Lösung (250 g/l) zudosieren;
- Titration mit 0,1 N KOH von pH 6 bis pH 9;
- das Ergebnis wird umgerechnet auf 5 g Kieselsäure, also auf Verbrauch 0,1 N KOH in ml pro 5 g Kieselsäure, um von pH 6 den pH 9 zu erreichen.

Es handelt sich bei der vorliegenden Bestimmung um eine Weiterentwicklung, Präzisierung und Verbesserung des in G.W. Sears, Analyt. Chemistry 12 (1956) 1982 beschriebenen Verfahrens.

Die Proben werden für 15-20 h bei 105°C getrocknet und die BET-Oberfläche gemäß DIN 66131 (volumetrisches Verfahren) bestimmt.

Die Proben wurden für 15-20 h bei 105°C getrocknet und das Mikroporenvolumen nach der t-Plot-Methode gemäß DIN 66135-2 bestimmt.

Die Proben werden für 15-20 h bei 105°C getrocknet und die Mesoporenverteilung nach der BJH-Methode gemäß DIN 66134 bestimmt.

Das Makroporenvolumen (Poren mit Weiten > 30 bzw. > 50 nm) wird mit einem Quecksilberporosimeter Autopore II 9220 (Fa. Micromeritics) gemäß den allgemein bekannten Regeln und Bedienvorschriften im Bereich bis 400 $\mu$m bestimmt. Die Proben werden zuvor für 15-20 h bei 105°C getrocknet. Das Verfahren dient der Bestimmung des Porenvolumens und der Porenverteilung poröser Feststoffe durch Messung des unter steigendem Druck eingepressten Quecksilbervolumens nach dem Verfahren von Ritter und Drake gemäß DIN 66133.

Die Porenmaxima für Meso- und Makroporen können direkt aus den entsprechenden Diagrammen (kumuliertes Intrusionsvolumen (ml/g) beziehungsweise log. differentielles Porenvolumen dV/dlog D) für die Porenvolumenverteilung (ml/g) in Abhängigkeit vom Porendurchmesser ($\mu$m) abgelesen werden.

Die Bestimmung der Perlverteilung beziehungsweise der Perlfraktionen durch Siebanalyse wird folgendermaßen durchgeführt:

Bestimmt wird die Perlgrößenverteilung vorgeformter, granulierter, mikrogranulierter oder mikrogeperlter Kieselsäuren. Dazu wird eine bestimmte Menge Kieselsäure mit einem Stapel von Sieben unterschiedlicher, genormter Maschenweite aufgetrennt.

Durch Auswiegen wird der Anteil der einzelnen Perlfraktionen ermittelt. Die dafür verwendeten Geräte: Mech. Siebmaschine (Ro-tap); Präzisionswaage: Genauigkeit $\pm$ 0.01 g (Fa. Mettler)

Standardsiebe U.S. Standard No. 120, Höhe 25 mm, $\varnothing$: 200 mm; Maschenweiten: 300 $\mu$m (50 mesh); 150 $\mu$m (100 mesh); 75 $\mu$m (200 mesh)

Die Siebe und ein Auffangbehälter werden in der vorgesehenen Reihenfolge, das heisst mit von oben nach unten abnehmender Öffnungsweite, zusammengesteckt. Man wiegt 100 g der zu untersuchenden Probe ab, wobei eine passende Schaufel verwendet wird. Eine Vorauswahl des Materials durch Ausgießen oder Umgießen der geformten Kieselsäure aus dem Vorratsbehälter ist zu vermeiden. Nach der Überführung der abgewogenen Kieselsäure auf das oberste Sieb wird ein Deckel aufgesetzt und der Stapel so in die Siebmaschine eingesetzt, daß ein Spiel von ca. 1,5 mm verbleibt und die Siebe somit frei rotieren können.

Die Siebe werden in der Maschine befestigt und dann 5 min lang - mit dem Rüttler beziehungsweise Klopfwerk in Betrieb - geschüttelt. Danach nimmt man die Siebe nacheinander auseinander und wiegt die jeweils darin befindliche Kieselsäuremenge auf 0,1 g genau aus. Von jeder Probe wird eine Doppelbestimmung durchgeführt. Es wird jeweils der Mittelwert der in den einzelnen Sieben und im Auffangbehälter gefundenen Kieselsäuremengen in % angegeben.

Die Bestimmung der Partikelgrößenverteilung der Proben erfolgt durch Laserbeugungsanalyse ohne Ultraschallbehandlung mit einem Coulter LS 100 mit Dry Powder Modul (Fa. Beckman-Coulter) gemäß den allgemein bekannten Regeln und Bedienvorschriften. Für 60 sec. wird ein kontinuierlicher Strom an originalen, unbehandelten Partikeln der zu messenden Probe in einem Luftstrahl durch einen Laserstrahl geführt. Der Partikelstrom wird durchstrahlt und die unterschiedlichen Korngrößen (Partikelgrößen) detektiert und statistisch ausgewertet. Die messbare Partikelgröße beträgt minimal 0,4 $\mu$m und maximal 900 $\mu$m.

Die Bestimmung der Partikelgrößenverteilung nach Ultraschallbehandlung (Abbauverhalten der Proben) erfolgt durch Laserbeugungsanalyse mit einem Coulter LS 100 mit Mikrovolumenmodul (Fa. Beckman-Coulter) gemäß den allgemein bekannten Regeln und Bedienvorschriften, nachdem die Probe in Ethanol vordispergiert und für 60 sec. in einem geschlossenen Schraubdeckelglas in einem Ultraschallbad (US-Bad RK100, Fa. Bandelin) behandelt wird. Die messbare Partikelgröße beträgt minimal 0,4 $\mu$m und maximal 900 $\mu$m.

Für die Bestimmung des durchschnittlichen Schwefelgehaltes der Proben werden in den Autoklaveneinsätzen an beiden Enden des Einsatzes und in der Mitte Proben entnommen und ihr Schwefelgehalt gemäß bekannter Verfahren durch:

- Schöniger-Aufschluß in einer Sauerstoffatmosphäre (vgl. F. Ehrenberger, S. Gorbauch, "Methoden der organischen Elementar- und Spurenanalyse", Verlag Chemie GmbH, Weinheim/Bergstraße, 1973) und

- nachgeschalteter ionenchromatographischen Analyse (Ion-Chromatograph 690 der Fa. Metrohm; PRP X-100-Säule der Fa. Hamilton; Laufmittel: 2 mmol Salicylat-Puffer, pH 7) gemäß DIN ISO 10304-2 bestimmt.

Der durchschnittliche Schwefelgehalt der Gesamtprobe ergibt sich dann als arithmetisches Mittel aus den so ermittelten 3 Werten der Einzelproben.

Der Wassergehalt der Proben wird wie folgt bestimmt:

10 g der silanisierten Kieselsäure werden 15 Sekunden mit einer Kaffeemühle zerkleinert und anschließend der Wassergehalt gemäß den bekannten und dem Fachmann vertrauten Regeln mit einem Karl Fischer Titrator (Fa. Metrohm, 720 KFS Titrino) und den von Merck erhältlichen Karl-Fischer Titrationschemikalien Nr. 1.09241, Nr. 1.09243 und Nr. 1.06664 (Dinatriumtartrat-Dihydrat) bestimmt.

Der Kohlenstoffgehalt der Proben wird nach bekannten Standardverfahren mittels eines Carbon/Sulfur Determinator CS-244 der Fa. LECO bestimmt.

In Anlehnung an die in Kautschuk, Gummi, Kunststoffe 51, (1998) 525 von Hunsche et al. beschriebenen Arbeitsvorschrift wird der auf dem Füllstoff befindliche Restalkohol (Ethanol) folgendermaßen bestimmt:

In einer Glasampulle, die nach dem Befüllen mit einer dicht schließenden Kappe versehen wird, werden 1 g des Füllstoffs mit 10 ml Diethylenglycolmonobutylether (DEGMBE) und 0,3 ml 0,5 mol/l $H_2SO_4$ versetzt. Die Mischung wird für 20 min bei 60°C in einem Wasserbad in der Glasampulle durchmischt. Anschließend wird zu der zügig auf 25°C temperierten Mischung 10 ml Decan zugegeben. Aus der durchmischten, organischen Phase werden dann entsprechende Mengen zur HPLC-Analyse (HPLC Gerät mit Jasco Autosampler 851-AS, Pumpe Jasco PU 980, RI-Detektor 7515A; TiO2-Säule, 250x4,5 mm, 5 $\mu$m, YMC; mobile Phase: DEGMBE mit Cyclohexan; Temperatur 25°C) auf Ethanol entnommen.

Der Formfaktor und Kreisformfaktor der Proben wird wie folgt bestimmt:

An einem Rasterelektronenmikroskop Jeol JSM 6400 werden REM-Untersuchungen von aufgestäubten Pulvern der Füllstoffe durchgeführt und mittels der Bildanalysensoftware Analysis 3.2 der Fa. SIS (soft imaging software) on-line gemäß den dem Fachmann bekannten üblichen Regeln und Vorgehensweisen analysiert:

Kreisformfaktor (FCIRCLE)

Der Kreisformfaktor (FCIRCLE), gibt an, wie sehr die Partikelform von der idealen Kreisform abweicht.

$$FCIRCLE = \frac{4\pi\ (Area)}{P^2}$$

(1,0 für Kreis, <1 für längliche oder verzweigte Aggregate)
Area = Fläche eines Partikels, wird berechnet aus der Zahl der Pixel, die auf einen Partikel fallen und der Teilfläche eines Pixels
P = Perimeter (das heißt Umfang des mehr oder minder komplexen Partikels)

Formfaktor (FSHAPE)

$$\text{Formfaktor FSHAPE} = \frac{D\ MIN}{D\ MAX}$$

Der Formfaktor (FSHAPE), gibt an, wie sehr die Partikelform von der idealen Kreisform abweicht, indem 2 mögliche Durchmesser eines Partikels (D min, D max) betrachtet werden.
(1,0 für Kreis und andere exakt isometrische Aggregate, <1 für längliche Aggregate)
D MIN = minimaler Durchmesser eines betrachteten Partikels
D MAX = maximaler Durchmesser desselben betrachteten Partikels

**Tabelle 6:**

| Beispiel Nr. | Durchschnittlicher Ethanolgehalt | Searszahlen | BET-Oberfläche | Mikroporen, (d<2 nm) | Mesoporen Volumen (d = 2-30 nm) | Mesoporen Volumen (d = 2-50 nm) | Poren-maximum, Mesoporen | Makroporen, Volumen, (>30 nm) | Makroporen, Volumen, (>50 nm) | Poren-maximum, Makroporen | Wasser gehalt | Kohlenstoff gehalt | Schwefelgehalt (Durchschnitt) |
|---|---|---|---|---|---|---|---|---|---|---|---|---|---|
| | $\mu$mol/g | | m$^2$/g | ml/g | ml/g | ml/g | nm | ml/g | ml/g | $\mu$m | in Gew.% | in Gew.% | in Gew.% |
| | | | | | | | | | | | | | |
| Ultrasil 7005 | 0 | 23,9 | 185±3 | 0,02±0,01 | 0,41±0,03 | 0,87±0,06 | 19±4 | 3,26±0,2 | 2,91±0,2 | 130±10 | 6,7 | 0,06 | 0,2 |
| | | | | | | | | | | | | | |
| 1 | 412 | 20,5 | 144±3 | <0,01 | 0,36±0,03 | 0,79±0,06 | 18±4 | 2,89±0,2 | 2,59±0,2 | 110±10 | 2,64 | 2,15 | 1,7 |
| | | | | | | | | | | | | | |
| 2 | 428 | 20,1 | 140±3 | <0,01 | 0,33±0,02 | 0,74±0,06 | 17±4 | 2,85±0,2 | 2,56±0,2 | 120±10 | 2,15 | 2,45 | 1,8 |
| | | | | | | | | | | | | | |
| 3 | 440 | 21,7 | 149±3 | <0,01 | 0,33±0,02 | 0,74±0,06 | 17±4 | 2,85±0,2 | 2,56±0,2 | 120±10 | 2,15 | 2,45 | 1,8 |
| | | | | | | | | | | | | | |
| 4 | 458 | 12,9 | 145±3 | <0,01 | 0,39±0,03 | 0,82±0,06 | 18±4 | 2,81±0,2 | 2,53±0,2 | 150±10 | 2,07 | 2,55 | 1,95 |
| | | | | | | | | | | | | | |
| 5 | 463 | 13,3 | 144±3 | <0,01 | 0,37±0,03 | 0,77±0,06 | 17±4 | 2,89±0,2 | 2,58±0,2 | 120±10 | 2,00 | 2,50 | 1,95 |

**Tabelle 6.1**

| Beispiel Nr. | Partikelgrößenverteilung mittels Coulter LS 100 mit Mikrovolumenmodul nach Ultraschallbehandlung (X % der Partikel sind größer als Y µm) | | | | | | | | Perlgrößenverteilung durch Siebanalyse | | | |
|---|---|---|---|---|---|---|---|---|---|---|---|---|
| | Mittelwert | Medianwert | Maximum | X=5% | X=10% | X=50 % | X=90 % | X=95 % | >300 µm | 150 - 300 µm | 75-150 µm | <75 µm |
| | µm | µm | µm | Y µm | Y µm | Y µm | Y µm | Y µm | | | | |
| Ultrasil 7005 | 347,7 | 338,8 | 361,8 | 624,8 | 551,8 | 338,8 | 169,3 | 81,80 | 73,5 | 23,7 | 2,6 | 0,1 |
| 1 | 37,61 | 34,46 | 47,19 | 80,46 | 70,48 | 34,46 | 10,17 | 7,25 | 61,3 | 37,5 | 0,8 | 0,4 |
| 2 | 59,17 | 46,27 | 58,48 | 164,0 | 120 | 46,27 | 11,19 | 7,75 | 75,1 | 20,7 | 4 | 0,2 |
| 3 | 34,12 | 31,40 | 42,39 | 72,05 | 62,65 | 31,40 | 9,97 | 7,13 | 72,9 | 23,2 | 3,6 | 0,2 |
| 4 | 44,20 | 37,12 | 52,53 | 106,9 | 89,60 | 37,12 | 10,16 | 7,15 | 71,5 | 25,4 | 3 | 0,2 |
| 5 | 36,98 | 34,12 | 47,19 | 78,95 | 68,67 | 34,12 | 9,96 | 7,03 | 51,3 | 46,7 | 1,5 | 0,5 |

| Durchschnittlicher ...anolhalt | Searszahlen | BET-Oberfläche | Mikroporen, (d<2 nm) | Mesoporen Volumen, (d = 2-30 nm) | Mesoporen Volumen, (d = 2-50 nm) | Poren­maximum, Mesoporen | Makro­poren, Volumen, (d > 30 nm) | Makroporen, Volumen, (d > 50 nm) | Porenmaximum, Makroporen | Wassergehalt | Kohlenstoffgehalt | Schwefelgehalt (Durchschnitt) |
|---|---|---|---|---|---|---|---|---|---|---|---|---|
| ...ol/g | | m²/g | ml/g | ml/g | ml/g | nm | ml/g | ml/g | µm | in Gew.% | in Gew.% | in Gew.% |
| 0 | 23,9 | 185 ±3 | 0,02 ±0,01 | 0,41 ±0,03 | 0,87 ±0,06 | 19 ±4 | 3,26 ±0,2 | 2,91 ±0,2 | 130 ±10 | 6,7 | 0,06 | 0,2 |
| 399 | 21,7 | 140 ±3 | <0,01 | 0,35 ±0,02 | 0,75 ±0,06 | 20 ±4 | 2,81 ±0,2 | 2,46 ±0,1 | 90 ±10 | 3,29 | 2,00 | 2,2 |
| 402 | 19,5 | 142 ±3 | <0,01 | 0,33 ±0,02 | 0,68 ±0,06 | 20 ±4 | 2,79 ±0,2 | 2,45 ±0,1 | 90 ±10 | 3,26 | 2,10 | 1,9 |
| 435 | 20,7 | 140 ±3 | <0,01 | 0,35 ±0,02 | 0,73 ±0,06 | 20 ±4 | 2,83 ±0,2 | 2,47 ±0,1 | 90 ±10 | 3,16 | 2,20 | 2,1 |
| 282 | 16,2 | 155 ±3 | 0,01 ±0,01 | 0,36 ±0,02 | 0,76 ±0,06 | 20 ±4 | 3 ±0,2 | 2,63 ±0,2 | 130 ±20 | 3,79 | 1,73 | 2,0 |

**Tabelle 7.1**

| Beispiel Nr. | Partikelgroßenverteilung mittels Coulter LS 100 mit Mikrovolumenmodul nach Ultraschallbehandlung (X % der Partikel sind größer als Y μm) | | | | | | | | Perlgrößenverteilung durch Siebanalyse | | | |
|---|---|---|---|---|---|---|---|---|---|---|---|---|
| | Mittel-wert | Median-wert | Maximum | X = 5 % | X = 10 % | X = 50 % | X = 90 % | X = 95 % | >300 μm | 150 - 300 μm | 75-150 μm | <75 μm |
| | μm | μm | μm | Y μm | Y μm | Y μm | Y μm | Y μm | | | | |
| Ultrasil | 347,7 | 338,8 | 361,8 | 624,8 | 551,8 | 338,8 | 169,3 | 81,80 | 73,5 | 23,7 | 2,6 | 0,1 |
| 6 | 306,9 | 300,5 | 325,0 | 538,0 | 476,6 | 300,5 | 153,5 | 66,17 | 44,7 | 52,3 | 2,3 | 0,6 |
| 7 | 258,3 | 259,8 | 291,9 | 465,8 | 402,0 | 259,8 | 102,6 | 31,30 | 59 | 40 | 1 | 0 |
| 8 | 249,8 | 245,4 | 262,3 | 462,3 | 402,9 | 245,4 | 77,30 | 23,56 | 53,6 | 44,7 | 0,8 | 1 |
| 9 | 213,6 | 208,7 | 235,6 | 410,6 | 356,8 | 208,7 | 70,80 | 18,62 | 50,9 | 47,2 | 1,6 | 0,3 |

**B:**

| Durch-schnitt-licher Ethanol-gehalt (µmol/g) | Sears-zahlen | BET-Ober-fläche (m²/g) | Mikro-poren, (d<2 nm) (ml/g) | Mesoporen Volumen, (d = 2-30 nm) (ml/g) | Mesoporen Volumen, (d = 2-50 nm) (ml/g) | Poren-maximum, Mesoporen (nm) | Makro-poren, Volumen, (d > 30 nm) (ml/g) | Makro-poren, Volumen, (d > 50 nm) (ml/g) | Poren-maximum, Makro-poren (nm) | Wasser-gehalt (in Gew.%) | Kohlen-stoff-gehalt (in Gew.%) | Schwefel-gehalt (Durch-schnitt) (in Gew.%) |
|---|---|---|---|---|---|---|---|---|---|---|---|---|
| 0 | 23,9 | 185 ±3 | 0,02 ±0,01 | 0,41 ±0,03 | 0,87 ±0,06 | 19 ±4 | 3,26 ±0,2 | 2,91 ±0,2 | 130 ±10 | 6,7 | 0,06 | 0,2 |
| 476 | 21,6 | 138 ±3 | <0,01 | 0,39 ±0,03 | 0,86 ±0,06 | 20 ±4 | 2,78 ±0,2 | 2,45 ±0,1 | 95 ±10 | 2,90 | 2,10 | 1,9 |
| 444 | 20,9 | 142 ±3 | <0,01 | 0,37 ±0,03 | 0,76 ±0,06 | 19 ±4 | 2,80 ±0,2 | 2,46 ±0,1 | 100 ±10 | 2,80 | 2,20 | 1,8 |
| 505 | 20,3 | 136 ±3 | <0,01 | 0,38 ±0,03 | 0,84 ±0,06 | 19 ±4 | 2,86 ±0,2 | 2,54 ±0,2 | 100 ±10 | 2,81 | 2,45 | 1,9 |
| 486 | 21,1 | 138 ±3 | <0,01 | 0,36 ±0,02 | 0,76 ±0,06 | 18 ±4 | 2,82 ±0,2 | 2,50 ±0,2 | 100 ±10 | 2,79 | 2,40 | 1,9 |

**Tabelle 8.1**

| Beispiel Nr. | Partikelgrößenverteilung mittels Coulter LS 100 mit Mikrovolumenmodul nach Ultraschallbehandlung (X % der Partikel sind größer als Y µm) | | | | | | | | Perlgrößenverteilung durch Siebanalyse | | | |
|---|---|---|---|---|---|---|---|---|---|---|---|---|
| | Mittel-wert | Median-wert | Maximum | X = 5 % | X = 10 % | X = 50 % | X = 90 % | X = 95 % | | | | |
| | µm | µm | µm | Y µm | Y µm | Y µm | Y µm | Y µm | >300 µm | 150 - 300 µm | 75-150 µm | <75 µm |
| Ultrasil 7005 | 347,7 | 338,8 | 361,8 | 624,8 | 551,8 | 338,8 | 169,3 | 81,80 | 73,5 | 23,7 | 2,6 | 0,1 |
| 11 | 284,3 | 289,1 | 361,8 | 518,0 | 477,9 | 289,1 | 83,1 | 26,2 | 64,8 | 34,8 | 0,4 | 0 |
| 12 | 145,5 | 116,3 | 153,4 | 403,5 | 324,4 | 116,3 | 15,96 | 10,04 | 55,4 | 44 | 0,5 | 0,2 |
| 13 | 284,9 | 287,3 | 291,9 | 485,7 | 434,9 | 287,3 | 130,2 | 54,1 | 61,6 | 37,9 | 0,4 | 0,1 |
| 15 | 251,4 | 240,4 | 291,9 | 515,9 | 444,6 | 240,4 | 73,45 | 20,64 | 61,4 | 37,9 | 0,4 | 0,1 |

**Tabelle 9:**

| Beispiele Nr. | Durch-schnittlicher Ethanol-gehalt | Searszahlen | BET-Oberfläche | Mikro-poren, (d<2 nm) | Mesoporen Volumen, (d = 2-30 nm) | Mesoporen Volumen, (d = 2-50 nm) | Poren-maximum, Mesoporen | Makroporen, Volumen, (d > 30 nm) | Makroporen, Volumen, (d > 50 nm) | Poren-maximum Makroporen | Wasser-gehalt | Kohlenstoff-gehalt | Schwefelgehalt (Durchschnitt |
|---|---|---|---|---|---|---|---|---|---|---|---|---|---|
| | $\mu$mol/g | | m$^2$/g | ml/g | ml/g | ml/g | nm | ml/g | ml/g | $\mu$m | in Gew.% | in Gew.% | in Gew.% |
| Ultrasil | | | | | | | | | | | | | |
| 7005 | 0 | 23,9 | 185 +/-3 | 0,02+/-0,01 | 0,41+/-0,03 | 0,87 +/-0,06 | 19 ±4 | 3,26 ±0,2 | 2,91 ±0,2 | 130 ±10 | 6,7 | 0,06 | 0,2 |
| | | | | | | | | | | | | | |
| | | | | | | | | | | | | | |
| 16 | 609 | 21,6 | 142 ±3 | 0,01+/-0,01 | 0,33±0,02 | 0,64±0,03 | 22 ±4 | 3,25 ±0,2 | 2,80 ±0,2 | 95 ±10 | 3,79 | 2,72 | 1,9 |
| | | | | | | | | | | | | | |
| | | | | | | | | | | | | | |
| 17 | 540 | 21,5 | 145 ±3 | 0,01+/-0,01 | 0,35 ±0,02 | 0,70 ±0,06 | 24 ±4 | 3,34 ±0,2 | 2,88 ±0,2 | 100 ±10 | 4,15 | 2,64 | 1,85 |
| | | | | | | | | | | | | | |
| | | | | | | | | | | | | | |
| 18 | 444 | 21,9 | 150 ±3 | 0,01+/-0,01 | 0,37+/-0,02 | 0,70+/-0,06 | 25 ±4 | 3,49 ±0,2 | 3,02 ±0,2 | 140 ±10 | 5,07 | 2,17 | 1,8 |
| | | | | | | | | | | | | | |

**Tabelle 10:**

| Beispiel Nr. | Durchschnittlicher Ethanolgehalt | Searszahlen | BET-Oberfläche | Mikroporen, (d<2 nm) | Mesoporen Volumen (d = 2-30 nm) | Mesoporen Volumen (d = 2-50 nm) | Poren-maximum, Mesoporen | Makroporen, Volumen, (>30 nm) | Makroporen, Volumen, (>50 nm) | Poren-maximum, Makroporen | Wasser gehalt | Kohlenstoff gehalt | Schwefelgehalt (Durchschnitt) |
|---|---|---|---|---|---|---|---|---|---|---|---|---|---|
| | $\mu$mol/g | | m$^2$/g | ml/g | ml/g | ml/g | nm | ml/g | ml/g | $\mu$m | in Gew.% | in Gew.% | in Gew.% |
| | | | | | | | | | | | | | |
| Ultrasil 7005 | 0 | 23,9 | 185+/-3 | 0,02+/-0,01 | 0,41+/-0,03 | 0,87+/-0,06 | 19$\pm$4 | 3,26$\pm$0,2 | 2,91$\pm$0,2 | 130$\pm$10 | 6,7 | 0,06 | 0,2 |
| | | | | | | | | | | | | | |
| 19 | 502 | 21,8 | 144+/-3 | 0,01+/-0,01- | 0,35+/-0,02 | 0,72+/-0,06 | 23$\pm$4 | 3,36+/-0,2 | 2,90+/-0,2 | 100$\pm$10 | 4,09 | 2,45 | 1,85 |
| | | | | | | | | | | | | | |
| 20 | 472 | 20,6 | 147+/-3 | 0,01+/-0,01- | 0,35+/-0,02 | 0,69+/-0,06 | 25$\pm$4 | 3,29+/-0,2 | 2,84+/-0,2 | 100$\pm$10 | 4,28 | 2,40 | 1,9 |
| | | | | | | | | | | | | | |
| 21 | 499 | 20,5 | 148+/-3 | 0,01+/-0,01- | 0,36+/-0,02 | 0,68+/-0,06 | 28$\pm$4 | 3,28+/-0,2 | 2,80+/-0,2 | 105$\pm$10 | 4,95 | 2,30 | 1,9 |
| | | | | | | | | | | | | | |

**Tabelle 10.1**

| Beispiel Nr. | Partikelgrößenverteilung mittels Coulter LS 100 mit Mikrovolumenmodul nach Ultraschallbehandlung (X % der Partikel sind größer als Y μm) | | | | | | | | Perlgrößenverteilung durch Siebanalyse | | | |
|---|---|---|---|---|---|---|---|---|---|---|---|---|
| | Mittel-wert | Median-wert | Maximum | X = 5 % | X = 10 % | X = 50 % | X = 90 % | X = 95 % | | | | |
| | μm | μm | μm | Y μm | Y μm | Y μm | Y μm | Y μm | >300 μm | 150 - 300 μm | 75-150 μm | <75 μm |
| Ultrasil 7005 | 347,7 | 338,8 | 361,8 | 624,8 | 551,8 | 338,8 | 169,3 | 81,80 | 73,5 | 23,7 | 2,6 | 0,1 |
| 16 | 29,24 | 28,53 | 38,08 | 55,84 | 50,53 | 28,53 | 9,58 | 7,07 | 38,6 | 55,8 | 4,2 | 1,6 |
| 17 | 32,44 | 31,80 | 42,39 | 62,27 | 56,09 | 31,80 | 10,08 | 7,39 | 39 | 55,1 | 5 | 0,9 |
| 18 | 28,46 | 27,86 | 38,08 | 53,71 | 48,32 | 27,86 | 9,81 | 7,28 | 38,2 | 54,2 | 4,1 | 3,5 |
| 19 | 31,64 | 31,07 | 42,39 | 60,37 | 54,26 | 31,07 | 10,18 | 7,49 | 46,7 | 48,7 | 3,8 | 0,8 |
| 20 | 28,58 | 28,04 | 38,08 | 53,84 | 48,49 | 28,04 | 9,81 | 7,28 | 45 | 50,2 | 2,8 | 2 |
| 21 | 28,49 | 27,85 | 38,08 | 54,01 | 48,58 | 27,85 | 9,73 | 7,23 | 40,3 | 52,9 | 4,3 | 2,5 |

**Tabelle 11**

| | Anzahl be-trachtete Partikel | Statistische Funktion | Formfaktor | | | | Kreisformfaktor | | | |
|---|---|---|---|---|---|---|---|---|---|---|
| | | | Minimum | Maximum | Mittel- wert | Standard-abweichung | Minimum | Maximum | Mittel- wert | Standard-abweichung |
| Ultrasil | 116 | | 0,508 | 0,996 | 0,899 7005 | 0,0974 | 0,147 | 0,982 | 0,653 | 0,192 |
| Beispiel Nr. 2 | 233 | | 0,625 | 0,991 | 0,900 | 0,0807 | 0,144 | 0,985 | 0,644 | 0,203 |
| Coupsil 8108 Pulver | 822 | | 0,302 | 1,000 | 0,801 | 0,1470 | 0,121 | 0,999 | 0,534 | 0,195 |

**Tabelle 12**

| ID - Klasse | von | bis | ID- Klasse | von | bis |
|---|---|---|---|---|---|
| 1 | 0,00 | 0,05 | 11 | 0,50 | 0,55 |
| 2 | 0,05 | 0,1 | 12 | 0,55 | 0,6 |
| 3 | 0,10 | 0,15 | 13 | 0,60 | 0,65 |
| 4 | 0,15 | 0,2 | 14 | 0,65 | 0,7 |
| 5 | 0,20 | 0,25 | 15 | 0,70 | 0,75 |
| 6 | 0,25 | 0,3 | 16 | 0,75 | 0,8 |
| 7 | 0,30 | 0,35 | 17 | 0,80 | 0,85 |
| 8 | 0,35 | 0,4 | 18 | 0,85 | 0,9 |
| 9 | 0,40 | 0,45 | 19 | 0,90 | 0,95 |
| 10 | 0,45 | 0,5 | 20 | 0,95 | 1 |

**Tabelle 13**

| Beispiel Nr. | Partikelgrößenbestimmung mittels Coulter LS 100 und Dry Powder Modul (X % der Partikel sind größer als Y μm) | | | | | | | | Perlgrößenverteilung durch Siebanalyse | | | |
| --- | --- | --- | --- | --- | --- | --- | --- | --- | --- | --- | --- | --- |
| | Mittel-wert | Median-wert | Maximum | X = 5 % | X = 10 % | X = 50 % | X = 90 % | X = 95 % | | | | |
| | μm | μm | μm | Y μm | Y μm | Y μm | Y μm | Y μm | > 300 μm | >150 μm, <300 μm | >75 μm; <150 μm | <75 μm |
| Ultrasil 7005 | 347,2 | 332,0 | 325,0 | 546,9 | 493,7 | 332,0 | 226,1 | 205,0 | | | | |
| 2 | 330,2 | 321,0 | 325,0 | 528,5 | 477,5 | 321,0 | 207,2 | 174,2 | 75,1 | 20,7 | 4 | 0,2 |
| 4 | 352,7 | 337,9 | 325,0 | 557,8 | 504,3 | 337,9 | 225,5 | 203,4 | 71,5 | 25,4 | 3 | 0,2 |
| 5 | 360,3 | 346,2 | 361,8 | 565,6 | 512,1 | 346,2 | 231,2 | 207,7 | 51,3 | 46,7 | 1,5 | 0,5 |
| 11 | 356,6 | 341,1 | 325,0 | 563,7 | 509,4 | 341,1 | 229,3 | 207,2 | 64,8 | 34,8 | 0,4 | 0 |
| 17 | 302,6 | 293,7 | 291,9 | 504,9 | 452,3 | 293,7 | 180,3 | 136,6 | 39 | 55,1 | 5 | 0,9 |
| 20 | 342,3 | 333,0 | 325,0 | 560,5 | 506,0 | 333,0 | 208,0 | 172,4 | 45 | 52,9 | 4,3 | 2,5 |
| Beispiel gemäß DE10122269.6 | 25,5 | 17,7 | 30,7 | 74,3 | 57,2 | 17,7 | 2,5 | 1,7 | 5,2 | 8,6 | 57,3 | 28,9 |
| Coupsil 8010 Pulver | 25,9 | 18,5 | 34,2 | 76,3 | 60,8 | 18,5 | 2,4 | 1,6 | 3,9 | 7,6 | 52,3 | 36,2 |
| Coupsil 8108 | 483,9 | 504,9 | 853,0 | 865,7 | 831,5 | 504,9 | 45,8 | 10,8 | 89,3 | 3,2 | 4,3 | 3,1 |

(fortgesetzt)

| | µm | µm | µm | Y µm | Y µm | Y µm | Y µm | Y µm | > 300 µm | >150 µm, <300 µm | >75 µm; <150 µm | <75 µm |
|---|---|---|---|---|---|---|---|---|---|---|---|---|
| Granulat | | | | | | | | | | | | |

Die Tabellen 6-10 zeigen die analytischen Ergebnisse.

Von den Proben Ultrasil 7005 und Beispiel Nr. 2 (Tabelle 11) werden bei einer Vergrößerung von 20:1 Aufnahmen im Format 5,65 x 4 mm am Rasterelektronenmikroskop aufgenommen (Ultrasil 7005: 2 Aufnahmen; Probe NR. 2: 4 Aufnahmen) und die erhaltenen Bilder statistisch anhand der in Tabelle 12 aufgeführten Kriterien selektiert und statistisch hinsichtlich des Formfaktors und des Kreisformfaktors analysiert.

Von der Probe Coupsil 8108 (Pulver) (Tabelle 11) wird bei einer Vergrößerung von 500:1 eine Aufnahme im Format 178 $\mu$m x 126 $\mu$m am Rasterelektronenmikroskop aufgenommen und das erhaltene Bild statistisch anhand der in Tabelle 12 aufgeführten Kriterien selektiert und statistisch hinsichtlich des Formfaktors und des Kreisformfaktors analysiert.

Die ID - Klassen (Tabelle 12) selektieren und definieren die diskreten Kriterien für Formfaktor und Kreisformfaktor aus den statistischen Analysen.

In der Tabelle 13 sind die Partikelgrößenverteilungen durch Laserbeugung ohne Ultraschallbehandlung aufgeführt.

Beispiele für die Verwendung von Füllstoffen in Kautschukmischungen

Herstellung von Kautschukmischungen

Die für die Kautschukmischungen verwendete Rezeptur ist in der folgenden Tabelle 14 angegeben. Dabei bedeutet die Einheit phr Gewichtsanteile, bezogen auf 100 Teile des eingesetzten Rohkautschuks. Das allgemeine Verfahren zur Herstellung von Kautschukmischungen und deren Vulkanisaten ist in dem folgenden Buch beschrieben: "Rubber Technology Handbook", W. Hofmann, Hanser Verlag 1994.

**Tabelle 14**

| Substanz | Rezeptur A In-situ Referenz [phr] | Rezeptur B [phr] | Rezeptur C In-situ Referenz [phr] | Rezeptur D Referenz [phr] |
|---|---|---|---|---|
| **1. Stufe** | | | | |
| Buna VSL 5025-1 | 96 | 96 | 96 | 96 |
| Buna CB 24 | 30 | 30 | 30 | 30 |
| Ultrasil 7005 | 80 | -- | -- | -- |
| Ultrasil VN 3 GR | | | 80 | |
| silanmodifizierte Kieselsäure hergestellt mit dem erf. Verfahren | -- | 83 | -- | -- |
| VP Coupsil 8108 | -- | -- | -- | 83 |
| ZnO | 3 | 3 | 3 | 3 |
| Stearinsäure | 2 | 2 | 2 | 2 |
| Naftolen ZD | 10 | 10 | 10 | 10 |
| Vulkanox 4020 | 1,5 | 1,5 | 1,5 | 1,5 |
| Protector G35P | 1 | 1 | 1 | 1 |
| Si 69 | 6,4 | -- | 6,4 | -- |
| **2. Stufe** | | | | |
| Batch Stufe 1 | | | | |
| **3. Stufe** | | | | |
| Batch Stufe 2 | | | | |
| Vulkacit D | 2 | 2 | 2 | 2 |
| Vulkacit CZ | 1,5 | 1,5 | 1,5 | 1,5 |
| Schwefel | 1,5 | 1,5 | 1,5 | 1,5 |

Bei dem Polymer VSL 5025-1 handelt es sich um ein in Lösung polymerisiertes SBR-Copolymer der Bayer AG mit einem Styrolgehalt von 25 Gew.-% und einem Butadiengehalt von 75 Gew.-%. Von dem Butadien sind 73 % 1,2, 10 % cis 1,4 und 17 % trans 1,4 verknüpft. Das Copolymer enthält 37,5 phr Öl und weist eine Mooney-Viskosität (ML 1+4/100°C) von 50 □4 auf.

Bei dem Polymer Buna CB 24 handelt es sich um ein cis 1,4-Polybutadien (Neodymtyp) der Bayer AG mit einem cis 1,4-Gehalt von 97 %, einem trans 1,4-Gehalt von 2 %, einem 1,2-Gehalt von 1 % und einer Mooney-Viskosität von 44 □5.

Als aromatisches Öl wird Naftolen ZD der Chemetall verwendet. Bei Vulkanox 4020 handelt es sich um 6PPD der Bayer AG und Protektor G35P ist ein Ozonschutzwachs der HB-Fuller GmbH. Vulkacit D (DPG) und Vulkacit CZ (CBS) sind Handelsprodukte der Bayer AG.

Das Kopplungsreagenz Si 69 ist ein Bis-(triethoxysilylpropyl)tetrasulfan der Degussa AG. Ultrasil 7005 ist eine geperlte, leicht dispergierbare gefällte Kieselsäure der Degussa AG mit einer BET Oberfläche von 185 $m^2/g$. Ultrasil VN 3 ist ebenfalls eine gefällte Kieselsäure der Degussa AG mit einer BET Oberfläche von 175 $m^2/g$. VP Coupsil 8108 ist ein bekannter silanmodifizierter Füllstoff und ist von der Degussa AG als Versuchsprodukt erhältlich. Dabei handelt es sich um die Kieselsäure Ultrasil VN 3, die mit 8 Gewichtsteilen Si 69 pro 100 Gewichtsteilen Ultrasil VN 3 vorsilanisiert ist.

Die Kautschukmischungen werden in einem Innenmischer entsprechend der Mischvorschrift in Tabelle 15 hergestellt.

### Tabelle 15

| Stufe 1 | | |
|---|---|---|
| Einstellungen | | |
| Mischaggregat | | Werner & Pfleiderer E-Typ |
| Drehzahl | | 70 min$^{-1}$ |
| Stempeldruck | | 5,5 bar |
| Leervolumen | | 1,58 L |
| Füllgrad | | 0,56 |
| Durchflußtemp. | | 70 °C |
| Mischvorgang | | |
| 0 bis | 1 min | Buna VSL 5025-1 + Buna CB 24 |
| 1 bis | 3 min | 1/2 Kieselsäure bzw. vorsilanisierte Kieselsäure, ZnO, Stearinsäure, Naftolen ZD, ggf. Silan |
| 3 bis | 4 min | 1/2 Kieselsäure beispielsweise vorsilanisierte Kieselsäure, Alterungsschutz |
| | 4 min | Säubern |
| 4 bis | 5 min | Mischen |
| | 5 min | Säubern |
| 5 bis | 6 min | Mischen und ausfahren |
| Batch-Temp. | | 140-155°C |
| Lagerung | | 24 h bei Raumtemperatur |
| Stufe 2 | | |
| Einstellungen | | |
| Mischaggregat | | Wie in Stufe 1 bis auf: |
| Durchflußtemp. | | 80 °C |
| Füllgrad | | 0,53 |
| Mischvorgang | | |
| 0 bis | 2 min | Batch Stufe 1 aufbrechen |
| 2 bis | 5 min | Batchtemperatur 155°C durch Drehzahlvariation halten |
| | 5 min | Ausfahren |
| Batch-Temp. | | 155°C |
| Lagerung | | 4 h bei Raumtemperatur |

(fortgesetzt)

| Stufe 2 | |
|---|---|
| | |
| Stufe 3 | |
| Einstellungen | |
| Mischaggregat<br>Drehzahl<br>Füllgrad<br>Durchflußtemp. | wie in Stufe 1 bis auf<br>40 min$^{-1}$<br>0,50<br>50 °C |
| Mischvorgang | |
| 0 bis    2 min<br>         2 min | Batch Stufe 2, Beschleuniger,<br>ausfahren und auf Labormischwalzwerk Fell bilden<br>(Durchmesser 200 mm, Länge 450 mm, Durchflußtemperatur 50°C)<br><br>Homogenisieren:<br>3* links, 3* rechts einschneiden und<br>8* bei engem Walzenspalt (1 mm) und<br>3* bei weitem Walzenspalt (3,5 mm)<br>Fell ausziehen. |
| Batch-Temp. | 85-95°C |

In Tabelle 16 sind die Methoden für die Gummitestung zusammengestellt.

**Tabelle 16**

| Physikalische Testung | Norm/ Bedingungen |
|---|---|
| ML 1+4, 100°C, 3. Stufe | DIN 53523/3, ISO 667 |
| Vulkameterprüfung, 165°C<br>    Dmax - Dmin [dNm]<br>    t10% und t90% [min] | DIN 53529/3, ISO 6502 |
| Zugversuch am Ring, 23°C<br>    Zugfestigkeit [MPa]<br>    Spannungswerte [MPa]<br>    Bruchdehnung [%] | DIN 53504, ISO 37 |
| Shore-A-Härte, 23°C [SH] | DIN 53 505 |
| Viskoelastische Eigenschaften,<br>0 und 60°C, 16 Hz, 50 N Vorkraft und 25 N Amplitudenkraft<br>    Dynamischer Modul E* [MPa]<br>    Verlustfaktor tan | DIN 53 513, ISO 2856 |
| Ball Rebound, 23°C, 60°C [%] | ASTM D 5308 |
| DIN-Abrieb, 10 N Kraft [mm$^3$] | DIN 53 516 |

Mischungsbeispiele 1 bis 4

In den Mischungsbeispielen 1 bis 4 wird die in-situ gemischte Referenzmischung (Rezeptur A) mit 6,4 phr des Kopplungsreagenzes Si 69 gegen vier Mischungen (Rezeptur B) mit den in Tabelle 17 wiedergegebenen silanmodifizierten Kieselsäuren verglichen.

**Tabelle 17**

| Mischungsbeispiel: | - 1 - | - 2 - | - 3 - | - 4 - |
|---|---|---|---|---|
| Beispiel-Nr. gemäß den Tabellen 1, 2, 3, 4 und 5: | 2 | 11 | 17 | 20 |

Beispiele für die Herstellung der Füllstoffe sind in den Tabellen 1, 2, 3, 4 und 5 beschrieben worden. Die verwendeten Rezepturen (A) und (B) sind in der Tabelle 14 aufgeführt, und die verwendete Mischvorschrift ist in Tabelle 15 gezeigt.

Die Ergebnisse der gummitechnischen Prüfungen sind in den Tabellen 18 bis 21 zusammengestellt.

**Tabelle 18**

| Mischungsbeispiel | | in-situ Referenz | -1- |
|---|---|---|---|
| Rezeptur | | (A) | (B) |
| ML (1+4) | [ME] | 72 | 88 |
| Dmax-Dmin | [dNm] | 18,1 | 20,3 |
| t10% | [min] | 1, 2 0, 9 | |
| t90% | [min] | 23,4 | 19,0 |
| Shore-A-Härte | [SH] | 66 | 67 |
| Zugfestigkeit | [MPa] | 14,5 | 12,9 |
| Spannungswert 100% | [MPa] | 2,2 | 2,4 |
| Spannungswert 300% | [MPa] | 10,2 | 11,3 |
| RF 300%/100% | [ ] | 4,6 | 4,7 |
| Bruchdehnung | [%] | 380 | 330 |
| DIN-Abrieb | [mm$^3$] | 78 | 77 |
| Ball-Rebound, 60°C | [%] | 60 | 59 |
| E* (0°C) | [MPa] | 24,1 | 35,1 |
| tanδ (0°C) | [ ] | 0,459 | 0,420 |
| E* (60°C) | [MPa] | 8,0 | 11,0 |
| tanδ (60°C) | [ ] | 0,146 | 0,145 |

**Tabelle 19**

| Mischungsbeispiel | | in-situ Referenz | - 2- |
|---|---|---|---|
| Rezeptur | | (A) | (B) |
| ML (1+4) | [ME] | 69 | 85 |
| Dmax-Dmin | [dNm] | 17,3 | 11,0 |
| t10% | [min] | 1, 5 | 0,5 |
| t90% | [min] | 20,0 | 23,3 |
| Shore-A-Härte | [SH] | 62 | 64 |
| Zugfestigkeit | [MPa] | 12,8 | 14,9 |
| Spannungswert 100% | [MPa] | 1,8 | 2,0 |
| Spannungswert 300% | [MPa] | 8,6 | 10,0 |
| RF 300%/100% | [ ] | 4,8 | 5,0 |
| Bruchdehung | [%] | 390 | 390 |
| DIN-Abrieb | [mm$^3$] | 81 | 80 |
| Ball-Rebound, 60°C | [%] | 58 | 59 |
| E* (0°C) | [MPa] | 22,2 | 30,5 |
| tanδ (0°C) | [ ] | 0,448 | 0,445 |
| E* (60°C) | [MPa] | 8,1 | 9,8 |

(fortgesetzt)

| Mischungsbeispiel | | in-situ Referenz | - 2- |
|---|---|---|---|
| tanδ (60°C) | [ ] | 0,135 | 0,136 |

**Tabelle 20**

| Mischungsbeispiel | | in-situ Referenz | - 3- |
|---|---|---|---|
| Rezeptur | | (A) | (B) |
| ML (1+4) | [ME] | 76 | 81 |
| Dmax-Dmin | [dNm] | 15,8 | 16,5 |
| t10% | [min] | 1,6 | 1,2 |
| t90% | [min] | 15,2 | 16,1 |
| Shore-A-Härte | [SH] | 63 | 61 |
| Zugfestigkeit | [MPa] | 12,6 | 13,4 |
| Spannungswert 100% | [MPa] | 2,3 | 2,1 |
| Spannungswert 300% | [MPa] | 11,5 | 10,7 |
| RF 300%/100% | [ ] | 5,0 | 5,1 |
| Bruchdehnung | [%] | 320 | 350 |
| DIN-Abrieb | [mm$^3$] | 62 | 74 |
| Ball-Rebound, 60°C | [%] | 63 | 63 |
| E* (0°C) | [MPa] | 20,9 | 21,5 |
| tanδ (0°C) | [ ] | 0,454 | 0,452 |
| E* (60°C) | [MPa] | 8,3 | 8,2 |
| tanδ (60°C) | [ ] | 0,124 | 0,129 |

**Tabelle 21**

| Mischungsbeispiel | | in-situ Referenz | - 4 - |
|---|---|---|---|
| Rezeptur | | (A) | (B) |
| ML (1+4) | [ME] | 76 | 83 |
| Dmax-Dmin | [dNm] | 15,8 | 17,6 |
| t10% | [min] | 1,6 | 1,0 |
| t90% | [min] | 15,2 | 15,5 |
| Shore-A-Härte | [SH] | 63 | 62 |
| Zugfestigkeit | [MPa] | 12,6 | 11,5 |
| Spannungswert 100% | [MPa] | 2,3 | 2,2 |
| Spannungswert 300% | [MPa] | 11,5 | 11,3 |
| RF 300%/100% | [ ] | 5,0 | 5,1 |
| Bruchdehnung | [%] | 320 | 300 |
| DIN-Abrieb | [mm$^3$] | 62 | 66 |
| Ball-Rebound, 60°C | [%] | 63 | 62 |
| E* (0°C) | [MPa] | 20,9 | 26,3 |
| tan□ (0°C) | [ ] | 0,454 | 0,460 |
| E* (60°C) | [MPa] | 8,3 | 9,8 |
| tan□ (60°C) | [ ] | 0,124 | 0,129 |

Wie man anhand der Daten in den Tabellen 18 bis 21 sieht, liegen die Viskositäten ML (1+4) und die Vulkanisationscharakteristika der Beispielmischungen auf ähnlichem Niveau wie die der in-situ Referenzmischungen. Die statischen und dynamischen Gummidaten sind im Rahmen der üblichen Schwankungen von Gummitestungen vergleichbar. Der für die Beispielmischungen im Vergleich zu den in-situ Refenzmischungen durchweg höhere Verstärkungsfaktor RF 300%/100% zeigt eine höhere Kieselsäure-Silan-Anbindung. Dies alles zeigt eindeutig, daß die Verwendung der Kieselsäure zu einem Gummiwertebild führt, das mit dem der in-situ Referenz vergleichbar beziehungsweise tendenziell besser ist. Dies ist mit silanisierten Füllstoffen die dem Stand der Technik entsprechen nicht erreichbar.

Mischungsbeispiel: Stand der Technik

Das Mischungsbeispiel für den Stand der Technik zeigt, daß gegenüber der in-situ Referenzmischung (Rezeptur C) das gummitechnische Eigenschaftsbild bei der Verwendung der handelsüblichen vorsilanisierten Kieselsäure VP Coupsil 8108 (Rezeptur D) abfällt. Die Rezepturen (C) und (D) basieren auf den in Tabelle 14 gezeigten Rezepturen. In Abänderung zu der in den Rezepturen (A) und (B) verwendeten und in Tabelle 14 angegebenen Mischvorschrift wird in diesem Beispiel die 2. Mischstufe mit einer Anfangsdrehzahl von 80 min$^{-1}$ bei einer Durchflußtemperatur von 80°C gemischt. Dies stellt jedoch keine gravierende Abweichung dar. Die Ergebnisse sind in Tabelle 22 zusammengestellt.

**Tabelle 22**

| Mischungsbeispiel | | in-situ Referenz | - 5 - |
|---|---|---|---|
| Rezeptur | | (C) | (D) |
| ML (1+4) | [ME] | 60 | 82 |
| Dmax-Dmin | [dNm] | 18,9 | 22,1 |
| t10% | [min] | 1,6 | 1,1 |
| t90% | [min] | 23,2 | 36,0 |
| Shore-A-Härte | [SH] | 62 | 69 |
| Zugfestigkeit | [MPa] | 13,0 | 13,0 |
| Spannungswert 100% | [MPa] | 1,9 | 2,3 |
| Spannungswert 300% | [MPa] | 8,9 | 9,1 |
| RF 300%/100% | [ ] | 4,7 | 4,0 |
| Bruchdehnung | [%] | 380 | 380 |
| DIN-Abrieb | [mm$^3$] | 91 | 88 |
| Ball-Rebound, 23°C | [%] | 32 | 33 |
| E* (0°C) | [MPa] | 15,4 | 20,5 |
| tanδ (0°C) | [ ] | 0,486 | 0,502 |
| E* (60°C) | [MPa] | 6,5 | 7,7 |
| tanδ (60°C) | [ ] | 0,138 | 0,144 |

Die Werte aus Tabelle 22 zeigen, daß mit der Verwendung der bekannten, vorsilanisierten Kieselsäure VP Coupsil 8108 nicht das hohe Niveau der in-situ Referenzmischung erreicht wird.
Sowohl die höhere Mooney-Viskosität als auch die höhere Shore-A-Härte zeigen eine ungenügend homogene Silanisierung an, die zu einem höheren Füllstoffnetzwerk von Mischung (D) führt. Zusätzlich fällt der Verstärkungsfaktor RF 300%/100% der Mischung (D) gegenüber der Referenz (C) deutlich ab.
Der Vorteil der Verwendung der Kieselsäuren liegt darin, daß entgegen der bekannterweise verwendeten in-situ Silanisierung nach dem Stand der Technik mit flüssigen Silanen, wie zum Beispiel Si 69, während des Mischprozesses keine chemische Reaktion bei der eine optimale Prozesskontrolle erforderlich ist durchgeführt werden muss. Zudem werden bei der bekannten in-situ Silanisierung nachteiligerweise erhebliche Mengen Alkohol freigesetzt, die aus der Mischung entweichen und so zu Problemen in der Abluft führen.
Die Mischungsbeispiele zeigen eindeutig, daß mit der Verwendung der vorsilanisierten Kieselsäuren im Gummi ein der in-situ Silanisierung nach dem Stand der Technik vergleichbares oder besseres Eigenschaftsbild erreicht wird, ohne die oben genannten Nachteile, wie sie bei der bekannten in-situ Silanisierung auftreten, zu bewirken. Im Gegensatz dazu wird mit der Verwendung von handelsüblichen vorsilanisierten Kieselsäuren, wie zum Beispiel VP Coupsil 8108 zwar das erwähnte Problem der Ethanolentwicklung beim Mischen vermieden, das hohe gummitechnische Niveau der in-situ Referenz jedoch nicht erreicht.

**Patentansprüche**

1. Verfahren zur Herstellung von silanmodifiziertem oxidischem oder silikatischem Füllstoff mit einer Perlfraktion Kleiner als 75 μm von weniger als 15 Gew-%, bestimmt durch Siebanalyse, und einem Madianwert der Partikelgröße zwischen 130 und 500 μm, bestimmt durch Laserbeugung ohne Ultraschallbehandlung, **dadurch gekennzeichnet, daß** man mindestens einen mikrogeperlten oder mikrogranulierten, oxidischen oder silikatischen Füllstoff in einem mittels Druck und / oder Temperatur verdichteten Gas mit mindestens einem Silan umsetzt.

2. Verfahren zur Herstellung von silanmodifiziertem oxidischem oder silikatischem Füllstoff nach Anspruch 1, **dadurch gekennzeichnet, daß** man als Silan eine Organosiliziumverbindung oder ein Gemisch aus Organosiliziumverbindungen der allgemeinen Formel (I)

$$Z\text{-}A\text{-}S_x\text{-}A\text{-}Z \qquad (I)$$

verwendet, in der
$x$ eine Zahl von 1 bis 14 ist,
$Z$ gleich $SiX^1X^2X^3$ ist und
$X^1$, $X^2$, $X^3$ jeweils unabhängig voneinander bedeuten können Wasserstoff (-H),
Halogen oder Hydroxy (-OH),
ein Alkyl-,
ein Alkylsäure- $(C_xH_{2x+1})$ -C (=O) O-,
ein Alkenylsäuresubstituent oder
ein substituierter Alkyl-, beziehungsweise Alkenylsäuresubstituent,
eine lineare oder verzweigte, ringförmige Kohlenwasserstoffkette mit 1-8 Kohlenstoffatomen,
ein Cycloalkanrest mit 5-12 Kohlenstoffatomen,
ein Benzylrest oder
ein halogen- oder alkylsubstituierter Phenylrest, Alkoxygruppen mit linearen oder verzweigten Kohlenstoffwasserstoffketten mit $(C_{1-24})$ Atomen, Alkoxygruppen mit linearen oder verzweigten Polyetherketten mit $(C_{1-24})$ Atomen, eine Cycloalkoxygruppe mit $(C_{5-12})$-Atomen,
eine halogen- oder alkylsubstituierte Phenoxygruppe oder eine Benzyloxygruppe,
$A$ eine verzweigte oder unverzweigte, gesättigte oder ungesättigte alliphatische, aromatische oder gemischt alliphatische/aromatische zweibindige $C_1$-$C_{30}$ umfassende Kohlenwasserstoffkette ist.

3. Verfahren zur Herstellung von silanmodifiziertem oxidischem oder silikatischem Füllstoff nach Anspruch 2, **dadurch gekennzeichnet, daß** man als Silan der Formel (I) $[(EtO)_3Si(CH_2)_3]_2S$, $[(EtO)_3Si(CH_2)_3]_2S_2$, $[(EtO)_3Si(CH_2)_3]_2S_3$, $[(EtO)_3Si(CH_2)_3]_2S_4$, $[(EtO)_3Si(CH_2)_3]_2S_5$, $[(EtO)_3Si(CH_2)_3]_2S_6$, $[(EtO)_3Si(CH_2)_3]_2S_7$, $[(EtO)_3Si(CH_2)_3]_2S_8$, $[(EtO)_3Si(CH_2)_3]_2S_9$, $[(EtO)_3Si(CH_2)3]_2S_{10}$, $[(EtO)_3Si(CH_2)_3]_2S_{11}$ $[(EtO)_3Si(CH_2)_3]_2S_{12}$, $[(EtO)_3Si(CH_2)_3]S_{13}$, $[(EtO)_3Si(CH_2)_3]_2S_{14}$, $[(C_yH_{yx+1}O)(R)_2Si(CH_2)_3]S_x[(CH_2)_3Si(R)_3]$, $[(C_yH_{2y+1}O)_2(R)Si(CH_2)_3]S_x[(CH_2)_3Si(R)_3]$, $[(C_yH_{2y+1}O)_3$-$Si(CH_2)_3]S_x[(CH_2)_3Si(R)_3]$, $[(C_yH_{2y+1}O)(R)_2Si(CH_2)_3]S_x[(CH_2)_3Si(C_yH_{2y+1}O)(R)_2]$, $[(C_yH_{2y+1}O)_2(R)Si(CH_2)_3]S_x[(C$-$H_2)_3Si(C_yH_{2y+1}O)(R)_2]$, $[(C_yH_{2y+1}O)_3Si(CH_2)_3]S_x[(CH_2)_3Si(C_yH_{2y+1}O)(R)_2]$, $[(C_yH_{2y+1}O)(R)_2Si(CH_2)_3]S_x[(CH_2)_3Si(C_yH_{2y+1}O)_2(R)]$, $[(C_yH_{2y+1}O)_2(R)Si(CH_2)_3]S_x[(CH_2)_3Si(C_yH_{2y+1}O)_2(R)]$, $[(C_yH_{2y+1}O)_3Si(CH_2)_3]S_x[(CH_2)_3Si(C_yH_{2y+1})_2(R)]$, $[(C_yH_{2y+1}O)(R)_2Si(CH_2)_3]S_x[(CH_2)_3Si(C_yH_{2y+10}O)_3]$, $[(C_yH_{2y+1}O)_2(R)Si(CH_2)_3]S_x[(CH_2)_3Si(C_yH_{2y+1}O)_3]$, $[(C_yH_{2y+1}O)_3Si(CH_2)_3]S_x[(CH_2)_3Si(C_yH_{2y+1}O)_3]$,
mit $x = 1$-$14$, $y = 10$-$24$ und $R = (MeO)$ oder/und $(EtO)$, oder Mischungen der einzelnen Silane verwendet.

4. Verfahren zur Herstellung von silanmodifiziertem oxidischem oder silikatischem Füllstoff nach Anspruch 1, **dadurch gekennzeichnet, daß** man als Silan eine Organosiliziumverbindung oder Mischungen von Organosiliziumverbindungen der allgemeinen Formel (II)

$$X^1X^2X^3Si\text{-}A\text{-}S\text{-}SiR^1R^2R^3 \qquad (II)$$

verwendet, in der
$X^1$, $X^2$, $X^3$ und $A$ unabhängig voneinander dieselbe Bedeutung wie in Formel (I) haben,
$R^1$, $R^2$, $R^3$ jeweils unabhängig voneinander sind und,
$(C_1$-$C_{16})$ Alkyl, $(C_1$-$C_{16})$ Alkoxy, $(C_1$-$C_{16})$ Haloalkyl, Aryl, $(C_7$-$C_{16})$ Aralkyl, -H, Halogen oder $X^1X^2X^3Si\text{-}A\text{-}S$- bedeuten.

5. Verfahren zur Herstellung von silanmodifiziertem oxidischem oder silikatischem Füllstoff nach Anspruch 1, **dadurch gekennzeichnet, daß** man als Silan eine Organosiliziumverbindung oder eine Mischung von Organosiliziumverbindungen der allgemeinen Formel (III)

$$X^1X^2X^3Si\text{-Alk} \qquad (III)$$

verwendet, in der

$X^1$, $X^2$, $X^3$ jeweils unabhängig voneinander dieselbe Bedeutung wie in Formel (I) haben und

Alk ein geradkettiger, verzweigter oder zyklischer $(C_1\text{-}C_{24})$Alkyl-, $(C_1\text{-}C_{24})$ Alkoxy-, Halogen-, Hydroxy-, Nitril-, Thiol-, $(C_1\text{-}C_4)$ Haloalkyl-, $-NO_2$, $(C_1\text{-}C_8)$ Thioalkyl-, $-NH_2$, $-NHR^1$, $-NR^1R^2$, Alkenyl, Allyl-, Vinyl-, Aryl- oder ein $(C_7\text{-}C_{16})$ Aralkyl Substituent ist.

6. Verfahren zur Herstellung von silanmodifiziertem oxidischem oder silikatischem Füllstoff nach Anspruch 5, **dadurch gekennzeichnet, daß** man als Silan der Formel (III) $(MeO)_3\text{-}Si\text{-}(CH_2)_3\text{-}H$, $(EtO)_3\text{-}Si\text{-}(CH_2)_3\text{-}H$, $(MeO)_3\text{-}Si\text{-}C(CH_3)_3$, $(EtO)_3\text{-}Si\text{-}C(CH_3)_3$, $(MeO)_3\text{-}Si\text{-}(CH_2)_8\text{-}H$, $(EtO)_3\text{-}Si\text{-}(CH_2)_8\text{-}H$, $(MeO)_3\text{-}Si\text{-}(CH_2)_{16}\text{-}H$, $(EtO)_3\text{-}Si\text{-}(CH_2)_{16}\text{-}H$, $Me_3Si\text{-}OMe$, $Me_3Si\text{-}OEt$, $Me_3Si\text{-}Cl$, $Et_3Si\text{-}Cl$, $(MeO)_3Si\text{-}CH=CH_2$, $(EtO)_3Si\text{-}CH=CH_2$, $(Me_3Si)_2N\text{-}C(O)\text{-}H$, $(Me_3Si)_2N\text{-}H$ oder Mischungen der Silane verwendet.

7. Verfahren zur Herstellung von silanmodifiziertem oxidischem oder silikatischem Füllstoff nach Anspruch 1, **dadurch gekennzeichnet, daß** man als Silan eine Organosiliziumverbindung oder eine Mischung von Organosiliziumverbindungen der allgemeinen Formel (IV) oder (V)

$$[[(ROC\,(=O))_P\text{-}(G)_j]_k\text{-}Y\text{-}S]_r\text{-}G\text{-}(SiX^1X^2X^3)_s \qquad (IV)$$

$$[(X^1X^2X^3Si)_q\text{-}G]_a\text{-}[Y\text{-}[S\text{-}G\text{-}SiX^1X^2X^3]_b]_c \qquad (V)$$

verwendet,

in der Y eine polyvalente Spezies $(Q)_zD(=E)$ darstellt, wobei folgendes gilt:

p ist 0 bis 5, r ist 1 bis 3, z ist 0 bis 2; q ist 0 bis 6,

a ist 0 bis 7, b ist 1 bis 3, j ist 0 bis 1, aber es kann, wenn p = 1 auch häufig 0 sein, c ist 1 bis 6, t ist 0 bis 5, s ist 1 bis 3, k ist 1 bis 2, unter der Vorraussetzung, daß

    (1) falls (D) ein Kohlenstoff, Schwefel oder Sulfonyl ist, gilt, daß a + b = 2 und k = 1,
    (2) falls (D) ein Phosphoratom ist, gilt, daß a + b = 3 solange c ≥ 1 und b = 1, wobei a = c + 1,
    (3) falls (D) ein Phosphoratom ist, gilt, daß k = 2, ist,

Y eine polyvalente Spezies $(Q)_zD(=E)$ darstellt,

in jeder dieser Gruppen ist das Atom (D) doppelt mit dem Heteroatom (E) verbunden, das wiederum mit dem Schwefelatom (S) verbunden ist, welches mittels einer Gruppe (G) mit dem Siliziumatom (Si) verknüpft ist,

$R^1$ unabhängig voneinander H, eine gerade, zyklische oder verzweigte Alkylkette bedeuten, gegebenenfalls Alkylketten die ungesättigte Anteile wie Doppelbindungen (Alkene), Dreifachbindungen (Alkine) oder auch Alkylaromaten (Aralkyl) oder Aromaten beinhalten und die die gleichen Bedeutungen in Formel (II) aufweisen,

G unabhängig von den übrigen Substituenten, Wasserstoff, eine gerade, zyklische oder verzweigte Alkylkette mit $(C_1\text{-}C_{18})$ bedeuten, gegebenenfalls können die Alkylketten einen ungesättigten Anteil,

wenn p = 0 in Formel ist, ist G vorzugsweise Wasserstoff (H),

G entspricht nicht der Struktur eines α, β-ungesättigten Fragments, welches mit dem Y -Fragment in der Weise verbunden ist, daß ein α, β-ungesättigtes Thiocarbonylfragment entsteht,

$X^1$, $X^2$ und $X^3$ jeweils unabhängig voneinander die Bedeutung wie in Formel (I) besitzen.

8. Verfahren zur Herstellung von silanmodifiziertem oxidischem oder silikatischem Füllstoff nach Anspruch 1, **dadurch gekennzeichnet, daß** man als Silan eine Organosiliziumverbindung oder eine Mischung von Organosiliziumverbindungen der allgemeinen Formel (VI)

$$X^1X^2X^3Si\text{-}A\text{-}Sub \qquad (VI)$$

verwendet, wobei $X^1$, $X^2$, $X^3$ und A, jeweils unabhängig voneinander, die Bedeutung gemäß Formel (I) haben und Sub - $-NH_2$, $-SH$, $-NH(A\text{-}SiX^1X^2X^3)$, $-N(A\text{-}SiX^1X^2X^3)_2$, $O\text{-}C(O)\text{-}CMe=CH_2$, oder $-SCN$ ist.

9. Verfahren zur Herstellung von silanmodifiziertem oxidischem oder silikatischem Füllstoff nach Anspruch 8, **dadurch**

**gekennzeichnet, daß** man als Silan der Formel (VI) $[(MeO)_3Si-(CH_2)_3-]_2NH$, $[(EtO)_3Si-(CH_2)_3-]_2NH$, $[(C_3H_7O)_3Si-(CH_2)_3-]_2NH$, $(MeO)_3Si-(CH_2)_3-NH_2$, $(EtO)_3Si-(CH_2)_3-NH_2$, $(C_3H_7O)_3Si-(CH_2)_3-NH_2$, $(MeO)_3Si-(CH_2)_3-NH-(CH_2)_2-NH_2$, $(EtO)_3Si-(CH_2)_3-NH-(CH_2)_2-NH_2$, $(C_3H_7O)_3Si-(CH_2)_3-NH-(CH_2)_2-NH_2$, $(MeO)_3Si-(CH_2)_3-SH$, $(EtO)_3Si-(CH_2)_3-SH$, $(C_3H_7O)_3Si-(CH_2)_3-SH$, $(MeO)_3Si-(CH_2)_3-O-C(O)-CMe=CH_2$, $(EtO)_3Si-(CH_2)_3-O-C(O)-CMe=CH_2$, $(C_3H_7O)_3Si-(CH_2)_3-O-C(O)-CMe=CH_2$, SCN, $(EtO)_3Si-(CH_2)_3-SCN$, $(C_3H_7O)_3Si-(CH_2)_3-SCN$, $[(C_yH_{2y+1}O)(EtO)_2Si(CH_2)_3]-NH_2$, $[(C_yH_{2y+1}O)_2(EtO)Si(CH_2)_3]-NH_2$, $[(C_yH_{2y+1}O)(EtO)_2Si(CH_2)_3]-SH$, $[(C_yH_{2y+1}O)_2(EtO)Si(CH_2)_3]-SH$, $[(C_yH_{2y+1}O)(EtO)_2Si(CH_2)_3]-O-C(O)-CMe=CH_2$, $[(C_yH_{2y+1}O)_2(EtO)Si(CH_2)_3]-O-C(O)-CMe=CH_2$, mit y = 10-24,
oder Mischungen der genannten Silane verwendet.

10. Verfahren zur Herstellung von silanmodifiziertem oxidischem oder silikatischem Füllstoff nach Anspruch 1, **dadurch gekennzeichnet, daß** man als Silan Mischungen der Silane der Formeln I-VI verwendet.

11. Verfahren zur Herstellung von silanmodifiziertem oxidischem oder silikatischem Füllstoff nach Anspruch 1, **dadurch gekennzeichnet, daß** man als Silan oligomere oder cooligomere Silane der Formeln I-VI oder deren Mischungen oder Mischungen von Silanen der Formeln I-VI und deren Oligomeren oder Cooligomeren verwendet.

12. Verfahren zur Herstellung von silanmodifiziertem oxidischem oder silikatischem Füllstoff nach Anspruch 1, **dadurch gekennzeichnet, daß** man als mikrogeperlten oder mikrogranulierten, oxidischen oder silikatischen Füllstoff einen natürlichen und/oder synthetischen Füllstoff verwendet.

13. Verfahren zur Herstellung von silanmodifiziertem oxidischem oder silikatischem Füllstoff nach Anspruch 1, **dadurch gekennzeichnet, daß** man als mikrogeperlten oder mikrogranulierten, oxidischen oder silikatischen Füllstoff mikrogeperltes oder mikrogranuläres Kaolin, Kieselgur, Mica, Diatomeenerden, Clay, Talkum, Wollastonit, Silikate unter anderem in Form von Glaskugeln, gemahlenen Glassplittern (Glasmehl), Glasfasern oder Glasgeweben, Zeolithe, Aluminiumoxid, Aluminiumhydroxid oder -trihydrat, Aluminiumsilikate, Silikate, gefällte Kieselsäuren mit BET-Oberflächen (gemessen mit gasförmigem Stickstoff) von 1 bis 1000 $m^2$/g, Zinkoxid, Boroxid, Magnesiumoxid oder allgemein Übergangsmetalloxide verwendet.

14. Verfahren zur Herstellung von silanmodifiziertem oxidischem oder silikatischem Füllstoff nach Anspruch 1, **dadurch gekennzeichnet, daß** man 10-250 Gew.-Teile mikrogeperlten oder mikrogranulierten, oxidischen oder silikatischen Füllstoff mit 0,1-50 Gew.-Teilen Silan umsetzt.

15. Verfahren zur Herstellung von silanmodifiziertem oxidischem oder silikatischem Füllstoff nach Anspruch 1, **dadurch gekennzeichnet, daß** man als verdichtetes Gas Kohlendioxid, Helium, Stickstoff, Distickstoffmonoxid, Schwefelhexafluorid, gasförmige Alkane mit 1 bis 5 C-Atomen, gasförmige Alkene mit 2 bis 4 C-Atomen, gasförmige Alkine, gasförmige Diene, gasförmige Fluorkohlenwasserstoffe, Chlor und/oder Fluorchlorkohlenwasserstoffe oder deren Ersatzstoffe oder Ammoniak, sowie Gemische dieser Stoffe verwendet.

16. Verfahren zur Herstellung von silanmodifiziertem oxidischem oder silikatischem Füllstoff nach Anspruch 1, **dadurch gekennzeichnet, daß** die verwendeten Silane in dem verdichteten Gas ungelöst, teilweise oder vollständig gelöst sind.

17. Verfahren zur Herstellung von silanmodifiziertem oxidischem oder silikatischem Füllstoff nach Anspruch 1, **dadurch gekennzeichnet, daß** der Druck zwischen 1 und 500 bar beträgt.

18. Verfahren zur Herstellung von silanmodifiziertem oxidischem oder silikatischem Füllstoff nach Anspruch 1, **dadurch gekennzeichnet, daß** die Umsetzung bei einer Temperatur zwischen 0 und 300°C erfolgt.

19. Verfahren zur Herstellung von silanmodifiziertem oxidischem oder silikatischem Füllstoff nach Anspruch 1, **dadurch gekennzeichnet, daß** man den Druck während der Umsetzung auf unterschiedlichen Druckniveaus für Zeiträume von 5 - 720 min konstant hält und der Füllstoff während dieser Zeit in das verdichtete Gas eintaucht, von diesem durchflossen oder in diesem gerührt wird.

20. Verfahren zur Herstellung von silanmodifiziertem oxidischem oder silikatischem Füllstoff nach Anspruch 1, **dadurch gekennzeichnet, daß** man den mikrogeperlten oder mikrogranulierten, oxidischen oder silikatischen Füllstoff und das Silan zuerst durchmischt beziehungsweise in Kontakt bringt und dann mit dem im verdichteten Zustand vorliegenden Gas mischt beziehungsweise in Kontakt bringt.

**21.** Verfahren zur Herstellung von silanmodifiziertem oxidischem oder silikatischem Füllstoff nach Anspruch 1, **dadurch gekennzeichnet, daß** man den mikrogeperlten oder mikrogranulierten, oxidischen oder silikatischen Füllstoff erst mit dem im verdichteten Zustand vorliegenden Gas durchmischt beziehungsweise in Kontakt bringt und dann mit dem Silan mischt beziehungsweise in Kontakt bringt.

**22.** Verfahren zur Herstellung von silanmodifiziertem oxidischem oder silikatischem Füllstoff nach Anspruch 1, **dadurch gekennzeichnet, daß** man das Silan, erst mit dem im verdichteten Zustand vorliegenden Gas durchmischt beziehungsweise in Kontakt bringt und dann mit dem entsprechenden mikrogeperlte oder mikrogranulierte, oxidische oder silikatische Füllstoff mischt beziehungsweise in Kontakt bringt.

**23.** Verfahren zur Herstellung von silanmodifiziertem oxidischem oder silikatischem Füllstoff nach Anspruch 1, **dadurch gekennzeichnet, daß** man dem mikrogeperlten oder mikrogranulierten, oxidischen oder silikatischen Füllstoff und/ oder dem Silan vor der Umsetzung im verdichteten Gas oder Gasgemisch zusätzliche Additive zusetzt.

**24.** Verfahren zur Herstellung von silanmodifiziertem oxidischem oder silikatischem Füllstoff nach Anspruch 1, **dadurch gekennzeichnet, daß** man dem silanmodifizierten oxidischen oder silikatischen Füllstoff während der Umsetzung im verdichteten Gas mit zusätzlichen Additiven in Kontakt bringt.

**25.** Verfahren zur Herstellung von silanmodifiziertem oxidischem oder silikatischem Füllstoff nach Anspruch 1, **dadurch gekennzeichnet, daß** man während der Umsetzung des mikrogeperlten oder mikrogranulierten, oxidischen oder silikatischen Füllstoffs im verdichteten Gas, in den zugeführten oder abgeführten Strom an verdichteten Gas der den silanmodifizierten oxidischen oder silikatischen Füllstoff durchströmt, zusätzlich Additive einbringt.

**26.** Verfahren zur Herstellung von silanmodifiziertem oxidischem oder silikatischem Füllstoff nach einem der Ansprüche 23-25, **dadurch gekennzeichnet, daß** man als Additive Ammoniak, Schwefeldioxid, Wasser, kurzkettige oder langkettige Alkohole, kurzkettige oder langkettige Polyether bzw. kurzkettige oder langkettige Amine, Emulsionsbildner oder auch kurzkettige oder langkettige Silikonöle verwendet.

**Claims**

**1.** Process for producing silane-modified oxidic or silicatic filler with a bead fraction smaller than 75 $\mu$m of less than 15% by weight, determined by sieve analysis, and a median particle size between 130 and 500 $\mu$m, determined by laser diffraction without ultrasound treatment, **characterized in that** at least one microbeaded or microgranulated oxidic or silicatic filler is reacted with at least one silane in a gas compressed by means of pressure and/or temperature.

**2.** Process for producing silane-modified oxidic or silicatic filler according to Claim 1, **characterized in that** the silane used is an organosilicon compound or a mixture of organosilicon compounds of the general formula (I)

$$\textbf{Z-A-S}_\textbf{x}\textbf{-A-Z} \qquad \text{(I)}$$

in which
x is a number from 1 to 14,
Z is $SiX^1X^2X^3$ and
$X^1$, $X^2$, $X^3$ may each independently be hydrogen (-H),
halogen or hydroxyl (-OH),
an alkyl,
an alkyl acid $(C_xH_{2X+1})$-C(=O)O-,
an alkenyl acid substituent or
a substituted alkyl acid or
alkenyl acid substituent,
a linear or branched cyclic
hydrocarbyl chain having 1 to 8 carbon atoms,
a cycloalkane radical having 5-12 carbon atoms,
a benzyl radical or
a halogen- or alkyl-substituted phenyl radical, alkoxy groups with linear or branched hydrocarbyl chains with ($C_{1-24}$) atoms,
alkoxy groups with linear or branched polyether chains having ($C_{1-24}$) atoms,

a cycloalkoxy group having ($C_{5-12}$) atoms,

a halogen- or alkyl-substituted phenoxy group or a benzyloxy group,

A is a branched or unbranched, saturated or unsaturated aliphatic, aromatic or mixed aliphatic/aromatic divalent hydrocarbon chain comprising $C_1$-$C_{30}$.

**3.** Process for producing silane-modified oxidic or silicatic filler according to Claim 2, **characterized in that** the silane of the formula (I) used is

$[(EtO)_3Si(CH_2)_3]_2S$, $[(EtO)_3Si(CH_2)_3]_2S_2$, $[(EtO)_3Si(CH_2)_3]_2S_3$, $[(EtO)_3Si(CH_2)_3]_2S_4$, $[(EtO)_3Si(CH_2)_3]_2S_5$, $[(EtO)_3Si(CH_2)_3]_2S_6$, $[(EtO)_3Si(CH_2)_3]_2S_7$, $[(EtO)_3Si(CH_2)_3]_2Se$, $[(EtO)_3Si(CH_2)_3]_2S_9$, $[(EtO)_3Si(CH_2)_3]_2S_{10}$, $[(EtO)_3Si(CH_2)_3]_2S_{11}$, $[(EtO)_3Si(CH_2)_3]_2S_{12}$, $[(EtO)_3Si(CH_2)_3]_2S_{13}$, $[(EtO)_3Si(CH_2)_3]_2S_{14}$, $[(C_yH_{2y+1}O)(R)_2Si(CH_2)_3S_x[(CH_2)_3Si(R)_3]$ $[(C_yH_{2y+1}O)_2(R)Si(CH_2)_3]S_x[(CH_2)_3Si(R)_3]$, $[(C_yH_{2y+1}O)_3Si(CH_2)_3S_x[(CH_2)_3Si(R)_3]$, $[(C_yH_{2y+1}O)(R)_2Si(CH_2)_3]S_x[(CH_2)_3Si(C_yH_{2y+1}O)(R)_2]$, $[(C_yH_{2y+1}O)_2(R)Si(CH_2)_3]S_x[(CH_2)_3Si(C_yH_{2+1}O)(R)_2]$, $[(C_yH_{2y+1}O)_3Si(CH_2)_3]S_x[(CH_2)_3Si(C_yH_{2y+1}O)(R)_2]$, $[(C_yH_{2y+1}O)(R)_2Si(CH_2)_3]S_x[(CH_2)_3Si(C_yH_{2y+1}O)_2(R)]$, $[(C_yH_{2y+1}O)_2(R)Si(CH_2)_3]S_x[(CH_2)_3Si(C_yH_{2y+1}O)_2(R)]$, $[(C_yH_{2y+1}O)_3Si(CH_2)_3]S_x[(CH_2)_3Si(C_yH_{2y+1}O]_2(R)]$, $[(C_yH_{2y+1}O)(R)_2Si(CH_2)_3]S_x[(CH_2)_3Si(C_yH_{2y+1}O)_3]$, $[(C_yH_{2y+1}O)_2(R)Si(CH_2)_3]S_x[(CH_2)_3Si(C_yH_{2y+1}O)_3]$, $[(C_yH_{2y+1}O)_3Si(CH_2)_3]S_x[(CH_2)_3Si(C_yH_{2y+1}O)_3]$,

where x = 1-14, y = 10-24 and R = (MeO) or/and (EtO), or mixtures of the individual silanes.

**4.** Process for producing silane-modified oxidic or silicatic filler according to Claim 1, **characterized in that** the silane used is an organosilicon compound or mixtures of organosilicon compounds of the general formula (II)

$$X^1X^2X^3Si \text{ -A-S-S i}R_1R^2R^3 \qquad \text{(II)}$$

in which

$X^1$, $X^2$, $X^3$ and A are each independently as defined in formula (I),

$R^1$, $R^2$, $R^3$ are each independently

($C_1$-$C_{16}$) alkyl, ($C_1$-$C_{16}$) alkoxy, ($C_1$-$C_{16}$) haloalkyl, aryl, ($C_7$-$C_{16}$) aralkyl, -H, halogen or $X^1X^2X^3Si$-A-S-.

**5.** Process for producing silane-modified oxidic or silicatic filler according to Claim 1, **characterized in that** the silane used is an organosilicon compound or a mixture of organosilicon compounds of the general formula (III)

$$X^1X^2X^3Si\text{-Alk} \qquad \text{(III)}$$

in which

$X^1$, $X^2$, $X^3$ are each independently as defined in formula (I) and

Alk is a straight-chain, branched or cyclic ($C_1$-$C_{24}$) alkyl, ($C_1$-$C_{24}$) alkoxy, halogen, hydroxy, nitrile, thiol, ($C_1$-$C_4$) haloalkyl, -$NO_2$, ($C_1$-$C_8$) thioalkyl, -$NH_2$, -$NHR^1$, -$NR^1R^2$, alkenyl, allyl, vinyl, aryl or a ($C_7$-$C_{16}$) aralkyl substituent.

**6.** Process for producing silane-modified oxidic or silicatic filler according to Claim 5, **characterized in that** the silane of the formula (III) used is

$(MeO)_3$-Si-$(CH_2)_3$-H, $(EtO)_3$-Si-$(CH_2)_3$-H, $(MeO)_3$-Si-$C(CH_3)_3$, $(EtO)_3$-Si-$C(CH_3)_3$, $(MeO)_3$-Si-$(CH_2)_8$-H, $(EtO)_3$-Si-$(CH_2)_8$-H, $(MeO)_3$-Si-$(CH_2)_{16}$-H, $(EtO)_3$-Si-$(CH_2)_{16}$-H, $Me_3Si$-OMe, $Me_3Si$-OEt, $Me_3Si$-Cl, $Et_3Si$-Cl, $(MeO)_3Si$-CH=$CH_2$, $(EtO))_3Si$-CH=$CH_2$, $(Me_3Si)_2$N-C(O)-H, $(Me_3Si)_2$N-H or mixtures of the silanes.

**7.** Process for producing silane-modified oxidic or silicatic filler according to Claim 1, **characterized in that** the silane used is an organosilicon compound or a mixture of organosilicon compounds of the general formula (IV) or (V)

$$[[(ROC (=O))_p\text{-}(G)_j]_k\text{-Y-S]}_r\text{-G-}(SiX^1X^2X^3)_s \qquad \text{(IV)}$$

$$[(X^1X^2X^3Si)_q\text{-G]}_a\text{-}[Y\text{-}[S\text{- G- }SiX^1X^2X^3]_b]_c \qquad \text{(V)}$$

in which Y represents a polyvalent species (Q) $_zD$ (=E) where:

p is 0 to 5, r is 1 to 3, z is 0 to 2; q is 0 to 6, a is 0 to 7, b is 1 to 3, j is 0 to 1, but may also frequently be 0 when p = 1, c is 1 to 6, t is 0 to 5, s is 1 to 3, k is 1 to 2, with the proviso that

(1) if (D) is a carbon, sulphur or sulphonyl, a + b = 2 and k = 1,
(2) if (D) is a phosphorus atom, a + b = 3 provided that c ≥ 1 and b = 1, where a = c + 1,
(3) if (D) is a phosphorus atom, k = 2,

Y represents a polyvalent species $(Q)_zD(=E)$,

in each of these groups the atom (D) is double-bonded to the heteroatom (E) which in turn is bonded to the sulphur atom (S) which is joined by means of a group (G) to the silicon atom (Si), $R^1$ is independently H, a straight, cyclic or branched alkyl chain, optionally alkyl chains which contain unsaturated components such as double bonds (alkenes), triple bonds (alkynes) or else alkylaromatics (aralkyl) or aromatics, and which have the same definitions in formula (II),

G independently of the other substituents is hydrogen, a straight, cyclic or branched alkyl chain of $(C_1-C_{18})$, and the alkyl chains may optionally have an unsaturated component,

when p = 0 in the formula, G is preferably hydrogen (H),

G does not correspond to the structure of an $\alpha,\beta$-unsaturated fragment which is bonded to the Y fragment in such a way as to form an $\alpha,\beta$-unsaturated thiocarbonyl fragment, $X^1$, $X^2$ and $X^3$ are each independently as defined in formula (I).

8. Process for producing silane-modified oxidic or silicatic filler according to Claim 1, **characterized in that** the silane used is an organosilicon compound or a mixture of organosilicon compounds of the general formula (VI)

$$X^1X^2X^3Si\text{-}A\text{-}Sub \qquad (VI)$$

where $X^1$, $X^2$, $X^3$ and A are each independently as defined in formula (I) and Sub is $-NH_2$, $-SH$, $-NH(A-SiX^1X^2X^3)$, $-N(A-SiX^1X^2X^3)_2$, $O-C(O)-CMe=CH_2$ or $-SCN$.

9. Process for producing silane-modified oxidic or silicatic filler according to Claim 8, **characterized in that** the silane of the formula (VI) used is

$[(MeO)_3Si-(CH_2)_3-]_2NH$, $[(EtO)_3Si-(CH_2)_3-]_2NH$, $[(C_3H_7O)_3Si-(CH_2)_3-]_2NH$, $(MeO)_3Si-(CH_2)_3-NH_2$, $(EtO)_3Si-(CH_2)_3-NH_2$, $(C_3H_7O)_3Si-(CH_2)_3-NH_2$, $(MeO)_3Si-(CH_2)_3-NH-(CH_2)_2-NH_2$, $(EtO)_3Si-(CH_2)_3-NH-(CH_2)_2-NH_2$, $(C_3H_7-O)_3Si-(CH_2)_3-NH-(CH_2)_2-NH_2$, $(MeO)_3Si-(CH_2)_3-SH$, $(EtO)_3Si-(CH_2)_3-SH$, $(C_3H_7O)_3Si-(CH_2)_3-SH$, $(MeO)_3Si-(CH_2)_3-O-C(O)-CMe=CH_2$, $(EtO)_3Si-(CH_2)_3-O-C(O)-CMe=CH_2$, $(C_3H_7O)_3Si-(CH_2)_3-O-C(O)-CMe=CH_2$, SCN, $(EtO)_3Si-(CH_2)_3-SCN$, $(C_3H_7O)_3Si-(CH_2)_3-SCH$, $[(C_yH_{2y+1}O)(EtO)_2Si(CH_2)_3]-NH_2$, $[(C_yH_{2y+1}O)_2(EtO)Si(CH_2)_3]-NH_2$, $[(C_yH_{2y+1}O)(EtO)_2Si(CH_2)_3]-SH$, $[(C_yH_{2y+1}O)_2(EtO)Si(CH_2)_3]-SH$, $[(C_yH_{2y+1}O)(EtO)_2Si(CH_2)_3]-O-C(O)-CMe=CH_2$, $[(C_yH_{2y+1}O)_2(EtO)Si(CH_2)_3]-O-C(O)-CMe=CH_2$, where y = 10-24,

or mixtures of the silanes mentioned.

10. Process for producing silane-modified oxidic or silicatic filler according to Claim 1, **characterized in that** the silane used is a mixture of the silanes of the formulae I-VI.

11. Process for producing silane-modified oxidic or silicatic filler according to Claim 1, **characterized in that** the silane used is an oligomeric or cooligomeric silane of the formula I-VI or mixture thereof, or a mixture of silanes of the formulae I-VI and oligomers or cooligomers thereof.

12. Process for producing silane-modified oxidic or silicatic filler according to Claim 1, **characterized in that** the microbeaded or microgranulated oxidic or silicatic filler used is a natural and/or synthetic filler.

13. Process for producing silane-modified oxidic or silicatic filler according to Claim 1, **characterized in that** the microbeaded or microgranulated oxidic or silicatic filler used is microbeaded or microgranular kaolin, kieselguhr, mica, diatomaceous earth, clay, talc, wollastonite, silicates including those in the form of glass beads, ground glass fragments (ground glass), glass fibres or glass fabrics, zeolites, aluminium oxide, aluminium hydroxide or trihydrate, aluminium silicates, silicates, precipitated silicas with BET surface areas (measured with gaseous nitrogen) of 1 to 1000 $m^2/g$, zinc oxide, boron oxide, magnesium oxide or transition metal oxides in general.

14. Process for producing silane-modified oxidic or silicatic filler according to Claim 1, **characterized in that** 10-250 parts by weight of microbeaded or microgranulated oxidic or silicatic filler are reacted with 0.1-50 parts by weight of silane.

15. Process for producing silane-modified oxidic or silicatic filler according to Claim 1, **characterized in that** the compressed gas used is carbon dioxide, helium, nitrogen, dinitrogen monoxide, sulphur hexafluoride, gaseous alkanes having 1 to 5 carbon atoms, gaseous alkenes having 2 to 4 carbon atoms, gaseous alkynes, gaseous dienes, gaseous hydrofluorocarbons, chlorine and/or chlorofluorocarbons or substitutes therefor or ammonia, and mixtures of these substances.

16. Process for producing silane-modified oxidic or silicatic filler according to Claim 1, **characterized in that** the silanes used are undissolved or partly or fully dissolved in the compressed gas.

17. Process for producing silane-modified oxidic or silicatic filler according to Claim 1, **characterized in that** the pressure is between 1 and 500 bar.

18. Process for producing silane-modified oxidic or silicatic filler according to Claim 1, **characterized in that** the reaction is effected at a temperature between 0 and 300°C.

19. Process for producing silane-modified oxidic or silicatic filler according to Claim 1, **characterized in that** the pressure during the reaction is kept constant at different pressure levels for periods of 5-720 min and the filler during this time is immersed into the compressed gas, or the gas flows through it, or the filler is stirred within it.

20. Process for producing silane-modified oxidic or silicatic filler according to Claim 1, **characterized in that** the microbeaded or microgranulated oxidic or silicatic filler and the silane are first mixed or contacted and then mixed or contacted with the gas present in the compressed state.

21. Process for producing silane-modified oxidic or silicatic filler according to Claim 1, **characterized in that** the microbeaded or microgranulated oxidic or silicatic filler is first mixed or contacted with the gas present in the compressed state and then mixed or contacted with the silane.

22. Process for producing silane-modified oxidic or silicatic filler according to Claim 1, **characterized in that** the silane is first mixed or contacted with the gas present in the compressed state and then mixed or contacted with the corresponding microbeaded or microgranulated oxidic or silicatic filler.

23. Process for producing silane-modified oxidic or silicatic filler according to Claim 1, **characterized in that** additional additives are added to the microbeaded or microgranulated oxidic or silicatic filler and/or the silane before the conversion in the compressed gas or gas mixture.

24. Process for producing silane-modified oxidic or silicatic filler according to Claim 1, **characterized in that** the silane-modified oxidic or silicatic filler is contacted with additional additives during the reaction in the compressed gas.

25. Process for producing silane-modified oxidic or silicatic filler according to Claim 1, **characterized in that** additional additives are introduced into the supplied or removed stream of compressed gas which flows through the silane-modified oxidic or silicatic filler during the reaction of the microbeaded or microgranulated oxidic or silicatic filler in the compressed gas.

26. Process for producing silane-modified oxidic or silicatic filler according to any of Claims 23-25, **characterized in that** the additives used are ammonia, sulphur dioxide, water, short-chain or long-chain alcohols, short-chain or long-chain polyethers or short-chain or long-chain amines, emulsion formers, or else short-chain or long-chain silicone oils.

**Revendications**

1. Procédé pour la production d'une charge de type oxyde ou silicate modifiée avec un silane, ayant une fraction de perles de taille inférieure à 75 $\mu$m de moins de 15 % en poids, déterminée par analyse par tamisage, et une valeur médiane de la taille de particule comprise entre 130 et 500 $\mu$m, déterminée par diffraction laser ou traitement par ultrasons, **caractérisé en ce qu'**on fait réagir une charge de type oxyde ou silicate microperlée ou microgranulée avec au moins un silane dans un gaz comprimé au moyen de pression et/ou de température.

2. Procédé pour la production d'une charge de type oxyde ou silicate modifiée avec un silane, selon la revendication 1, **caractérisé en ce qu'**on utilise comme silane un composé organosilicié ou un mélange de composés organosiliciés de formule générale (I)

$$Z\text{-}A\text{-}S_x\text{-}A\text{-}Z \qquad (I)$$

dans laquelle
x est un nombre de 1 à 14,

Z est égal à $SiX^1X^2X^3$ et

$X^1$, $X^2$, $X^3$ peuvent représenter chacun indépendamment un atome d'hydrogène (-H),

un atome d'halogène ou le groupe hydroxy (-OH),

un substituant alkyle,

un substituant acide alkylique $(C_xH_{2x+1})$-C(=O)O-,

un substituant acide alcénylique ou

un substituant acide alkylique ou acide alcénylique substitué,

une chaîne hydrocarbonée linéaire ou ramifiée, cyclique ayant 1-8 atomes de carbone,

un radical cycloalcane ayant 5-12 atomes de carbone,

un radical benzyle ou

un radical phényle substitué par halogène ou alkyle, des groupes alcoxy à chaînes hydrocarbonées linéaires ou ramifiées ayant 1-24 atomes de carbone,

des groupes alcoxy à chaînes polyéther linéaires ou ramifiées ayant 1-24 atomes de carbone,

un groupe cycloalcoxy ayant 5-12 atomes de carbone,

un groupe phénoxy substitué par halogène ou alkyle ou un groupe benzyloxy,

A est une chaîne hydrocarbonée aliphatique aromatique ou mixte aliphatique/aromatique comprenant 1-30 atomes de carbone, à deux liaisons, ramifiée ou non ramifiée, saturée ou insaturée.

3. Procédé pour la production d'une charge de type oxyde ou silicate modifiée avec un silane, selon la revendication 2, **caractérisé en ce qu'**on utilise comme silane de formule (I)

$[(EtO)_3Si(CH_2)_3]_2S$, $[(EtO)_3Si(CH_2)_3]_2S_2$, $[(EtO)_3Si(CH_2)_3]_2S_3$, $[(Et_O)_3Si(CH_2)_3]_2S_4$, $[(EtO)_3Si(CH_2)_3]_2S_5$, $[(EtO)_3Si(CH_2)_3]_2S_6$, $[(EtO)_3Si(CH_2)_3]_2S_7$, $[(Et_O)_3Si(CH_2)_3]_2S_8$, $[(EtO)_3Si(CH_2)_3]_2S_9$, $[(EtO)_3Si(CH_2)_3]_2S_{10}$, $[(EtO)_3Si(CH_2)_3]_2S_{11}$, $[(EtO)_3Si(CH_2)_3]_2S_{12}$, $[(EtO)_3Si(CH_2)_3]_2S_{13}$, $[(EtO)_3Si(CH_2)_3]_2S_{14}$, $[(C_yH_{2y+1}O)(R)_2Si(CH_2)_3]S_x[(CH_2)_3Si(R)_3]$, $(C_yH_{2y+1}O)_2(R)Si(CH_2)_3]S_x[(CH_2)_3Si(R)_3]$, $[(C_yH_{2y+1}O)_3Si(CH_2)_3]S_x[(CH_2)_3Si(R)_3]$, $[(C_yH_{2y+1}O)(R)_2Si(CH_2)_3]S_x[(CH_2)_3Si(C_yH_{2y+1}O)(R)_2]$, $[(C_yH_{2y+1}O)_2(R)Si(CH_2)_3]S_x[(CH_2)_3Si(C_yH_{2y+1}O)(R)_2]$, $[(C_yH_{2y+1}O)_3Si(CH_2)_3]S_x[(CH_2)_3Si(C_yH_{2y+1}O)(R)_2]$, $[(C_yH_{2y+1}O)(R)_2Si(CH_2)_3]S_x[(CH_2)_3Si(C_yH_{2y+1}O)_2(R)]$, $[(C_yH_{2y+1}O)_2(R)Si(CH_2)_3]S_x[(CH_2)_3Si(C_yH_{2y+1}O)_2(R)]$, $[(C_yH_{2y+1}O)_3Si(CH_2)_3]S_x[(CH_2)_3Si(C_yH_{2y+1}O)_2(R)]$, $[(C_yH_{2y+1}O)(R)_2Si(CH_2)_3]S_x[(CH_2)_3Si(C_yH_{2y+1}O)_3]$, $[(C_yH_{2y+1}O)_2(R)Si(CH_2)_3]S_x[(CH_2)_3Si(C_yH_{2y+1}O)_3]$, $[(C_yH_{2y+1}O)_3Si(CH_2)_3]S_x[(CH_2)_3Si(C_yH_{2y+1}O)_3]$,

où x = 1-14, y = 10-24 et R = (MeO) et/ou (EtO), ou des mélanges des silanes individuels.

4. Procédé pour la production d'une charge de type oxyde ou silicate modifiée avec un silane, selon la revendication 1, **caractérisé en ce qu'**on utilise comme silane un composé organosilicié ou des mélanges de composés organosiliciés de formule générale (II)

$$X^1X^2X^3Si\text{-}A\text{-}S\text{-}SiR^1R^2R^3 \qquad (II)$$

dans laquelle

$X^1$, $X^2$, $X^3$ et A ont, indépendamment les uns des autres, la même signification que dans la formule (I),

$R^1$, $R^2$, $R^3$ sont indépendants chacun et représentent un groupe alkyle en $C_1$-$C_{16}$, alcoxy en $C_1$-$C_{16}$, halogénoalkyle $(C_1$-$C_{16})$, aryle, aralkyle en $C_7$-$C_{16}$, -H, un atome d'halogène ou $X^1X^2X^3Si$-A-S-.

5. Procédé pour la production d'une charge de type oxyde ou silicate modifiée avec un silane, selon la revendication 1, **caractérisé en ce qu'**on utilise comme silane un composé organosilicié ou un mélange de composés organosiliciés de formule générale (III)

$$X^1X^2X^3Si\text{-}Alk \qquad (III)$$

dans laquelle

$X^1$, $X^2$, $X^3$ ont chacun indépendamment la même signification que dans la formule (I) et

Alk est un substituant alkyle en $C_1$-$C_{24}$, alcoxy en $C_1$-$C_{24}$, à chaîne droite, ramifié ou cyclique, halogène, hydroxy, nitrile, thiol, halogénoalcoxy$(C_1$-$C_4)$, -$NO_2$, thioalkyle $(C_1$-$C_8)$, -$NH_2$, -$NHR^1$, -$NR^1R^2$, alcényle, allyle, vinyle, aryle ou un substituant aralkyle en $C_7$-$C_{16}$.

6. Procédé pour la production d'une charge de type oxyde ou silicate modifiée avec un silane, selon la revendication 5, **caractérisé en ce qu'**on utilise comme silane de formule (III) $(MeO)_3$-Si-$(CH_2)_3$-H, $(EtO)_3$-Si-$(CH_2)_3$-H, $(MeO)_3$-Si-C $(CH_3)_3$, $(EtO)_3$-Si-C $(CH_3)_3$, $(MeO)_3$-Si-$(CH_2)_8$-H, $(EtO)_3$-Si-$(CH_2)_8$-H, $(MeO)_3$-Si- $(CH_2)_{16}$-H, $(EtO)_3$-S1-$(CH_2)_{16}$-H, $Me_3$Si-OMe, $Me_3$Si-OEt, $Me_3$Si-Cl, $Et_3$Si-Cl, $(MeO)_3$Si-CH=$CH_2$, $(EtO)_3$Si-CH=$CH_2$, $(Me_3Si)_2$N-C

(O)-H, (Me$_3$Si)$_2$N-H ou des mélanges des silanes.

7.  Procédé pour la production d'une charge de type oxyde ou silicate modifiée avec un silane, selon la revendication 1, **caractérisé en ce qu'**on utilise comme silane un composé organosilicié ou un mélange de composés organosiliciés de formule générale (IV) ou (V) [[(ROC(=O))$_p$-(G)$_j$]$_k$-Y-S)$_r$-G-(SiX$^1$X$^2$X$^3$)$_s$ (IV) [(X$^1$X$^2$X$^3$Si)$_q$-G]$_a$-[Y-S-O-SiX$^1$X$^2$X$^3$]$_b$]$_c$ (V) formules dans lesquelles Y représente une espèce polyvalente (Q)$_z$D(=E), où s'applique ce qui suit :
    p vaut 0 à 5, r vaut 1 à 3, z vaut 0 à 2 ; q vaut 0 à 6, a vaut 0 à 7, b vaut 1 à 3, j vaut 0 à 1, mais il peut, lorsque p = 1, être souvent également 0, c vaut 1 à 6, t vaut 0 à 5, s vaut 1 à 3, k vaut 1 à 2, avec la condition que

    (1) si (D) est un atome de carbone ou de soufre ou un groupe carbonyle, il s'applique que a + b = 2 et k = 1,
    (2) si (D) est un atome de phosphore, il s'applique que a + b = 3 tant que c ≥ 1 et b = 1, a étant égal à c + 1,
    (3) si (D) est un atome de phosphore, il s'applique que k = 2,

    Y représente une espèce polyvalente (Q)$_z$D(=E),
    dans chacun de ces groupes l'atome (D) est doublement lié à l'hétéroatome (E), qui à son tour est lié à l'atome de soufre (S) qui est attaché par un groupe (G) à l'atome de silicium (Si),
    R$^1$ représente chaque fois indépendamment H, une chaîne alkyle droite, cyclique ou ramifiée, éventuellement des chaînes alkyle qui comportent des fragments insaturés tels que des doubles liaisons (alcènes), des triples liaisons (alcynes) ou bien des fragments alkylaromatiques (aralkyle) ou aromatiques et qui ont les mêmes significations que dans la formule (II),
    G représente, indépendamment des autres substituants, un atome d'hydrogène, une chaîne alkyle droite, cyclique ou ramifiée en C$_1$-C$_{18}$, les chaînes alkyle peuvent éventuellement comporter un fragment insaturé, lorsque p = 0 dans la formule, G est de préférence un atome d'hydrogène (H),
    G ne correspond pas à la structure d'un fragment α,β-insaturé qui est lié au fragment Y de manière qu'il en résulte un fragment thiocarbonyle α,β-insaturé, X$^1$, X$^2$ et X$^3$ ont chacun indépendamment la même signification que dans la formule (I).

8.  Procédé pour la production d'une charge de type oxyde ou silicate modifiée avec un silane, selon la revendication 1, **caractérisé en ce qu'**on utilise comme silane un composé organosilicié ou un mélange de composés organosiliciés de formule générale (VI)

    $$X^1X^2X^3\ Si\text{-}A\text{-}Sub \qquad (VI)$$

    dans laquelle X$^1$, X$^2$, X$^3$ et A ont chacun, indépendamment les uns des autres, la signification selon la formule (I) et Sub est -NH$_2$, -SH, -NH (A-SiX$^1$X$^2$X$^3$), -N(A-SiX$^1$X$^2$X$^3$)$_2$, -O-C(O)-CMe=CH$_2$ ou -SCN.

9.  Procédé pour la production d'une charge de type oxyde ou silicate modifiée avec un silane, selon la revendication 8, **caractérisé en ce qu'**on utilise comme silane de formule (VI) [(MeO)$_3$Si-(CH$_2$)$_3$-]$_2$NH, [(EtO)$_3$Si-(CH$_2$)$_3$-]$_2$NH, [(C$_3$H$_7$O)$_3$Si-(CH$_2$)$_3$-]$_2$NH, (MeO)$_3$Si-(CH$_2$)$_3$-NH$_2$, (EtO)$_3$Si-(CH$_2$)$_3$-NH$_2$, (C$_3$H$_7$O)$_3$Si-(CH$_2$)$_3$-NH$_2$, (MeO)$_3$Si-(CH$_2$)$_3$-NH-(CH$_2$)$_2$-NH$_2$, (EtO)$_3$Si-(CH$_2$)$_3$-NH-(CH$_2$)$_2$-NH$_2$, (C$_3$H$_7$O)$_3$Si-(CH$_2$)$_3$-NH-(CH$_2$)$_2$-NH$_2$, (MeO)$_3$Si-(CH$_2$)$_3$-SH, (EtO)$_3$Si-(CH$_2$)$_3$-SH, (C$_3$H$_7$O)$_3$Si-(CH$_2$)$_3$-SH, (MeO)$_3$Si-(CH$_2$)$_3$-O-C(O)-CMe=CH$_2$, (EtO)$_3$Si-(CH$_2$)$_3$-O-C(O)-CMe=CH$_2$, (C$_3$H$_7$O)$_3$Si-(CH$_2$)$_3$-O-C(O)-CMe=CH$_2$, SCN, (EtO)$_3$Si-(CH$_2$)$_3$-SCN, (C$_3$H$_7$O)$_3$Si-(CH$_2$)$_3$-SCN, [(C$_y$H$_{2y+1}$O)(EtO)$_2$Si(CH$_2$)$_3$]-NH$_2$, [(C$_y$H$_{2y+1}$O)$_2$(EtO)Si(CH$_2$)$_3$]-NH$_2$, [(C$_y$H$_{2y+1}$O)(EtO)$_2$Si(CH$_2$)$_3$]-SH, [(C$_y$H$_{2y+1}$O)$_2$(EtO)Si(CH$_2$)$_3$]-SH, [(C$_y$H$_{2y+1}$O)(EtO)$_2$Si(CH$_2$)$_3$]-O-C(O)-CMe=CH$_2$, [(C$_y$H$_{2y+1}$O)$_2$(EtO)Si(CH$_2$)$_3$]-O-C(O)-C-Me=CH$_2$, où y = 10-24,
    ou des mélanges des silanes nommés.

10. Procédé pour la production d'une charge de type oxyde ou silicate modifiée avec un silane, selon la revendication 1, **caractérisé en ce qu'**on utilise comme silane des mélanges des silanes de formules I-VI.

11. Procédé pour la production d'une charge de type oxyde ou silicate modifiée avec un silane, selon la revendication 1, **caractérisé en ce qu'**on utilise comme silane des silanes oligomères ou co-oligomères de formules I-VI ou des mélanges de ceux-ci ou des mélanges de silanes de formules I-VI et de leurs oligomères ou co-oligomères.

12. Procédé pour la production d'une charge de type oxyde ou silicate modifiée avec un silane, selon la revendication 1, **caractérisé en ce qu'**on utilise comme charge de type oxyde ou silicate microperlée ou microgranulée une charge naturelle et/ou synthétique.

**13.** Procédé pour la production d'une charge de type oxyde ou silicate modifiée avec un silane, selon la revendication 1, **caractérisé en ce qu'**on utilise comme charge de type oxyde ou silicate microperlée ou microgranulée du kaolin microperlé ou microgranulé, du kieselguhr, du mica, de la terre à diatomées, de l'argile, du talc, de la wollastonite, des silicates en particulier sous forme de billes de verre, d'éclats de verre broyés (poudre de verre), de fibres de verre ou de tissus de verre, des zéolithes, de l'oxyde d'aluminium, de l'hydroxyde ou du trihydrate d'aluminium, des silicates d'aluminium, des silicates, des acides siliciques précipités à surfaces BET (mesurées avec de l'azote gazeux) de 1 à 1 000 m$^2$/g, de l'oxyde de zinc, de l'oxyde de bore, de l'oxyde de magnésium ou d'une façon générale des oxydes de métaux de transition.

**14.** Procédé pour la production d'une charge de type oxyde ou silicate modifiée avec un silane, selon la revendication 1, **caractérisé en ce qu'**on fait réagir 10-250 parties en poids de charge de type oxyde ou silicate microperlée ou microgranulée avec 0,1 - 50 parties en poids de silane.

**15.** Procédé pour la production d'une charge de type oxyde ou silicate modifiée avec un silane, selon la revendication 1, **caractérisé en ce qu'**on utilise comme gaz comprimé le dioxyde de carbone, l'hélium, l'azote, le protoxyde d'azote, de l'hexafluorure de soufre, des alcanes gazeux ayant de 1 à 5 atomes de carbone, des alcènes gazeux ayant de 2 à 4 atomes de carbone, des alcynes gazeux, des diènes gazeux, des fluorohydrocarbures gazeux, le chlore et/ou des fluorochlorohydrocarbures ou leurs substituts ou l'ammoniac, ainsi que des mélanges de ces substances.

**16.** Procédé pour la production d'une charge de type oxyde ou silicate modifiée avec un silane, selon la revendication 1, **caractérisé en ce que** les silanes utilisés sont non dissous, partiellement ou totalement dissous dans le gaz comprimé.

**17.** Procédé pour la production d'une charge de type oxyde ou silicate modifiée avec un silane, selon la revendication 1, **caractérisé en ce que** la pression est comprise entre 1 et 500 bars.

**18.** Procédé pour la production d'une charge de type oxyde ou silicate modifiée avec un silane, selon la revendication 1, **caractérisé en ce que** la réaction s'effectue à une température de 0 à 300 °C.

**19.** Procédé pour la production d'une charge de type oxyde ou silicate modifiée avec un silane, selon la revendication 1, **caractérisé en ce qu'**on maintient constamment la pression pendant la réaction à différents niveaux de pression pendant des périodes de 5 - 720 minutes et pendant ce temps la charge est introduite dans le gaz comprimé, traversée par celui-ci ou agitée dans celui-ci.

**20.** Procédé pour la production d'une charge de type oxyde ou silicate modifiée avec un silane, selon la revendication 1, **caractérisé en ce que** d'abord on mélange ou met en contact la charge de type oxyde ou silicate, microperlée ou microgranulée, et le silane et ensuite on les mélange ou met en contact avec le gaz se trouvant à l'état comprimé.

**21.** Procédé pour la production d'une charge de type oxyde ou silicate modifiée avec un silane, selon la revendication 1, **caractérisé en ce que** d'abord on mélange ou met en contact la charge de type oxyde ou silicate, microperlée ou microgranulée, avec le gaz se trouvant à l'état comprimé et ensuite on la mélange ou met en contact avec le silane.

**22.** Procédé pour la production d'une charge de type oxyde ou silicate modifiée avec un silane, selon la revendication 1, **caractérisé en ce que** d'abord on mélange ou met en contact le silane avec le gaz se trouvant à l'état comprimé et ensuite on les mélange ou met en contact avec la charge de type oxyde ou silicate, microperlée ou microgranulée.

**23.** Procédé pour la production d'une charge de type oxyde ou silicate modifiée avec un silane, selon la revendication 1, **caractérisé en ce que** qu'avant la réaction dans le gaz ou mélange de gaz comprimé on ajoute des additifs supplémentaires à la charge de type oxyde ou silicate, microperlée ou microgranulée et/ou au silane.

**24.** Procédé pour la production d'une charge de type oxyde ou silicate modifiée avec un silane, selon la revendication 1, **caractérisé en ce que** pendant la réaction dans le gaz comprimé on met en contact avec des additifs supplémentaires la charge de type oxyde ou silicate modifiée avec un silane.

**25.** Procédé pour la production d'une charge de type oxyde ou silicate modifiée avec un silane, selon la revendication 1, **caractérisé en ce que** pendant la réaction de la charge de type oxyde ou silicate, microperlée ou microgranulée on introduit des additifs supplémentaires dans le gaz comprimé, dans le courant introduit ou évacué de gaz comprimé qui traverse la charge de type oxyde ou silicate modifiée avec un silane.

26. Procédé pour la production d'une charge de type oxyde ou silicate modifiée avec un silane, selon l'une quelconque des revendications 23 à 25, **caractérisé en ce qu'**on utilise comme additifs l'ammoniac, l'anhydride sulfureux, l'eau, des alcools à courte chaîne ou à longue chaîne, des polyéthers à courte chaîne ou à longue chaîne ou des amines à courte chaîne ou à longue chaîne, des émulsifiants ou bien des huile de silicone à courte chaîne ou à longue chaîne.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 2141159
- DE 2212239
- US 3978103 A
- DE 2542534
- DE 2405758
- DE 19541404
- DE 19734295
- US 3997356 A
- DE 3314742
- US 4076550 A
- US 4151154 A
- US 3567680 A
- US 4044037 A

- EP PS0126871 A
- US 3227675 A
- DE 3314742 C2
- DE 3014007
- DE 10122269
- DE 19844607
- EP 0958298 A
- WO 9909036 A
- EP 652245 B1
- EP 0700951 B1
- EP 0978525 A2
- DE 19929021 A1
- US PS4076550 A

### In der Beschreibung aufgeführte Nicht-Patentliteratur

- **E.Stahl ; K.W.Quirin ; D.Gerard.** Verdichtete Gase zur Extraktion und Raffination. Springer-Verlag, 12-13
- **G.W. Sears.** *Analyt. Chemistry,* 1956, vol. 12, 1982

- **F. Ehrenberger ; S. Gorbauch.** Methoden der organischen Elementar- und Spurenanalyse. Verlag Chemie GmbH, 1973
- **Hunsche.** *Kautschuk, Gummi, Kunststoffe,* 1998, vol. 51, 525
- **W. Hofmann.** Rubber Technology Handbook. Hanser Verlag, 1994